# EUROPÄISCHE PATENTSCHRIFT

(11) **EP 4 555 876 B1**
(45) Veröffentlichungstag und Bekanntmachung des Hinweises auf die Patenterteilung: **01.04.2026**
(21) Anmeldenummer: 24196843.7
(22) Anmeldetag: 27.08.2024
(51) Int. Cl.: A24F 40/05, A24B 15/167, B05B 17/00, B05B 17/06

(54) **AEROSOLERZEUGUNGSVORRICHTUNG UND VERFAHREN ZUM ERZEUGEN EINES AEROSOLS**
AEROSOL GENERATING DEVICE AND METHOD FOR GENERATING AN AEROSOL
DISPOSITIF DE GÉNÉRATION D'AÉROSOL ET PROCÉDÉ DE GÉNÉRATION D'UN AÉROSOL

(30) Priorität: 28.08.2023 DE 202023001809 U; 28.08.2023 DE 202023001810 U; 28.08.2023 DE 202023001823 U; 28.08.2023 DE 202023001824 U
(43) Veröffentlichungstag der Anmeldung: 21.05.2025
(73) Patentinhaber: Kersten, Olaf, 47802 Krefeld (DE); Deppe, Marc, 47661 Issum (DE)
(72) Erfinder: Kersten, Olaf, 47802 Krefeld (DE); Deppe, Marc, 47661 Issum (DE)
(74) Vertreter: Dammertz, Ulrich

(56) Entgegenhaltungen:
- WO-A1-2020/141112
- WO-A1-2022/122651
- WO-A1-2022/152529
- CN-A- 101 282 792
- IT-A1- 202100 005 027
- JP-A- 2015 062 892
- US-A1- 2011 315 786
- US-A1- 2018 255 834
- US-A1- 2019 104 762
- US-A1- 2021 236 746

## Beschreibung

Die Erfindung betrifft eine Aerosolerzeugungsvorrichtung zum Erzeugen eines Aerosols nach dem Oberbegriff von Anspruch 1, und ein Verfahren zum Erzeugen eines Aerosols.

In den letzten Jahren hat sich die Verwendung von Aerosolen, die durch Nichtverbrennungstechniken erzeugt werden, immer mehr durchgesetzt. Elektrische Zigaretten (auch E-Zigaretten oder elektronische Zigaretten genannt) sind üblicherweise elektrisch beheizte Geräte zur Verdampfung eines Verbrauchsstoffs, insbesondere einer aromatisierten Flüssigkeit (auch Liquid genannt). Meist bestehen diese Geräte aus einem Tank, einem Verdampfer und einem Akkuträger. Bei den Tanksystemen unterscheidet man zwischen Cartomizer, Tröpfler, Clearomizer und Kartuschen. Beim Cartomizer ist die Heizwendel von einem als Flüssigkeitsspeicher dienenden Vliesstoff oder Watte umgeben. Ein Tröpfler ist technisch gesehen ein Cartomizer mit sehr geringem Aufnahmevolumen. Der Verbrauchsstoff wird hier vom Benutzer direkt auf die Dochte getropft, die zugleich als Flüssigkeitsspeicher dienen und muss nach wenigen Zügen erneut durch den Benutzer aufgebracht werden. Ein Clearomizer besteht aus einem Tank, in dem sich das Liquid frei vom Trägermaterial und abgekapselt vom Verdampfer befindet. Die vorstehend genannten Systeme haben alle gemeinsam, dass der Benutzer regelmäßig neuen Verbrauchsstoff nachfüllen muss. Bei Kartuschensystemen werden mit Liquid gefüllte Kartuschen in die E-Zigarette eingelegt. Die Verdampfungseinheit dient zum Verdampfen des Liquids. In der Regel findet in einem E-Zigaretten-Verdampfer kein Verbrennungsprozess statt, sondern eine Flüssigkeit gelangt insbesondere durch die Kapillarwirkung eines Dochtes von einem Tank zu einer Verdampfungseinheit, die meist als Heizwendel ausgebildet ist. Sobald der Benutzer durch Inhalieren einen Unterdruck innerhalb des Verdampfers erzeugt, wird die Verdampfungseinheit von einem Luftstrom umströmt, wobei das entstandene Aerosol durch die Sogwirkung vom Benutzer eingeatmet wird. Die Energieversorgung der Verdampfungseinheit erfolgt in der Regel über einen mit Akkus oder Batterien bestückten Akkuträger. Im Stand der Technik sind mehrere verschiedene Schnittstellen zur Verbindung eines Akkuträgers mit einem Verdampfer bekannt. Verdampfer der eingangs erwähnten Art sind in einer Vielzahl von unterschiedlichen Ausführungsformen bekannt.

Die Benutzer passen sich an die Verwendung von Aerosolen an, die ohne Verbrennung erzeugt werden, um bekannte nachteilige Auswirkungen der Verbrennung zu vermeiden, wie z. B. bei herkömmlichen Zigaretten, bei denen ein Ende angezündet wird, um Tabak oder andere Inhaltsstoffe zu verbrennen, um Rauch zu erzeugen. Aerosolgeneratoren ohne Verbrennung, auch als persönliche Verdampfer, Inhalatoren und E-Zigaretten bezeichnet, werden immer beliebter. Solche Aerosolgeneratoren verwenden elektrische Heiz- oder Ultraschalltechniken, um eine im Inneren des Aerosolgenerators gespeicherte Flüssigkeit zu aerosolisieren.

Ein wesentlicher Nachteil dieser vorbekannten Verdampfer besteht insbesondere darin, dass nach dem Inhalieren beim Ausatmen die Umgebung mit Restmengen des verdampften Liquids belastet wird.

Bei der Ultraschalltechnik, die z.B aus DE 202018000122 U1, US 2020/245692 A1, WO 2021/081009 A1, EP 3247435 A1, US 2020/0245692 A1, WO 2021/081009 A1 oder EP 3 247 435 B1 bekannt ist, wird ein Ultraschallgenerator (auch als Ultraschallaktor oder -wandler bezeichnet) mit Piezokeramik verwendet, der Ultraschall erzeugt, um die Flüssigkeit effizient zu aerosolisieren oder zu vernebeln.

US 2021/236746 A1 zeigt eine Aerosolerzeugungsvorrichtung mit den Merkmalen nach dem Oberbegriff von Anspruch 1.

US 2011/315786 A1 zeigt eine Aerosolerzeugungsvorrichtung in Form einer Zerstäubereinheit, mit einem piezoelektrischen Schwinger, einer elastischen Folie und einer ersten Flüssigkeitszuführeinrichtung. Der piezoelektrische Schwinger umfasst eine vibrierende Membran mit einer zentralen Zerstäubungsregion, die Durchgangslöcher aufweist, und ist am piezoelektrischen Körper befestigt, der die Membran in Schwingung versetzt. Die elastische Folie ist der Zerstäubungsregion zugewandt und hält die Flüssigkeitszuführeinrichtung so, dass zwischen ihr und der vibrierenden Membran ein Abstand besteht, wobei die Flüssigkeit in die Zerstäubungsregion geleitet wird. Ist die erste Flüssigkeitszuführeinrichtung aus weichen Materialien wie Filz, Vliesstoff oder Vliespapier gefertigt, kann während des Betriebs ein Kontakt mit der vibrierenden Membran entstehen; der jedoch aufgrund der weichen Materialeigenschaften der ersten Flüssigkeitszuführeinrichtung keine Schäden an der Membran verursacht.

Bei diesem Stand der Technik liegt ein Docht direkt (oder mittelbar, vgl. EP 3 247 435 B1) am Ultraschallelement an oder er steht mit dem Ultraschallelement in Verbindung. Durch den Kontakt des Dochtes mit dem Ultraschallelement wird die freie Schwingung des Ultraschallelementes verhindert, wodurch es zu Frequenzstörungen am Ultraschallelement kommt und damit die Aerosolbildung behindert. Wenn der Aerosolgenerator nicht aktiv zur Erzeugung von Aerosol verwendet wird, setzen sich Dämpfe aus der im Aerosolgenerator gespeicherten Flüssigkeit auf einer Unterseite des Ultraschallgenerators (d. h. der dem Reservoir zugewandten Seite) ab und bilden Tröpfchen (z. B. durch Kondensation), die sich an verschiedenen Stellen ansammeln, einschließlich an einer Oberseite des Ultraschallgenerators (d. h. der gegenüberliegenden Seite oder Rückseite der Unterseite). Die Tröpfchen verhindern einen effizienten und schnellen Neustart der Vernebelung, wenn der Ultraschallgenerator verwendet wird.

Ein ähnliches Problem entsteht, wenn Flüssigkeit durch Kapillarwirkung aus dem Aerosolgenerator in Richtung des Ultraschallgenerators austritt und sich auf dem Ultraschallgenerator, insbesondere auf einer Oberseite des Ultraschallgenerators, absetzt. Flüssigkeiten, insbesondere wasserbasierte Flüssigkeiten, die aufgrund der Kapillarwirkung in den Öffnungen des Ultraschallelementes verbleiben, verursachen Korrosion durch ihren PH-Wert an dem Ultraschallelement.

Die Verkeimung eines solchen Aerosolgenerators unter Verwendung einer wasserbasierten Flüssigkeit ist ebenfalls ein sehr großes Problem, die zwingend zu unterbinden ist. Hierzu werden diese Flüssigkeiten z.B. durch die Zugabe von Alkohol so angepasst, dass eine Verkeimung verhindert wird. Die Zugabe solcher Stoffe wie z.B. Alkohol verringert aber die Oberflächenspannung von Wasser. Die Oberflächenspannung einer Flüssigkeit betrifft die Grenzfläche zwischen der Flüssigkeit und der Luft. Sie entsteht aufgrund der Anziehungskräfte zwischen den Molekülen der Flüssigkeit an der Oberfläche. Alkoholmoleküle haben eine geringere Anziehungskraft als Wassermoleküle, wodurch sie die Anziehungskräfte an der Oberfläche von Wasser stören können. Wenn Alkohol zu Wasser hinzugefügt wird, verringert dies die Oberflächenspannung, da die Alkoholmoleküle zwischen den Wassermolekülen platziert werden und die Anziehungskräfte abschwächen.

Aufgrund eines oder mehrerer der oben genannten Probleme kommt es zum einen zu einer gestörten Aerosolerzeugung und zum anderen zu einer Verzögerung bei der Aerosolerzeugung. Hierbei ist es erforderlich, das Gerät zu schütteln oder zu schlagen, um die Tröpfchen vom Ultraschallgenerator abzuschlagen und die Aerosolerzeugung zu unterstützen. Ist die Korrosion stark fortgeschritten, ist die Funktion des Ultraschallgenerators nicht mehr gewährleistet.

Die oben genannten Probleme der Verdampfung und Kondensation und/oder Leckage werden noch verschärft, wenn die in dem Aerosolgenerator gespeicherte Flüssigkeit Komponenten wie Wasser enthält.

JP 2015 062892 A zeigt eine Aerosolerzeugungsvorrichtung in Form eines Ultraschall-Zerstäubers zur Vernebelung von Flüssigkeiten wie Wasser oder chemischen Lösungen mittels piezoelektrischer Schwingung. Dieser Ultraschall-Zerstäuber umfasst einen austauschbaren Flüssigkeitsbehälter mit einem integriertem Absorberelement. Das Absorberelement kann zusammen mit dem Flüssigkeitsbehälter als Einheit ausgetauscht werden, wodurch Verstopfungen der Membranporen durch getrocknete Fasern verhindert werden. Dieser Ultraschall-Zerstäuber nutzt einen piezoelektrischen Vibrator mit Membran, die über feine Löcher verfügt, um durch Ultraschallvibration die vom Absorberelement zugeführte Flüssigkeit zu zerstäuben.

CN 101 282 792 A offenbart eine Aerosolerzeugungsvorrichtung in Form eines piezoelektrischen Ultraschall-Zerstäubers, insbesondere für medizinische Inhalatoren oder Luftbefeuchter. Eine Flüssigkeit wird aus einem Vorratsbehälter über einen kapillaren Flüssigkeitsleiter zu einer Ultraschall-Zerstäubungseinheit gefördert, die ein piezoelektrisches Element mit einer vibrierenden Metallmembran umfasst. In der Membran sind zahlreiche feine Öffnungen vorgesehen, durch welche die Flüssigkeit beim Schwingen in fein verteilte Tröpfchen überführt wird. Hierbei ist ein direkter Kontakt zwischen dem Flüssigkeitsleiter und der Membran vorgesehen, um eine ausreichende Zerstäubung der Flüssigkeit sicherzustellen. Zur Optimierung des Flüssigkeits-transports dient eine spezielle Tunnel- oder Boss-Struktur.

US 2019/104762 A1 zeigt eine Aerosolerzeugungsvorrichtung, insbesondere für nikotinhaltige Flüssigkeiten, bei der ein Ultraschallvernebler zur Erzeugung eines inhalierbaren Aerosols eingesetzt wird. Diese Vorrichtung umfasst einen Flüssigkeitsbehälter sowie ein kapillar wirkendes Element, das die Flüssigkeit zu einer Ultraschall-Zerstäubereinheit fördert. Diese besteht aus einem piezoelektrischen Aktor und einer perforierten Membran, die durch den Aktor in Schwingung versetzt wird, um dadurch die Flüssigkeit, die sich auf oder an der Membran befindet, in ein Aerosol zu überführen.

Somit ist eine Technik zur Erzeugung von Aerosol erwünscht, die einen oder mehrere der oben genannten Nachteile verringert oder vermeidet.

Entsprechend liegt der Erfindung die Aufgabe zugrunde, den Betrieb einer Aerosolerzeugungsvorrichtung und eines Verfahrens zum Erzeugen von Aerosol hinsichtlich der Aerosolbildung zu optimieren.

Die obige Aufgabe wird durch eine Vorrichtung mit den Merkmalen von Anspruch 1, und durch ein Verfahren mit den Merkmalen nach Anspruch 10 gelöst. Vorteilhafte Weiterbildungen der Erfindung sind Gegenstand der abhängigen Ansprüche.

Eine Aerosolerzeugungsvorrichtung zum Erzeugen eines Aerosols gemäß der vorliegenden Erfindung kann ein persönlicher Verdampfer, ein Inhalator, eine elektrische Zigarette oder eine E-Zigarette sein. Die Aerosolerzeugungsvorrichtung kann eine tragbare und/oder eine Vorrichtung im Taschenformat sein. Die Aerosolerzeugungsvorrichtung (im Folgenden auch als Aerosolgenerator oder einfach als Vorrichtung bezeichnet) umfasst einen Behälter (auch als Reservoir bezeichnet), einen Docht (auch als Kapillarelement bezeichnet), einen Ultraschallgenerator (auch als Ultraschallsonde, Wandler, Aktor bezeichnet) und ein Mundstück.

Eine Aerosolerzeugungsvorrichtung gemäß der vorliegenden Erfindung dient zum Erzeugen eines Aerosols, und umfasst einen Behälter mit einem darin ausgebildeten Tank, in dem eine zu versprühenden Flüssigkeit gespeichert aufgenommen ist, wobei der Behälter aufweist: eine Auslassöffnung, einen Ultraschallgenerator, mittels dessen das Aerosol aus der Flüssigkeit erzeugbar ist, ein an dem Behälter angrenzend zu dessen Auslassöffnung angebrachtes Mundstück, das eine Austrittsöffnung zum Freisetzen des von dem Ultraschallgenerator erzeugten Aerosols aufweist, und einen in der Auslassöffnung des Behälters angeordneten Docht zum Transport der Flüssigkeit aus dem Tank zum Ultraschallgenerator, wobei der Docht ein im Inneren des Tanks angeordnetes Einlassende zur Aufnahme der in dem Tank gespeicherten Flüssigkeit und ein angrenzend zum Ultraschallgenerator angeordnetes Auslassende zur Bereitstellung der Flüssigkeit für den Ultraschallgenerator aufweist. Der Ultraschallgenerator ist zwischen der Austrittsöffnung des Mundstücks und dem Docht angeordnet ist. Der Ultraschallgenerator umfasst einen ersten Teil mit einem piezoelektrischen Element und einen zweiten Teil mit einer Vielzahl von Durchgängen, durch welche die Flüssigkeit hindurchtreten kann. Das Auslassende des Dochtes ist so weit von dem zweiten Teil des Ultraschallgenerators beabstandet, dass der in Schwingung versetzte zweite Teil mit der Vielzahl von Durchgängen nur die Flüssigkeit, die sich durch die Oberflächenspannung der selbigen Flüssigkeit am Auslassende des Dochts angesammelt hat und dort in Folge ihrer Oberflächenspannung einen bogenförmigen Tropfen bildet, berührt oder in diese Flüssigkeit eintaucht und dadurch die Flüssigkeit in den Durchgängen des zweiten Teils des Ultraschallgenerators aufgenommen wird.

Der vorliegenden Erfindung liegt die wesentliche Erkenntnis zugrunde, dass das Auslassende des Dochtes so weit von dem Ultraschallgenerator beabstandet ist, dass dessen zweiter Teil, der zum Erzeugen eines Aerosols erfindungsgemäß in Schwingung versetzt wird, den Docht an seinem Auslassende nicht direkt berüht bzw. damit in Kontakt steht. Hierdurch wird der Vorteil erreicht, dass im Betrieb der Aerosolerzeugungsvorrichtung die Schwingung des zweiten Teils des Ultraschallgenerators nicht durch eine Berührung mit dem Docht beeinflusst bzw. beeinträchtigt wird. Anders ausgedrückt, kann der zweite Teil des Ultraschallgenerators, in dem die Vielzahl von Durchgängen ausgebildet ist, beabstandet zum Docht und somit ungehindert vom Docht in Schwingung versetzt werden. Hierbei ist die Aufnahme von Flüssigkeit, die sich bedingt durch deren Oberflächenspannung an der Auslassöffnung des Dochtes angesammelt hat, dadurch gewährleistet, dass der zweite Teil des Ultraschallgenerators, wenn er in Schwingung versetzt und dabei in Richtung des Dochts verformt wird, dann in diese Ansammlung von Flüssigkeit eintaucht und dadurch die Flüssigkeit in den Durchgängen des zweiten Teils des Ultraschallgenerators aufgenommen wird. Hier tritt dann die Flüssigkeit durch die Durchgänge des zweiten Teils des Ultraschallgenerators zur entgegensetzten Seite des zweiten Teils hindurch, wobei dann bei fortgesetzter Schwingung des zweiten Teils die Flüssigkeit aus den Durchgängen nach außen herausgeschleudert und sich hierdurch das gewünschte Aerosol erzeugt wird.

Gemäß einer vorteilhaften Weiterbildung der Erfindung kann vorgesehen sein, dass der zweite Teil des Ultraschallgenerators gegenüber dem Auslassende des Dochts gesehen einen konkaven Abschnitt aufweist, wobei die Durchgänge des zweiten Teils des Ultraschallgenerators in dem konkaven Abschnitt ausgebildet sind. Bedingt durch den besagten konkaven Abschnitt ist erfindungsgemäß gewährleistet, dass der zweite Teil des Ultraschallgenerators, sowohl in Ruhestellung als auch insbesondere in seinem Schwingungszustand, stets ausreichend weit von dem Docht und dessen Auslassende beabstandet ist, so dass die Schwingung des zweiten Teils des Ultraschallgenerators nicht durch eine Berührung mit dem Docht beeinträchtigt wird.

Die vorliegende Erfindung sieht ebenfalls ein Verfahren zum Erzeugen eines Aerosols unter Verwendung einer Aerosolerzeugungsvorrichtung mit einem Docht vor, wobei es sich bei dieser Aerosolerzeugungsvorrichtung um die vorstehend genannte erfindungsgemäße Vorrichtung handeln kann. Jedenfalls wird bei diesem Verfahren eine Aerosolerzeugungsvorrichtung mit einem Docht und einem Ultraschallgenerator verwendet, der einen ersten Teil mit einem piezoelektrischen Element und einen zweiten Teil mit einer Vielzahl von für Flüssigkeit durchlässigen Durchgängen umfasst. Des Weiteren ist bei dem erfindungsgemäßen Verfahren vorgesehen, dass eine Flüssigkeit, mit der das Aerosol erzeugt wird, eine wasserbasierte Nikotinflüssigkeit ist, wobei die Flüssigkeit eine Oberflächenspannung hat, die ausreichend hoch gewählt bzw. eingestellt ist, dass sich an einem Auslassende des Dochtes und gegenüberliegend zum Ultraschallgenerator eine Ansammlung von Flüssigkeit als bogenförmiger Tropfen zur Übergabe an den Ultraschallgenerator bildet, wobei Flüssigkeit von der Flüssigkeitsansammlung am Auslassende des Dochtes dann, wenn der Ultraschallgenerator bei seiner Aktuierung in Schwingung versetzt wird und sich dabei in Richtung des Auslassendes des Dochts verformt, an den zweiten Teil des Ultraschallgenerators übergeben wird.

Gemäß einer vorteilhaften Weiterbildung der Erfindung kann die Flüssigkeit verkeimungshemmende Stoffe enthalten. Erfindungsgemäß ist das Merkmal "verkeimungshemmende Stoffe" dahingehend zu verstehen, dass es sich hierbei um zumindest einen solchen Stoff bzw. eine Substanz handeln kann, mit dem bzw. der eine Verkeimung zumindest gehemmt oder gar verhindert wird.

Bei dem vorstehend genannten Verfahren gemäß der vorliegenden Erfindung besteht ein charakteristisches Merkmal darin, dass die Flüssigkeit eine wasserbasierte Nikotinflüssigkeit, wobei andere Flüssigkeitsanteile, die ggf. eine Verkeimung der Aerosolerzeugungsvorrichtung verhindern, die Oberflächenspannung der Flüssigkeit nicht so weit reduzieren, dass es nicht mehr zu einer Ansammlung von Flüssigkeit an dem Auslassende des Dochtes kommt.

Gemäß einer vorteilhaften Weiterbildung der Erfindung ist in der Flüssigkeit als verkeimungshemmender Stoff zumindest eine Substanz gebildet aus der Gruppe Wasserstoffperoxid, ätherische Öle, Silberionen-Lösung und Natriumchlorid enthalten. Vorzugsweise kann in der Flüssigkeit eine Kombination von Substanzen gebildet aus der Gruppe von Wasserstoffperoxid, ätherische Öle, Silberionen-Lösung und/oder Natriumchlorid enthalten sein.

Eine erfindungsgemäße Aerosolerzeugungsvorrichtung gemäß einer weiteren Ausführungsform, der eine eigenständige Bedeutung zukommt, umfasst einen Behälter mit einem darin ausgebildeten Tank zum Speichern einer zu versprühenden Flüssigkeit, wobei der Behälter aufweist: eine Auslassöffnung, einen Ultraschallgenerator, mittels dessen das Aerosol aus der Flüssigkeit erzeugbar ist, ein an dem Behälter angrenzend zu dessen Auslassöffnung angebrachtes Mundstück, das eine Austrittsöffnung zum Freisetzen des von dem Ultraschallgenerator erzeugten Aerosols aufweist, und einen in der Auslassöffnung des Behälters angeordneten Docht zum Transport der Flüssigkeit aus dem Tank zum Ultraschallgenerator, wobei der Docht ein im Inneren des Tanks angeordnetes Einlassende zur Aufnahme der in dem Tank gespeicherten Flüssigkeit und ein angrenzend zum Ultraschallgenerator angeordnetes Auslassende zur Bereitstellung der Flüssigkeit für den Ultraschallgenerator aufweist. Der Ultraschallgenerator ist zwischen der Austrittsöffnung des Mundstücks und dem Docht angeordnet ist. Charakteristische Merkmale der Erfindung bestehen darin, dass die Flüssigkeit eine wasserbasierte Nikotinflüssigkeit ist, dass die Flüssigkeit verkeimungshemmende Stoffe enthält, und dass die Flüssigkeit eine Oberflächenspannung hat, mit der sich an dem Auslassende des Dochtes eine Ansammlung von Flüssigkeit zur Übergabe an den Ultraschallgenerator bildet.

Gemäß einer vorteilhaften Weiterbildung der Erfindung können die besagten verkeimungshemmenden Stoffe bzw. anderen Flüssigkeitsanteile, mit denen u.a. die gewünschte Verhinderung einer Verkeimung erreicht wird, folgende Substanzen enthalten oder hieraus gebildet sein:
- Wasserstoffperoxid: Diese Substanz wirkt oxidierend und kann Mikroorganismen effektiv abtöten; und/oder
- Ätherische Öle: Einige Öle, wie Teebaumöl, haben natürliche antimikrobielle Eigenschaften; und/oder
- Silberionen-Lösungen: Silber hat die Eigenschaft eines antimikrobiellen Mittels und leistet somit ebenfalls einen Beitrag zur Verhinderung einer Verkeimung; und/oder:
- Natriumchlorid: Diese Substanz hat ebenfalls eine konservierende Eigenschaft und kann dazu beitragen, eine Verkeimung zu verhindern.

Gemäß einer vorteilhaften Weiterbildung der Erfindung kann vorgesehen sein, dass die Flüssigkeit Nikotin in einer Menge enthält, die aus einem Bereich von 0,05 mg/ml bis 20 mg/ml gewählt ist.

Gemäß einer vorteilhaften Weiterbildung der Erfindung kann vorgesehen sein, dass die Flüssigkeit Natriumchlorid in einer Menge von 0,5 bis 3 Gewichtsprozent bezogen auf den Anteil von Wasser enthält. Zweckmäßigerweise kann die Flüssigkeit 0,9 Gewichtsprozent Natriumchlorid (bezogen auf den Anteil von Wasser) enthalten. Erfindungsgemäß hat Natriumchlorid zwei Auswirkungen auf die Eigenschaft der zu vernebelnden Flüssigkeit: Es hat einerseits konservierende Eigenschaften und kann wie bereits erläutert dazu beitragen, eine Verkeimung zu verhindern. Des Weiteren wird mit Natriumchlorid auch die Oberflächenspannung der Flüssigkeit vorteilhaft erhöht. Damit kommt es beispielsweise an dem Auslassende des Dochts in gewünschter Weise zu einer Ansammlung von Flüssigkeit, die dann von dem Ultraschallgenerator, wenn dieser in Schwingung versetzt wird, geeignet aufgenommen und aerosolisiert bzw. vernebelt wird.

Im Sinne der vorliegenden Erfindung versteht sich, dass die vorstehend genannten Substanzen in der Flüssigkeit entweder alleine oder in einer Kombination hiervon enthalten sein können, jeweils in verschiedenen geeigneten Konzentrationen.

Gemäß einer vorteilhaften Weiterbildung der Erfindung kann vorgesehen sein, dass die Flüssigkeit enthält: 85 Volumenprozent Wasser, 15 Volumenprozent Ethanol, 0,5 mg/ml Nikotin, 0,9 Gewichtsprozent Natriumchlorid (bezogen auf den Anteil von Wasser).

Gemäß einer vorteilhaften Weiterbildung der Erfindung kann vorgesehen sein, dass in den Docht der Vorrichtung zumindest eine Substanz gebildet aus der Gruppe Wasserstoffperoxid, ätherische Öle, Silberionen-Lösung und Natriumchlorid eingebracht ist, vorzugsweise, dass in den Docht eine Kombination von Substanzen gebildet aus der Gruppe von Wasserstoffperoxid, ätherische Öle, Silberionen-Lösung und/oder Natriumchlorid eingebracht ist. In Folge eines Durchströmens des Dochts nimmt dann die Flüssigkeit die Substanz(en), die in den Docht zuvor eingebracht worden ist bzw. sind, bei einem Durchströmen des Dochts auf, so dass damit dann diese Substanzen in der Flüssigkeit gelöst bzw. aufgenommen werden und beispielsweise die vorstehend genannte Wirkung zwecks der Verhinderung einer Verkeimung ausüben.

Für die vorstehend erwähnte Variante der Erfindung, wonach zumindest eine der genannten Substanzen geeignet in den Docht eingebracht ist, ist erfindungsgemäß zu verstehen, dass es hierbei dann möglich ist, dass diese Substanz (oder vorzugsweise alle dieser Substanzen, falls es mehrere sind), falls der Docht in Folge seiner Kapillarwirkung oder Dochtwirkung von der Flüssigkeit durchströmt wird, dann an die Flüssigkeit abgegeben wird.

Eine erfindungsgemäße Aerosolerzeugungsvorrichtung gemäß einer weiteren Ausführungsform, der eine eigenständige Bedeutung zukommt, umfasst einen Behälter mit einem darin ausgebildeten Tank zum Speichern einer zu versprühenden Flüssigkeit, wobei der Behälter eine Auslassöffnung aufweist, einen Ultraschallgenerator, mittels dessen das Aerosol aus der Flüssigkeit erzeugbar ist, ein an dem Behälter angrenzend zu dessen Auslassöffnung angebrachtes Mundstück, das eine Austrittsöffnung zum Freisetzen des von dem Ultraschallgenerator erzeugten Aerosols aufweist, und einen in der Auslassöffnung des Behälters angeordneten Docht zum Transport der Flüssigkeit aus dem Tank zum Ultraschallgenerator, wobei der Docht ein im Inneren des Tanks angeordnetes Einlassende zur Aufnahme der in dem Tank gespeicherten Flüssigkeit und ein angrenzend zum Ultraschallgenerator angeordnetes Auslassende zur Bereitstellung der Flüssigkeit für den Ultraschallgenerator aufweist. Der Ultraschallgenerator ist zwischen der Austrittsöffnung des Mundstücks und dem Docht angeordnet ist. In dem Mundstück ist eine Aerosolkammer ausgebildet, die sich zwischen der Austrittsöffnung des Mundstücks und dem Ultraschallgenerator befindet, so dass das vom Ultraschallgenerator erzeugte Aerosol in die Aerosolkammer abgegeben wird. Des Weiteren ist an oder in dem Mundstück zumindest ein Bypass ausgebildet, durch den eine Fluidverbindung zwischen der Aerosolkammer und einem Außenbereich des Mundstücks gebildet ist, wobei zumindest ein aromatisiertes Element mit einer vorbestimmten Geschmacksrichtung an dem Mundstück strömungstechnisch zwischen der Austrittsöffnung und dem Bypass des Mundstücks angeordnet ist.

Gemäß einer weiteren Ausführungsform der vorliegenden Erfindung, der eine eigenständige Bedeutung zukommt, ist auch ein Verfahren zum Erzeugen eines Aerosols unter Verwendung einer Aerosolerzeugungsvorrichtung mit einem Mundstück vorgesehen, wobei es sich bei hierbei verwendeten Aerosolerzeugungsvorrichtung um eine der vorstehend genannte erfindungsgemäßen Vorrichtungen handeln kann. Jedenfalls ist bei dem erfindungsgemäßen Verfahren vorgesehen, dass zusammen mit der Aerosolerzeugungsvorrichtung zumindest ein insbesondere austauschbares aromatisiertes Element mit einer vorbestimmten Geschmacksrichtung bereitgestellt und an dem Mundstück angebracht wird, derart, dass eine Luftströmung, die vom Anwender zusammen mit dem erzeugten Aerosol inhaliert wird, vor einer Inhalation durch den Anwender an dem aromatisierten Element vorbeiströmt und/oder durch das aromatisierte Element hindurchströmt und dadurch die Luftströmung zusammen mit dem erzeugten Aerosol die vorbestimmte Geschmacksrichtung des aromatisierte Elements annimmt.

Der zuletzt genannten Ausführungsform der Erfindung liegt die wesentliche Erkenntnis zugrunde, dass es mittels eines aromatisierten Elements, welches über eine vorbestimmte Geschmacksrichtung verfügt, in einfacher Weise möglich ist, dem erzeugten Aerosol diese vorbestimmte Geschmacksrichtung zu verleihen bzw. hinzuzufügen. Dies kann erfindungsgemäß in einfacher Weise dadurch erreicht werden, dass das besagte aromatisierte Element mit der vorbestimmten Geschmacksrichtung an dem Mundstück der Aerosolerzeugungsvorrichtung, welche Gegenstand der vorliegenden Erfindung ist oder zur Durchführung des erfindungsgemäßen Verfahrens eingesetzt wird, angebracht wird. Durch den Bypass, der an oder in dem Mundstück ausgebildet ist, wird dann erreicht, dass der Anwender beim Inhalieren des erzeugten Aerosols stets auch Frischluft von außerhalb des Mundstücks mitansaugt, wobei diese Frischluft dabei an dem aromatisierten Element vorbeiströmt und/oder durch das aromatisierte Element hindurchströmt und dadurch die Luftströmung zusammen mit dem erzeugten Aerosol die vorbestimmte Geschmacksrichtung des aromatisierten Elements annimmt.

Der Behälter der erfindungsgemäßen Aerosolerzeugungsvorrichtung verfügt über einen Tank zum Speichern oder Halten oder Lagern einer Flüssigkeit, die aerosolisiert werden soll. Der Docht transportiert die Flüssigkeit aus dem Tank (d.h. die im Tank gespeicherte Flüssigkeit) zum Ultraschallgenerator. Der Docht hat ein Einlassende, um die Flüssigkeit aus dem Tank in den Docht aufzunehmen. Das Einlassende ist im Inneren des Behälters, d.h. im Tank, zur Aufnahme der Flüssigkeit angeordnet. Der Docht hat ein Auslassende, das keinen direkten Kontakt mit dem Ultraschallgenerator hat. Das Auslassende des Dochts versorgt den Ultraschallgenerator durch seine Kapillare mit der Flüssigkeit. Am Ende einer Kapillare bildet sich ein bogenförmiger Wassertropfen, wenn die Oberflächenspannung des Wassers die Kapillarität überwiegt. Die Oberflächenspannung entsteht aufgrund der Anziehungskräfte zwischen den Wassermolekülen an der Grenzfläche zwischen dem Wasser und der Luft. In der Kapillare steigt das Wasser aufgrund der Kapillarität aufgrund der Adhäsion (Anziehungskraft zwischen Wasser und der Kapillarwand) und Kohäsion (Anziehungskraft zwischen den Wassermolekülen selbst).

Erfindungsgemäß ist die Kapillarität des Dochtes gegenüber der Oberflächenspannung der wasserbasierten Lösung so gewählt, dass die Oberflächenspannung der wasserbasierten Lösung dominiert, wodurch sich am Ende der Kapillare ein bogenförmiger Wassertropfen bildet.

Erfindungsgemäß ist der Ultragenerator mit seinem zweiten Teil so weit vom Docht entfernt, dass der schwingende zweite Teil des Ultraschallgenerators nur in den Wassertropfen hineintaucht, ohne jedoch den Docht selbst zu berühren. Dadurch wird eine negative Frequenzbeeinflussung des Ultraschallgenerators vermieden und die Aerosolisierung kann wie gewünscht erfolgen. In Folge dessen kommt es nicht zu ungewünschten Tropfenvereinigungen im Aerosol, die dann die Inhalation zu den Alveolen in der Lunge behindern würden.

Der Ultraschallgenerator wendet Ultraschall auf die Flüssigkeit an, die vom Auslassende des Dochtes aufgenommen wird, wodurch die Flüssigkeit vernebelt und das Aerosol aus der Flüssigkeit erzeugt wird. Das Mundstück gibt das erzeugte Aerosol an eine Außenseite des Geräts ab, z. B. wenn es von einem Benutzer angesaugt wird. Der Tank, der Docht und das Mundstück definieren zusammen einen Fluidleitungspfad für den Transport der Flüssigkeit vom Tank zum Ultraschallgenerator und für den Transport des vom Ultraschallgenerator erzeugten Aerosols zum Mundstück. Der Ultraschallgenerator ist beispielsweise direkt zwischen dem Mundstück, d.h. einer Austrittsöffnung des Mundstücks, und dem Docht angeordnet. Der Fluidleitungspfad verläuft auch durch den Ultraschallgenerator.

Der Behälter weist eine Auslassöffnung auf. Der Docht ist in der Auslassöffnung derart angeordnet, dass der Docht die Auslassöffnung zumindest teilweise oder vollständig ausfüllt.

Im Sinne der vorliegenden Erfindung kann die Aerosolerzeugung auch als Vernebelung, Aerosolisierung, Zerstäubung, Vernebelung und mit ähnlichen Begriffen bezeichnet sein.

Sofern nicht anders angegeben, kann das Merkmal "in Berührung angeordnet" im Sinne der vorliegenden Erfindung die gleiche Bedeutung haben wie "in direktem Kontakt angeordnet", "in direktem physischem Kontakt angeordnet" oder "in Oberflächenkontakt angeordnet".

Sofern nicht anders angegeben, kann das Merkmal "Füllungen" im Sinne der vorliegenden Erfindung die gleiche Bedeutung haben wie "vollständig gefüllt", "vollgestopft", "eingeklemmt" bzw. "nimmt eine ganze Querschnittsfläche ein". Dabei wird die Querschnittsebene bzw. -fläche senkrecht zur Längsachse der Vorrichtung und/oder des Tanks und/oder der Führung definiert.

Die Funktionsweise der der erfindungsgemäßen Aerosolerzeugungsvorrichtung kann wie folgt verstanden werden: Die Flüssigkeit aus dem Tank wird über das Einlassende des Dochtes in den Docht aufgenommen. Die aufgenommene Flüssigkeit wandert in Folge der Kapillarwirkung oder Dochtwirkung des Dochtes durch den Docht zu seinem Auslassende. Das Auslassende des Dochts stellt die Flüssigkeit dem Ultraschallgenerator zur Verfügung, wobei es gemäß einer vorteilhaften Ausführungsform der Erfindung keinen direkten Kontakt zwischen dem Auslassende des Dochts und dem zweiten Teil des Ultraschallgenerators gibt. Jedenfalls versorgt das Auslassende des Dochts den Ultraschallgenerator durch die Kapillare mit der zu vernebelnden Flüssigkeit. Hierbei bildet sich am Ende einer Kapillare ein bogenförmiger Wassertropfen durch die Oberflächenspannung der wasserbasierten Flüssigkeit. Die Oberflächenspannung entsteht aufgrund der Anziehungskräfte zwischen den Wassermolekülen an der Grenzfläche zwischen dem Wasser und der Luft. In der Kapillare steigt das Wasser aufgrund der Kapillarität aufgrund der Adhäsion und Kohäsion. Die Kapillarität des Dochtes ist gegenüber der Oberflächenspannung der wasserbasierten Lösung so gewählt, dass die Oberflächenspannung der wasserbasierten Lösung dominiert, wodurch sich am Ende der Kapillare, d.h. am Auslassende des Dochts ein bogenförmiger Wassertropfen bildet.

Der Ultraschallgenerator erzeugt Ultraschall, z. B. wenn er von einem Benutzer des Geräts betätigt oder gesteuert oder aktiviert wird, z. B. wenn er mit elektrischer Energie aus einer Batterie oder einer Stromquelle versorgt wird. Der erzeugte Ultraschall versprüht die Flüssigkeit, die vom Auslassende des Dochtes bereitgestellt wird, wodurch das Aerosol erzeugt wird, das dann über das Mundstück an eine Außenseite des Geräts abgegeben wird. Die Zerstäubung und das anschließende Entfernen der Flüssigkeit vom Auslassende des Dochtes durch die Wirkung des Ultraschallgenerators stellt eine Saugkraft bereit oder hält eine Kapillar- oder Dochtwirkung aufrecht, bei der die Flüssigkeit aus einem Behälter in das Einlassende des Dochtes und von diesem zum Auslassende des Dochtes geleitet oder transportiert wird.

Gemäß einer vorteilhaften Ausführungsform der Erfindung kann der Docht beabstandet von der Oberfläche des Ultraschallgenerators angeordnet sein und hierbei räumlich so verschlossen sein, dass außer der Flüssigkeit, die durch den Docht transportiert wird, keine weitere Flüssigkeit in den Ultraschallgenerator hinein gelangen kann. Anders ausgedrückt, kann die Flüssigkeit den Ultraschallgenerator ausschließlich über das Kapillarelement bzw. den Docht erreichen.

Gemäß einer vorteilhaften Weiterbildung der Erfindung ist der Behälter, bis auf seine Auslassöffnung, flüssigkeits- bzw. fluiddicht ausgebildet, so dass die Flüssigkeit aus dem Tank nur über den Docht zum Ultraschallgenerator transportiert wird. Hiermit wird der Vorteil erreicht, dass unerwünschte Leckagen der Flüssigkeit aus dem Behälter heraus vermieden werden, und dass die in dem Tank des Behälters gespeicherte Flüssigkeit ausschließlich durch den Docht hindurch zum Ultraschallgenerator zu gelangen. Die Ansammlung von Flüssigkeit, die sich bedingt durch deren Oberflächenspannung an dem Auslassende des Dochts bildet, gewährleistet dann eine Aufnahme von Flüssigkeit durch den zweiten Teil des Ultraschallgenerators, wenn dieser zweite Teil in Schwingung versetzt wird und sich dabei in Richtung des Auslassendes des Dochts verformt.

Gemäß einer vorteilhaften Weiterbildung der Erfindung kann vorgesehen sein, dass der Docht mit seinem Auslassende sich in Kontakt mit dem ersten Teil des Ultraschallgenerators befindet. Für diesen Fall kann es zweckmäßig sein, dass der Docht durch eine Druckfeder entlang seiner Längserstreckung in Richtung des Ultraschallgenerators unter Vorspannung gesetzt ist, derart, dass damit der Docht mit seinem Auslassende gegen den ersten Teil des Ultraschallgenerators gedrückt wird. Hiermit wird erfindungsgemäß der Vorteil erreicht, dass die Ansammlung von Flüssigkeit, die sich in Folge von deren Oberflächenspannung an dem Auslassende des Dochts bildet, sich tatsächlich nur in Gegenüberstellung zum zweiten Teil des Ultraschallgenerators befindet, wobei ein ggf. unkontrolliertes Wegfließen von Flüssigkeit zur Seite hin nicht möglich ist. Anders ausgedrückt, wird durch den Kontakt des Dochts mit dem ersten Teil des Ultraschallgenerators eine Dichtwirkung zur Seite hin erzielt, so dass sich die Ansammlung von Flüssigkeit an dem Auslassende des Dochts tatsächlich nur zentrisch gegenüber dem zweiten Teil des Ultraschallgenerators befindet. Damit ist eine wirkungsvolle Aufnahme von Flüssigkeit in den Durchgängen des zweiten Teils des Ultraschallgenerators gewährleistet, wenn sich dieser zweite Teil im Schwingungszustand in Richtung des Dochts verformt und dabei wie erläutert in die Flüssigkeit eintaucht.

Gemäß einer vorteilhaften Weiterbildung der Erfindung kann die vorstehend genannte Federvorspannung für den Docht dadurch erreicht werden, dass die Druckfeder in axialer Ausrichtung zum Docht angeordnet ist und an einer Stirnseite des Dochts anliegt, an der dessen Einlassende ausgebildet ist. Anders ausgedrückt, ist die Druckfeder an dem Docht an dessen unteren Stirnseite angeordnet, die entgegensetzt zum Ultraschallgenerator angeordnet ist. Beispielsweise kann die Druckfeder sich hierbei an einem Bodenbereich des Behälters bzw. an einem zugehörigen Deckel abstützen, so dass dadurch die Federkraft auf den Docht in Richtung des Ultraschallgenerators ausgeübt wird.

Die vorstehend genannte Federvorspannung für den Docht führt zu dem Vorteil, dass der Docht stets an dem ersten Teil des Ultraschallgenerators dichtend anliegt. Dies bedingt einerseits eine Dichtwirkung in Bezug auf das Auslassende des Dochts, so dass ein ggf. unkontrolliertes Wegfließen bzw. -strömen der Flüssigkeit, die sich in Folge ihrer Oberflächenspannung an dem Auslassende des Dochts ansammelt, zur Seite hin nicht möglich ist. Des Weiteren bewirkt die besagte Federvorspannung, dass die Schwingungen des Ultraschallgenerators, die durch dessen ersten Teil induziert werden, von dem Docht "begleitet" werden können. Dies bedeutet, dass diese Schwingungen des ersten Teils ggf. auf den Docht übertragen werden können, ohne dass dabei der Kontakt zwischen dem Docht und dem ersten Teil des Ultraschallgenerators abreißt. Sprich: Im Verlauf der Schwingungen des ersten Teils des Ultraschallgenerators wird der Docht mit seinem Auslassende in Folge der Federvorspannung der Druckfeder fortwährend gegen den ersten Teil des Ultraschallgenerators gedrückt, wobei bei einer Übertragung der Schwingungen von dem ersten Teil des Ultraschallgenerators auf den Docht dessen Kontakt mit dem ersten Teil des Ultraschallgenerators erhalten bleibt bzw. nicht verloren geht. Insoweit bleibt die erläuterte Dichtwirkung, die durch den Kontakt des Dochts mit dem ersten Teil des Ultraschallgenerators erzielt wird, auch im Schwingungszustand unverändert bestehen.

Der Docht kann aus gewebtem oder nicht gewebtem Gewebe, wie z. B. Baumwolle, bestehen.

Gemäß einer vorteilhaften Weiterbildung der Erfindung kann der Docht folgende Merkmale aufweisen:
- Der Docht kann eine faserige Struktur oder eine schwammige Struktur haben.
- Der Docht kann Materialien auf Keramik- oder Graphitbasis enthalten.
- Der Docht kann mindestens eine der Celluloseacetat-, Polyester-, Polyolefin-, Polyethylen-, Polypropylen- oder Nylonfasern enthalten.
- Der Docht kann auch aus pflanzlichen und tierischen Naturfasern besteht, wie z.B. Baumwolle, Leinen, Hanf, Seide oder Wolle.

Jegliche Leitung oder Bewegung oder Bewegung oder Transport der Flüssigkeit durch den Docht und/oder in dem Docht und/oder innerhalb des Dochtes kann auf Dochtwirkung oder Kapillarwirkung zurückzuführen sein, z. B. wenn sie durch eine Aerosolisierung der Flüssigkeit am Auslassende des Dochtes durch den vom Ultraschallgenerator erzeugten Ultraschall erzwungen oder durchgeführt oder bewirkt wird.

Ein Material des Dochtes kann derart sein, dass Kapillaren des Dochtes die Flüssigkeit halten, so dass die Flüssigkeit nicht von selbst entweicht, d. h. in Abwesenheit der Dochtkraft, die durch den Betrieb (d. h. die Ultraschallerzeugung) des Ultraschallgenerators erzeugt wird. Nur durch die Schwingungen des Ultraschallgenerators kann die Flüssigkeit im Docht aus den Kapillaren des Dochtes entweichen.

Eine Position und/oder Ausrichtung des Dochtes kann in Bezug auf den Behälter lokalisiert oder festgelegt sein, insbesondere in Bezug auf die Auslassöffnung des Behälters, beispielsweise, aber nicht ausschließlich, mittels einer Presspassung oder einem Presssitz.

Der vorstehend genannte Fluidleitungspfad kann im Sinne der vorliegenden Erfindung derart verstanden werden, dass er mindestens zwei Abschnitte umfasst oder Teile oder Segmente - einen ersten Abschnitt oder ein Segment, das als Flüssigkeitstransportabschnitt oder -segment bezeichnet werden kann, und einen zweiten Abschnitt oder ein zweites Segment, das als Aerosoltransportabschnitt oder -segment bezeichnet werden kann.

Der Flüssigkeitstransportabschnitt kann zumindest durch den Docht definiert sein, beispielsweise durch das Einlassende des Dochtes, das Auslassende des Dochtes und den dazwischen liegenden Teil oder mittleren Teil des Dochtes (d. h. Teil des Dochtes, der sich zwischen dem Einlassende und dem Auslassende des Dochtes erstreckt). Über die Flüssigkeitstransportstrecke wird die Flüssigkeit aus dem Tank zum Ultraschallgenerator transportiert. Der Flüssigkeitstransportabschnitt erstreckt sich vom Tank bis zum Ultraschallgenerator.

Der Aerosoltransportabschnitt kann zumindest durch einen Raum oder eine Kammer oder einen Kanal zwischen dem Ultraschallgenerator und einer Austrittsöffnung (d.h. einer Abflussöffnung) des Mundstücks definiert sein. Über die Aerosoltransportstrecke wird das am und durch den Ultraschallgenerator erzeugte Aerosol zur Austrittsöffnung des Mundstücks transportiert.

Die Austrittsöffnung kann eine Öffnung des Mundstücks sein, durch die bzw. über die das Aerosol schließlich aus dem Mundstück und/oder der Aerosolerzeugungsvorrichtung aus- oder abgegeben wird, beispielsweise zu einem Benutzer.

Der Flüssigkeitstransportabschnitt und der Aerosoltransportabschnitt sind miteinander verbunden oder überlappen sich kontinuierlich oder teilweise, wobei diese beiden Abschnitte zusammen den Fluidleitpfad bilden.

Der Fluidleitungspfad verläuft durch den Ultraschallgenerator hindurch. Der Ultraschallgenerator kann eine Aufnahmefläche (auch als erste Oberfläche oder Unterseite oder dochtseitige Oberfläche oder Bodenfläche bezeichnet) und eine Austrittsfläche (auch als zweite Oberfläche oder Oberseite oder Entladungsöffnung zugewandte Fläche oder obere Oberfläche bezeichnet) aufweisen.

Die Aufnahmefläche und die Austrittsfläche des Ultraschallgenerators können umgedrehte Seiten oder Flächen, d.h. entgegengesetzte Seiten oder Oberflächen des Ultraschallgenerators sein.

Die Aufnahmefläche des Ultraschallgenerators ist dem Docht zugewandt. Gemäß einer vorteilhaften Ausführungsform der Erfindung steht diese Aufnahmefläche nicht im direkten physikalischen Kontakt (Oberflächenkontakt) mit dem Docht, d. h. mit dem Auslassende des Dochtes.

Die Aufnahmefläche des Ultraschallgenerators nimmt, wenn der Ultraschallgenerator in Schwingung versetzt wird, die Flüssigkeit von einem bogenförmigen Tropfen auf, der sich auf bzw. an dem Auslassende des Dochtes bildet.

Die Austrittsfläche des Ultraschallgenerators ist der Austrittsöffnung des Mundstücks zugewandt.

Das am und durch den Ultraschallgenerator erzeugte Aerosol verlässt den Ultraschallgenerator von der Austrittsfläche in Richtung der Austrittsöffnung des Mundstücks.

Der Ultraschallgenerator kann so konfiguriert werden, dass er mit einer Frequenz in einem Bereich von 1,4 kHz bis 3 MHz betrieben wird.

Die Aerosolerzeugungsvorrichtung, insbesondere der Ultraschallgenerator, kann so konfiguriert sein, dass sie Aerosol aus einer Flüssigkeit auf Wasserbasis (d. h. einer Flüssigkeit, die 80 Gew.-% Wasser oder mehr enthält) erzeugt, wobei das Aerosol Tröpfchen aufweist mit einer Größe oder einer mittleren Größe von 10 µm oder weniger, insbesondere kleiner oder gleich 5 µm, und/oder gleich oder größer als 1 µm, insbesondere größer oder gleich 2 µm, und/oder ohne Erwärmung mindestens eines der Fluide, der Flüssigkeit und des Aerosols (d.h. durch Aufrechterhaltung einer Temperatur der Flüssigkeit kann eine Temperatur des Aerosols der Temperatur der Flüssigkeit entsprechen). Das Aerosol kann eine Vielzahl von Tröpfchen mit einer Größenverteilung mit einem Mittelwert oder Mittelwert kleiner oder gleich 10 µm, insbesondere kleiner oder gleich 5 µm umfassen.

Der Ultraschallgenerator kann einen ersten Teil, beispielsweise ein piezoelektrisches oder piezokeramisches Teil oder Element, und einen zweiten Teil, beispielsweise ein Netz- oder Metallgitterteil, oder ein Teil mit einer Vielzahl von darin ausgebildeten Durchgängen oder ein perforiertes Teil, aufweisen. Mit anderen Worten kann der Ultraschallgenerator einen ersten Teil, der ein piezoelektrisches Element umfasst, und einen zweiten Teil aufweisen, der eine Vielzahl von Durchgängen (beispielsweise ein Netz oder eine Lochplatte) hat, um das Fluid hindurchzulassen.

An dieser Stelle wird nochmals gesondert hervorgehoben, dass erfindungsgemäß das Auslassende des Dochtes mit dem zweiten Teil des Ultraschallgenerators nicht in Kontakt steht. In Folge dessen steht das Auslassende des Dochtes auch nicht in Kontakt mit den Durchgängen, die in dem zweiten Teil des Ultraschallgenerators ausgebildet sind. Hierdurch wird erfindungsgemäß der Vorteil erreicht, dass die Schwingungen dieses zweiten Teils des Ultraschallgenerators im Betrieb der Aerosolerzeugungsvorrichtung nicht durch den Docht beeinflusst oder beeinträchtigt werden.

Der zweite Teil des Ultraschallgenerators kann durch dessen ersten Teil in Schwingung versetzt werden.

Der zweite Teil des Ultraschallgenerators kann scheibenförmig oder kreisförmig sein. Ein Durchmesser des zweiten Teils des Ultraschallgenerators kann gleich oder größer als ein Durchmesser der Auslassöffnung des Behälters und/oder als ein Durchmesser des Auslassendes des Dochtes sein. Wie bereits erläutert, (welches, wie vorstehend bereits erläutert, sich nicht in Kontakt mit dem zweiten Teil befindet) sein.

Der zweite Teil des Ultraschallgenerators kann eine ebene Form haben.

Alternativ hierzu ist es möglich, dass der zweite Teil des Ultraschallgenerators, wie vorstehend bereits genannt, einen konkaven Abschnitt aufweist. Dies bedeutet, dass dieser konkave Abschnitt des zweiten Teils des Ultraschallgenerators eine gekrümmte Form aufweist, die sich weg von dem Docht erstreckt. Mittels dieses konkaven Abschnitts des zweiten Teils wird der gewünschte Abstand zwischen dem Auslassende des Dochts und dem zweiten Teil des Ultraschallgenerators, in dem die Vielzahl von Durchgängen ausgebildet sind und im Betrieb der erfindungsgemäßen Aerosolerzeugungsvorrichtung in Schwingung versetzt wird, erreicht.

Die Form des Auslassendes des Dochtes kann auch eine gekrümmte Form aufweisen.

Eine Form des Dochtes am Auslassende, d. h. die Form des Auslassendes des Dochtes, kann der Form des zweiten Teils des Ultraschallgenerators entsprechen. Wenn beispielsweise der zweite Teil des Ultraschallgenerators eine planare Form hat, kann die Form des Auslassendes des Dochtes ebenfalls planar sein. Alternativ hierzu kann vorgesehen sein, dass der zweite Teil des Ultraschallgenerators eine gekrümmte Form aufweist, die in Richtung des Dochtes oder in Bezug auf den Docht hervorsteht - beispielsweise eine konkave oder konvexe Form, wie z. B. eine konkave oder konvexe Kuppelform.

Beispiel: Eine konkave oder konvexe Form, z. B. eine konkave oder konvexe Kuppelform. Einfach ausgedrückt können die Form des Auslassendes des Dochtes und die Form des zweiten Teils des Ultraschallgenerators aneinander angepassst sein oder übereinstimmen oder einander entsprechen oder sich ähneln oder ergänzen. Jedoch sollte ein direkter Kontakt zwischen dem Auslassende des Dochtes und zumindest dem zweiten Teil des Ultraschallgenerators unterbunden sein.

Der zweite Teil des Ultraschallgenerators kann vom ersten Teil umgeben sein.

Der erste Teil des Ultraschallgenerators kann eine ringförmige Form aufweisen, beispielsweise eine ringförmige Kreis- oder Scheibenform, und der zweite Teil kann an oder in der Mitte der ringförmigen Form angeordnet sein.

Die Aufnahmefläche und die Austrittsfläche des Ultraschallgenerators können Oberflächen von dessen zweiten Teil sein.

Die Aufnahmefläche und die Austrittsfläche des Ultraschallgenerators können über das Gewebe oder die Durchgänge oder die Perforationen des zweiten Teils in Fluidverbindung miteinander stehen.

Der Fluidleitungspfad kann durch den zweiten Teil des Ultraschallgenerators, d. h. durch das Gewebe oder die Durchgänge oder die Perforationen des zweiten Teils von der Aufnahmefläche zur Austrittsfläche des Ultraschallgenerators verlaufen. Mit anderen Worten können die Durchgänge oder das Netz oder die Perforationen einen Teil oder ein Segment oder einen Abschnitt des Fluidleitungspfades bilden.

Ein Abschnitt oder ein Segment des Fluidleitungspfades, d. h. der Abschnitt, der in oder innerhalb oder durch den Ultraschallgenerator angeordnet ist, kann von dem ersten Teil des Ultraschallgenerators umgeben sein.

Der erste Teil des Ultraschallgenerators kann eine ringförmige Form aufweisen, beispielsweise eine ringförmige Kreis- oder Scheibenform, und der Fluidleitungspfad kann sich erstrecken oder durch die Mitte der ringförmigen Form verlaufen.

Der Docht ist in der Auslassöffnung des Behälters so angeordnet, dass der Docht die Auslassöffnung zumindest teilweise ausfüllt oder vollständig ausfüllt.

Der Docht kann die Auslassöffnung derart ausfüllen, dass damit der Behälter in Bezug auf die darin gespeicherte Flüssigkeit auslaufsicher bzw. dicht ist.

Der Behälter kann auslaufsicher sein, wenn der Docht in der Auslassöffnung angeordnet ist und diese ausfüllt.

Der Docht kann die Auslassöffnung des Behälters derart ausfüllen, dass ein Kontaktbereich zwischen dem Docht und der Öffnung so auslaufsicher ist, dass der Behälter auslaufsicher und somit dicht ist.

Der Docht kann mindestens zwei Abschnitte oder Teile oder Segmente aufweisen, einen inneren oder ersten Abschnitt des Dochtes, der aus der Auslassöffnung heraus und in den Tank hineinragend angeordnet ist, und einen Öffnungsabschnitt oder zweiten Abschnitt des Dochtes, der in oder innerhalb der Auslassöffnung angeordnet ist (d. h. nicht aus einer beliebigen Seite der Auslassöffnung herausragt oder einfach begrenzt oder bündig mit der Außen- und Innenfläche des Behälters angeordnet ist).

Die beiden Abschnitte oder Teile oder Segmente des Dochtes können einstückig ausgebildet sein.

Zusätzlich kann der Docht optional einen dritten Abschnitt oder einen Teil oder ein Segment aufweisen - einen äußeren Abschnitt des Dochtes, der aus der Auslassöffnung herausragend zu einer Außenseite des Behälters angeordnet ist, d.h. außerhalb des Tanks und außerhalb der Auslassöffnung angeordnet ist.

Beim Einführen des Dochts in die Auslassöffnung kann der Docht in die Auslassöffnung hinein gepresst werden, d. h. durch Wände oder Flächen (des Behälters), welche die Auslassöffnung definieren, wobei der Docht somit die Querschnittsform und/oder -größe (z. B. Durchmesser) der Auslassöffnung annimmt.

Eine Standardquerschnittsform des Dochtes, z. B. des Öffnungsabschnitts des Dochtes, und eine Querschnittsform der Auslassöffnung können gleich sein, z. B. können beide eine kreisförmige Form haben. Alternativ kann eine Standard-querschnittsform des Dochtes, z. B. des Öffnungsabschnitts des Dochtes, und eine Querschnittsform der Auslassöffnung unterschiedlich sein, und der Docht wird beim Einsetzen in die Auslassöffnung in die Auslassöffnung gedrückt und nimmt somit die Querschnittsform der Auslassöffnung an. Eine Standardquerschnittsform des Dochtes kann als eine Querschnittsform des Dochtes verstanden werden, bevor er in die Auslassöffnung eingeführt wird.

Eine Standardquerschnittsfläche des Dochtes, z. B. des Öffnungsabschnitts des Dochtes, kann größer sein als die Querschnittsfläche der Auslassöffnung. Eine voreingestellte Querschnittsfläche des Dochtes kann als eine Querschnittsfläche des Dochtes verstanden werden, bevor er in die Auslassöffnung eingeführt wird. Die Auslassöffnung kann vollständig mit dem Docht gefüllt oder durch den Docht gepacktvollgestopft sein. Mit anderen Worten wird die gesamte Querschnittsfläche der Auslassöffnung von dem Docht eingenommen, so dass die Flüssigkeit im Tank durch die Auslassöffnung nur über oder durch den Docht strömt. Einfach ausgedrückt, ist der Docht durch oder in der Auslassöffnung derart angeordnet, dass in der Auslassöffnung kein Spalt oder Luftspalt oder räumlicher Spalt verbleibt, d. h. zwischen einer Außenfläche (z. B. einer äußeren Umfangsfläche) des Dochtes und einer die Auslassöffnung definierenden Oberfläche des Behälters. Da zwischen der Außenfläche des Dochtes und der Oberfläche des Behälters, welche die Auslassöffnung definiert, kein Raum oder Kanal oder Spalt vorhanden ist, wird jeder unbeabsichtigte Durchfluss von Flüssigkeit oder Dampf aus dem Behälter heraus vermindert oder unterbunden.

Der Docht kann in der Auslassöffnung eingebracht oder eingepresst sein, beispielsweise in der Weise, dass die gesamte umlaufende Fläche der Auslassöffnung in direktem Kontakt (oder in Flächen-zu-Fläche-Kontakt) mit einer äußeren Umfangsfläche des Dochtes steht.

Mit Ausnahme seiner Auslassöffnung kann der Behälter, beispielsweise der durch den Behälter definierten Tank, flüssigkeitsdicht oder hermetisch verschlossen oder fluiddicht sein, insbesondere für den Durchfluss von Flüssigkeit von einem Inneren des Behälters (d. h. von dem Tank) zu einer Außenseite des Behälters (d. h. außerhalb einer Außenfläche des Behälters). Vereinfacht ausgedrückt, kann der Behälter, bis auf seine Auslassöffnung, flüssigkeitsdicht oder hermetisch oder fluiddicht verschlossen sein, so dass die Flüssigkeit aus dem Tank nur über den in der Auslassöffnung angeordneten Docht zum Ultraschallgenerator transportiert wird.

Die Auslassöffnung des Behälters kann die einzige Austritts- oder Abflussöffnung aus dem Tank zu einer Außenseite des Behälters sein.

Zusätzlich und optional kann der Docht einen festen Körper aufweisen, der so konfiguriert ist, dass er eine Kapillarwirkung oder Dochtwirkung entfaltet.

Eine Innenfläche des Behälters definiert den Tank. Dieser Tank kann ein Volumen von 3 ml oder weniger, insbesondere von 2 ml oder weniger, haben.

Gemäß einer vorteilhaften Ausführungsform kann der Behälter eine Führung aufweisen, die rohrförmig ausgebildet ist und sich von der Auslassöffnung des Behälters in den Tank hinein erstreckt. Die Führung und der Behälter können einstückig ausgebildet sein. Zweckmäßigerweise kann hierbei ein Innendurchmesser der Führung gleich einem Durchmesser der Auslassöffnung des Behälters sein

Die Führung kann einen Aufnahmeraum aufweisen. Der Docht ist in dem Aufnahmeraum aufgenommen.

Die Führung (einschließlich des Aufnahmeraums) darf nur einen Teil des Tanks einnehmen. Zum Beispiel kann die Führung zwischen 10 und 90 Prozent des Gesamtvolumens des Tanks einnehmen, oder zwischen 20 und 80 Prozent des Gesamtvolumens des Tanks, oder zwischen 30 und 70 Prozent des Gesamtvolumens des Tanks oder zwischen 10 und 30 Prozent des Gesamtvolumens des Tanks.

Der Aufnahmeraum kann durchgehend mit der Auslassöffnung verbunden sein und/oder in Fluidverbindung mit der Auslassöffnung stehen.

Der Aufnahmeraum kann sequentiell mit der Auslassöffnung ausgebildet sein.

Die Auslassöffnung kann vorzugsweise ausschließlich über den Aufnahmeraum in Fluidverbindung mit dem Tank stehen.

Der Aufnahmeraum kann konzentrisch und/oder koaxial mit der Auslassöffnung ausgebildet sein.

Ein Querschnittsbereich des Aufnahmeraumes kann durch den Docht ausgefüllt werden. Die Querschnittsfläche des Aufnahmeraums kann gleich oder kleiner als eine Querschnittsfläche der Austrittsöffnung sein. Zusätzlich oder alternativ kann die Querschnittsfläche des Aufnahmeraums entlang einer Längsachse davon konstant sein.

Ein gesamtes oder vollständiges Innenvolumen der Führung kann durch den Docht gefüllt oder vollständig gefüllt oder gepackt sein.

Der Aufnahmeraum kann durch den Docht vollständig oder vollständig (im Querschnitt und in Längsrichtung) oder vollständig gepackt sein.

Die standardmäßige Querschnittsform des Dochtes, z. B. des inneren Abschnitts des Dochtes, und eine Querschnittsform des Aufnahmeraums können identisch sein, z. B. können beide eine kreisförmige Form haben. Alternativ können die Standard-Querschnittsform des Dochtes, z. B. des inneren Abschnitts des Dochtes, und eine Querschnittsform des Aufnahmeraums unterschiedlich sein, und der Docht kann, wenn er in den Aufnahmeraum eingeführt wird, durch eine Umfangswand (die den Aufnahmeraum definiert) der Führung gedrückt werden und somit die Querschnittsform des Aufnahmeraums annehmen.

Die Standard-Querschnittsfläche des Dochtes, z. B. des inneren Abschnitts des Dochtes, kann größer sein als die Querschnittsfläche des Aufnahmeraums.

Mit anderen Worten kann, mit Ausnahme der Auslassöffnung und des damit verbundenen Aufnahmeraums, der Behälter, beispielsweise der durch den Behälter definierte Tank, flüssigkeitsdicht oder hermetisch verschlossen oder fluiddicht sein.

Mit Ausnahme der Auslassöffnung und des damit verbundenen Aufnahmeraums kann der Behälter, beispielsweise der durch den Behälter definierte Tank, flüssigkeitsdicht oder hermetisch verschlossen oder fluiddicht sein, insbesondere für den Durchfluss von Flüssigkeit aus dem Inneren des Behälters (d. h. aus dem Tank) zur Außenseite des Behälters (d. h. außerhalb einer Außenfläche des Behälters).

Die Auslassöffnung und der mit ihr verbundene Aufnahmeraum können den einzigen Ausgang aus dem Tank zur Außenseite des Behälters definieren oder bilden.

Die Auslassöffnung und/oder der Aufnahmeraum können vollständig mit dem Docht gefüllt sein, oder durch den Docht vollgestopft oder gepackt sein. Mit anderen Worten wird die gesamte Querschnittsfläche der Auslassöffnung und/oder des Aufnahmeraums von dem Docht eingenommen, so dass die Flüssigkeit in dem Tank nur über den Docht oder durch ein Inneres des Dochtes oder durch einen Körper des Dochtes oder durch Kapillarkanäle oder Zwischenräume des Dochtes durch die Auslassöffnung und/oder den Aufnahmeraum strömt. Vereinfacht ausgedrückt, kann der Docht durch oder in der Auslassöffnung und/oder dem Aufnahmeraum derart angeordnet sein, dass kein Spalt oder Luftspalt oder Luftkanal in der Auslassöffnung und/oder im Aufnahmeraum verbleibt zwischen einer Außenfläche, z. B. einer äußeren Umfangsfläche, des Dochtes und der Oberfläche des Behälters, die die Auslassöffnung definiert, und/oder der Umfangswand der Führung, die den Aufnahmeraum definiert.,. Da es keinen Raum oder Kanal oder Spalt zwischen der Außenfläche des Dochtes und der Oberfläche des Behälters gibt, der die Auslassöffnung definiert, und/oder der Umfangswand der Führung, die den Aufnahmeraum definiert, wird jede unbeabsichtigte Strömung von Flüssigkeit oder Dampf aus dem Behälter vermindert oder unterbunden.

Vereinfacht ausgedrückt, kann der Behälter bis auf die Auslassöffnung und den damit verbundenen Aufnahmeraum flüssigkeitsdicht sein, so dass die Flüssigkeit aus dem Tank nur bzw. ausschließlich über den durch den Aufnahmeraum und die Auslassöffnung angeordneten Docht zum Ultraschallgenerator transportiert wird.

Die Führung kann ein Einlassende mit einem Einlass- oder Einlassloch aufweisen. Das Einlassende des Dochtes kann über den Einlass der Führung in Fluidverbindung mit dem Tank stehen.

Die Führung kann eine umlaufende Wand aufweisen, die sich zwischen dem Einlassende der Führung und einem Auslassende der Führung erstreckt. Das Auslassende der Führung kann einen Auslass oder ein Auslassloch aufweisen, die mit der Auslassöffnung in Verbindung steht. Der Auslass der Führung kann den gleichen Durchmesser und/oder die gleiche Querschnittsfläche wie die Auslassöffnung des Behälters haben.

Das Auslassende der Führung kann mit der Innenfläche des Behälters verbunden oder gekoppelt sein. Die Kupplung kann fluiddicht oder flüssigkeitsdicht sein.

Die umlaufende Wand der Führung kann derart flüssigkeitsdicht sein, dass die Flüssigkeit im Aufnahmeraum nur über den Einlass der Führung in den Docht eintritt.

Zwischen der Führung, d. h. dem Einlassende der Führung, und der Innenfläche des Behälters kann ein Spalt definiert sein. Die in dem Tank gespeicherte Flüssigkeit wird über den Spalt in den Docht aufgenommen.

Der Docht kann derart angeordnet sein, dass er aus der Führung, d. h. aus dem Einlassende der Führung, in den Tank hineinragt.

Der Docht kann derart angeordnet sein, dass er aus der Führung, d. h. aus dem Einlassende der Führung, in den Spalt hineinragt.

Die Führung kann den Aufnahmeraum des Behälters hermetisch von dem Ultraschallgenerator trennen, außer durch den Aufnahmeraum.

Der Docht kann aus oder von der Auslassöffnung zum Ultraschallgenerator herausragen. Dies bedeutet, dass die Auslassöffnung von dem Ultraschallgenerator beabstandet sein kann. Der Docht kann aus der Auslassöffnung herausragen und über eine Außenfläche des Behälters hinausragen. Mit anderen Worten, der hervorstehende Abschnitt oder der äußere Abschnitt oder ein Teil des Kapillarelements bzw. des Dochts kann sich vollständig außerhalb des Behälters befinden.

Eine Länge der Führung, die sich von der Auslassöffnung des Behälters hinein in den Tank erstreckt, kann gleich oder größer als 50 Prozent einer Länge des Tanks oder gleich oder größer als 70 Prozent einer Länge des Tanks sein, kann gleich oder größer als 80 Prozent einer Länge des Tanks sein, oder kann gleich oder größer als 90 Prozent einer Länge des Tanks sein.

Eine Länge des Dochtes, die sich von der Auslassöffnung des Behälters hinein in den Tank erstreckt, kann gleich oder größer als 50 Prozent einer Länge des Tanks sein oder kann gleich oder größer als 70 Prozent einer Länge des Tanks sein, kann gleich oder größer als 80 Prozent einer Länge des Tanks oder kann gleich oder größer als 90 Prozent einer Länge des Tanks sein.

Eine (Gesamt-)Länge des Dochtes kann größer sein als eine Länge der Führung und/oder kann größer sein als eine kombinierte Länge der Führung und der Auslassöffnung. Die Länge der Führung kann von einem dem Aufnahmeraum zugewandten Rand der Austrittsöffnung gemessen werden.

Die Länge des Tanks kann von einem dem Tank zugewandten Rand der Auslassöffnung bis zu einer Bodenfläche des Tanks gemessen werden. d.h. eine Fläche der unteren Wand des Tanks, die den Tank definiert.

Die Führung kann eine Zylinderform aufweisen, z.B. die Form eines geraden Kreiszylinders, und kann unterschiedliche Querschnittsformen aufweisen, wie z.B. kreisförmig, dreieckig, polygonal.

Der Docht kann eine Zylinderform aufweisen, z. B. die Form eines geraden Kreiszylinders, und kann verschiedene Querschnittsformen aufweisen, wie z.B. kreisförmig, dreieckig, polygonal.

Der Behälter kann eine Zylinderform aufweisen, z.B. die Form eines geraden Kreiszylinders oder eines Quaders, und kann unterschiedliche Querschnittsformen aufweisen, wie z.B. kreisförmig, dreieckig, polygonal.

Der Behälter kann auch verschiedene Teile oder Abschnitte mit unterschiedlichen Formen aufweisen - wie z. B. einen ersten Teil oder ein Segment mit einer Zylinderform und einen zweiten Teil oder ein Segment mit einer kegelstumpfförmigen oder quaderförmigen oder kegelstumpfförmigen oder kegelstumpfförmigen Form.

Die Führung und/oder der Docht kann sich entlang einer Längsachse des Behälters und/oder der Aerosolerzeugungsvorrichtung erstrecken.

Die Führung und/oder der Docht kann koaxial und/oder konzentrisch mit dem Behälter und/oder mit der Aerosolerzeugungsvorrichtung sein.

Die Führung und/oder der Docht können entlang der Längsachse des Behälters und/oder der Aerosolerzeugungsvorrichtung angeordnet sein.

Die Längsachse kann eine zentrale Längsachse des Behälters und/oder der Aerosolerzeugungsvorrichtung sein.

Der Behälter kann einen Körper umfassen. Der Körper kann einteilig geformt sein, d.h. einstückig geformt, z.B. geformt oder 3-D-gedruckt in einem Teil. Alternativ kann der Körper in mehreren Teilen ausgebildet sein. Die Teile können mit einer fluiddichten Verbindung miteinander verbindbar sein, um den Körper des Behälters zu bilden. Die Teile können geformt oder 3D-gedruckt werden.

Der Behälter kann eine Einlassöffnung zum Befüllen oder Nachfüllen der Flüssigkeit in den Tank aufweisen.

Der Behälter kann einen Verschluss zum Verschließen der Einlassöffnung aufweisen.

Der Verschluss kann eine Dichtung oder ein Deckel sein, die bzw. der aus elastomerem Material, wie z. B. Gummi, gebildet ist, das von der Öffnung zum Auffüllen des Tanks gelöst werden kann. Alternativ kann der Verschluss, wenn der an der Einlassöffnung befestigt ist, absichtlich von bzw. mit einer Nachfülleinrichtung (z. B. einer Spritzennadel) durchdrungen werden, um den Tank aufzufüllen.

Der Verschluss kann ein Ventil sein, z. B. ein Einwegventil oder ein Füllventil (Füllventil oder Nachfüllventil).

Der Behälter kann eine Dichtung oder ein Dichtungselement an der Auslassöffnung und in Kontakt mit einer Außenfläche des Dochtes aufweisen. Das Dichtungselement kann innerhalb der Auslassöffnung oder außerhalb der Auslassöffnung angeordnet sein. Das Dichtungselement kann in die Auslassöffnung hineinragen. Ein Beispiel für das Dichtungselement kann eine Dichtung sein, z. B. eine Dichtung, die aus elastischem Material wie einer Gummidichtung gebildet ist.

Die Vorrichtung kann ferner ein Halteelement aufweisen, das zwischen dem Mundstück und dem Behälter angeordnet und/oder am oder auf dem Behälter angeordnet ist, um den Ultraschallgenerator zu stützen oder zu montieren oder zu fixieren.

Der Ultraschallgenerator kann auf dem Halteelement montiert oder darin aufgenommen werden.

Das Halteelement kann beispielsweise aus einem elastischen oder elastomeren Material, wie einem Silikon oder Gummi, gebildet sein und kann ein Kissen sein, um den Ultraschallgenerator zu halten und zu stützen.

Das Halteelement kann so konfiguriert sein, dass es die Schwingungen und/oder Geräusche dämpft, die von dem Ultraschallgenerator erzeugt werden, der auf den Behälter und/oder das Mundstück und/oder eine äußere Oberfläche der Vorrichtung übertragen wird.

Der Behälter kann ein Behälterverbindungselement aufweisen, und das Halteelement kann ein Halteelementverbindungselement aufweisen. Das Behälterverbindungselement und das Halteelement-Verbindungselement können einander entsprechen und können miteinander in Eingriff stehen, um den Behälter und das Halteelement mit dem darauf montierten Ultraschallgenerator zu fixieren oder zu koppeln. Die Kupplung kann lösbar oder reversibel sein.

Das Mundstück, der Ultraschallgenerator und der Docht können axial oder linear ausgerichtet, d. h. in einer geraden Linie, ausgerichtet sein.

Der Ultraschallgenerator kann z. B. in einer geraden Linie auf der Auslassöffnung des Behälters und dem Docht gestapelt werden; und das Mundstück kann z.B. in einer geraden Linie auf dem Ultraschallgenerator gestapelt werden.

In der Aerosolerzeugungsvorrichtung können eine oder mehrere der folgenden Anordnungen in einem zusammengebauten Zustand der Vorrichtung implementiert sein:
(i) Eine Längsachse des Ultraschallgenerators und eine Längsachse des Dochtes 20 können koaxial oder überlappend oder gleich sein.
(ii) Eine Längsachse des Ultraschallgenerators, eine Längsachse des Dochtes und eine Längsachse des Mundstücks können koaxial oder überlappend oder gleich sein.
(iii) Eine Längsachse des Ultraschallgenerators, eine Längsachse des Dochtes, eine Längsachse des Mundstücks und eine Längsachse des Behälters können koaxial oder überlappend oder gleich sein.
(iv) Eine jeweilige Längsachse des Ultraschallgenerators, des Dochtes und der Führung können koaxial oder überlappend oder gleich sein.
(v) Eine Längsachse des Ultraschallgenerators, eine Längsachse des Dochtes und der Führung und eine Längsachse des Mundstücks können koaxial oder überlappend oder gleich sein.
(vi) Eine Längsachse des Ultraschallgenerators, eine Längsachse des Dochtes und der Führung, eine Längsachse des Mundstücks und eine Längsachse des Behälters können koaxial oder überlappend oder gleich sein.

Gemäß einem der vorstehenden Aspekte (i) - (vi) kann die Längsachse des Ultraschallgenerators eine Längsachse des ersten Teils und des zweiten Teils des Ultraschallgenerators bedeuten. Der erste Teil und der zweite Teil können die gleiche Längsachse haben.

Gemäß einem der vorstehenden Aspekte (i) - (vi) kann die Längsachse der Führung die Längsachse des Aufnahmeraums der Führung bedeuten.

Gemäß jedem der vorstehenden Aspekte (i) - (vi) kann die Längsachse des Behälters die Längsachse des Aufnahmeraums des Behälters bedeuten.

Der Docht kann im Tank angeordnet werden, ohne dabei den Tank vollständig auszufüllen.

Ein Abschnitt oder Teil des Untervolumens oder Segments des Tanks kann frei von Docht oder absorbierendem Material sein und kann ein freier Raum oder ein freies Volumen sein, um die Flüssigkeit in freier Form oder im freien Zustand aufzunehmen und zu halten (d.h. ohne vom Docht oder einem absorbierenden Material adsorbiert oder gehalten zu werden). Dadurch kann zumindest ein Teil des Tanks ausschließlich zur Aufnahme der zu vernebelnden Flüssigkeit genutzt werden, die dann über den Docht zum Ultraschallgenerator transportiert werden kann.

Alternativ kann der Docht in dem Tank angeordnet sein und einen Querschnitt des Tanks oder den gesamten Tank ausfüllen. In einer solchen Struktur bzw. Anordnung kann optional vorgesehen sein, dass die Führung nicht enthalten ist. Mit anderen Worten kann der Docht in dem Tank so angeordnet sein, dass er einen Querschnitt des Tanks ausfüllt, d. h. mit der Innenfläche des Tanks in Kontakt steht. Die Austrittsöffnung kann einen Durchmesser und/oder eine Form und/oder eine Querschnittsfläche aufweisen, die derjenigen des Aufnahmeraums entspricht. Der Docht kann derart angeordnet sein, dass er eine Querschnittsfläche eines ersten Abschnitts des Tanks ausfüllt. Ein zweiter Teil des Tanks kann zwischen dem Docht und einem Bodenbereich des Tanks sein. Der zweite Teil ist frei von Dochtmaterial und/oder Kapillarmaterial. Alternativ kann der Docht den gesamten Tank ausfüllen.

Der Behälter kann ein Führungssegment, das den Aufnahmeraum definiert, umfassen oder vollständig durch dieses Führungssegment gebildet sein.

Der Docht kann in der Auslassöffnung angeordnet sein, wobei das Führungssegment derart angeordnet ist, dass der Docht die Auslassöffnung und/oder den Aufnahmeraum des Führungssegments ausfüllt oder vollständig ausfüllt.

Das Führungssegment kann die Flüssigkeit aufnehmen, die in den angeordneten Docht adsorbiert ist.

Das Führungssegment kann den gesamten Tank bilden.

Zusätzlich und optional kann der Behälter zusätzlich zu dem Führungssegment ein Reservoirsegment aufweisen. Das Reservoirsegment enthält den Docht nicht oder ist frei von dem Docht, d. h. es ist nicht durch den Docht gefüllt und kann entsprechend die Flüssigkeit darin aufnehmen.

Das Reservoirsegment des Tanks kann frei von dem Docht oder irgendeinem absorbierenden Material sein und kann ein freier Raum oder ein freies Volumen sein, um die Flüssigkeit in freier Form oder in freiem Zustand aufzunehmen und zu halten (d. h. ohne durch den Docht oder irgendein absorbierendes Material adsorbiert oder gehalten zu werden). Infolgedessen kann das Reservoirsegment ausschließlich zur Aufnahme der zu aerosolisierenden Flüssigkeit verwendet werden, die dann wie erläutert über den in dem Führungssegment angeordneten Docht zum Ultraschallgenerator transportiert werden kann.

Gemäß einer vorteilhaften Weiterbildung der Erfindung ist vorgesehen, dass der Tank, der Docht und das Mundstück einen Fluidleitungspfad definieren zum Transport der Flüssigkeit aus dem Tank zum Ultraschallgenerator und zum Transport des vom Ultraschallgenerator erzeugten Aerosols zum Mundstück, vorzugsweise, dass der Fluidleitungspfad durch den Ultraschallgenerator und den Behälter hindurch verläuft, weiter vorzugsweise, dass der Docht in der Auslassöffnung des Behälters angeordnet ist und die Auslassöffnung ausfüllt.

Bei der Flüssigkeit, die zusammen mit der erfindungsgemäßen Aerosolerzeugungsvorrichtung und bei dem erfindungsgemäßen Verfahren verwendet wird, kann es sich um eine wasserbasierte Nikotinflüssigkeit handeln. Jedenfalls ist für diese Flüssigkeit von Bedeutung, dass sie eine Oberflächenspannung hat, mit der sich an dem Auslassende des Dochtes eine ausreichende Ansammlung von Flüssigkeit zur Übergabe an den Ultraschallgenerator bildet.

Die Flüssigkeit kann mindestens einen zusätzlichen Bestandteil enthalten, z. B. Tabakextrakt, Nikotinextrakt, Aromastoff wie Minzextrakt, Nelkenextrakt usw.

Eine Menge Ethanol und/oder mindestens ein zusätzlicher Bestandteil in der Flüssigkeit kann gleich oder größer als 10 Gew.-% und gleich oder kleiner als 20 Gew.-% sein.

Die Flüssigkeit darf nicht eines oder mehrere oder alle der folgenden Stoffe enthalten - Glycerin, Propylenglykol und Öl. Die Flüssigkeit darf nicht mindestens eines oder alle Glycerin, Propylenglykol und Öl enthalten, vorzugsweise mit Ausnahme von ätherischen Ölen und/oder mit Ausnahme von Aromazusätzen.

Für die Aerosolisierung, d. h. das Erzeugen von Aerosol aus der Flüssigkeit, können eine Temperatur der Flüssigkeit und eine Temperatur des Aerosols gleich sein. Somit wird keine Heizwirkung durch den Ultraschallgenerator durchgeführt. Insbesondere wegen der geringen Oberflächenspannung der wässrigen Flüssigkeit mit einem Wassergehalt von 80 Gew.-% oder mehr ist keine Erhitzungswirkung erforderlich, um die Flüssigkeit in das Aerosol umzuwandeln.

Zum Zeitpunkt und/oder am Ort der Aerosolisierung, d.h. der Erzeugung von Aerosol aus der Flüssigkeit, darf keine Erhitzungswirkung durchgeführt werden.

Beim Gebrauch der erfindungsgemäßen Vorrichtung und/oder bei Durchführung des erfindungsgemäßen Verfahrens kann der Ultraschallgenerator mit einer Frequenz kleiner oder gleich 3,5 MHz (Megahertz) oder kleiner oder gleich 3 MHz, insbesondere mit einer Frequenz im Bereich von 1,4 kHz bis 3 MHz, schwingen.

Beim Gebrauch der erfindungsgemäßen Vorrichtung und/oder bei Durchführung des erfindungsgemäßen Verfahrens kann ein Durchmesser der Tröpfchen des durch den Ultraschallgenerator erzeugten Aerosols kleiner oder gleich 10 µm, insbesondere kleiner oder gleich 5 µm und/oder gleich oder größer als 1 µm sein, insbesondere größer oder gleich 2 µm.

Die Tröpfchengröße kann durch Lichtbeugung, z. B. Laserlichtbeugung, oder durch Lichtstreuung, z. B. Laserlichtstreutechnik, gemessen werden. Ein Beispiel für ein geeignetes System zur Messung der Tröpfchengröße ist Spraytec^{™} von Malvern Panalytical Ltd oder der Partikelanalysator LS 13 320 XR von Beckmann Coulter GmbH.

Das Merkmal "flüssigkeitsdicht" bedeutet im Sinne der vorliegenden Erfindung undurchlässig für jedes Fluid, einschließlich eines Gases, Dampfes oder einer Flüssigkeit, z. B. undurchlässig für die Flüssigkeit, die in dem Tank gespeichert ist, oder für den Dampf, der aus der in dem Tank gespeicherten Flüssigkeit erzeugt wird.

Das Merkmal "flüssigkeitsdicht" bedeutet im Sinne der vorliegenden Erfindung: undurchlässig für jede Flüssigkeit, z. B. undurchlässig für die Flüssigkeit, die in dem Tank gespeichert ist.

Die Ausdrücke 'stromaufwärts', 'stromabwärts', 'oben', 'oben', 'unten', 'unten' und ähnliche Ausdrücke können in Bezug auf eine Richtung oder einen Weg der (zu aerosolisierenden) Flüssigkeit vom Tank zum Ultraschallgenerator und des Aerosols vom Ultraschallgenerator zum Mundstück verstanden werden.

Sofern nicht anders angegeben, sind alle Bezugnahmen auf "radial", "radial", "umfangsmäßig", "umfangsmäßig" und ähnliche Ausdrücke unter Bezugnahme auf eine Mittelachse des Bauteils zu verstehen, auf das Bezug genommen wird.

Mittels der Vorrichtung und des Verfahrens gemäß der vorliegenden Erfindung wird die unbeabsichtigte Ansammlung von Tröpfchen auf der der Auslassöffnung des Behälters zugewandten Oberfläche des Ultraschallgenerators, d. h. der Innenfläche oder der Unterseite des Ultraschallgenerators, verringert oder unterbunden. Dadurch wird folglich jegliche Migration der Flüssigkeit durch den Ultraschallgenerator, z.B. durch die Öffnung oder Durchgänge oder Maschen des Ultraschallgenerators zur Oberseite des Ultraschallgenerators, verringert oder gestoppt und somit die Tröpfchenbildung auf der Oberseite des Ultraschallgenerators verringert bzw. gestoppt.

Darüber hinaus erhöht der vorstehend genannte Effekt nicht nur die Geschwindigkeit bzw. Erffizienz für Neustart und Vernebelung, sondern ermöglicht des Weiteren auch die Verwendung von Flüssigkeit mit hohem Wassergehalt.

Die vorstehend erwähnten Eigenschaften und sonstigen Merkmale und Vorteile der vorliegenden Erfindung und die Art und Weise, wie sie erreicht werden, werden anhand der nachfolgenden Beschreibung von vorteilhaften Ausführungsformen der vorliegenden Erfindung Technik in Verbindung mit der beigefügten Zeichnung deutlich.

Nachstehend sind verschiedene Ausführungsformen der Erfindung anhand einer schematisch vereinfachten Zeichnung im Detail beschrieben. Es zeigen:
- Fig. 1, 2: perspektivische Ansichten einer beispielhaften Ausführungsform einer Aerosolerzeugungsvorrichtung im montierten Zustand,
- Fig. 3: die Aerosolerzeugungsvorrichtung von Fig. 1 bzw. Fig. 2 in einem zerlegten Zustand,
- Fig. 4a, 4b und 4c: jeweils schematische Querschnittsansichten verschiedener Ausführungsbeispiele eines Behälters der Aerosolerzeugungsvorrichtung zum Speichern einer zu aerosolisierenden Flüssigkeit;
- Fig. 5a, 5b: jeweils schematische Querschnittsansichten des Behälters von Fig. 4a bzw. Fig. 4b, die mit einem Docht versehen sind,
- Fig. 6a, 6b: jeweils schematische Querschnittsansichten des Behälters entlang der Linie I-I und II-II von Fig. 5a bzw. Fig. 5b,
- Fig. 7: schematisch eine Draufsicht auf einen Ultraschallgenerator, der Teil der erfindungsgemäßen Aerosolerzeugungsvorrichtung gemäß der Fig. 1-3 ist,
- Fig. 8: schematisch eine beispielhafte Ausführungsform einer Anordnung einer Dichtung am Docht von Fig.5a,
- Fig. 9a. 9b: jeweils schematische Querschnittsansichten des Behälters von Fig. 5a bzw. Fig. 5b dar, die mit einem Ultraschallgenerator ausgestattet sind,
- Fig. 10: eine perspektivische Schnittansicht eines Teils einer beispielhaften Ausführungsform der Aerosolerzeugungsvorrichtung,
- Fig 11: schematisch eine Querschnittsansicht des Teils der Aerosolerzeugungsvorrichtung von Fig. 10,
- Fig. 12: eine Querschnittsansicht eines Teils einer weiteren beispielhaften Ausführungsform der Aerosolerzeugungsvorrichtung,
- Fig. 13: schematisch die Abmessungen der Strukturen/Komponenten einer erfindungsgemäßen Aerosolerzeugungsvorrichtung,
- Fig. 14: eine schematische Darstellung von weiteren Dimensionen der Strukturen/Komponenten der erfindungsgemäßen Aerosolerzeugungsvorrichtung,
- Fig. 15: eine ausschnittsweise Ansicht der Anordnung zwischen einem Ultraschallgenerator und einem Auslassende des Dochtes im Ruhezustand des Ultraschallgenerators,
- Fig. 16: eine ausschnittsweise Ansicht der Anordnung zwischen dem Ultraschallgenerator von Fig. 15 und dem Auslassende des Dochtes im schwingenden Zustand des Ultraschallgenerators,
- Fig. 17: einen Axialschnitt für die Anordnung von aromatisierten Elementen,
- Fig. 18-20: jeweils verschiedene Ansichten einer Kartusche, die Teil der erfindungsgemäßen Aerosolerzeugungsvorrichtung von Fig. 1-3 ist,
- Fig. 21: eine Querschnittsansicht der Kartusche entlang der Linie A-A von Fig. 20,
- Fig. 21a: eine vergrößerte Ansicht des Bereichs I von Fig. 21,
- Fig. 21b: eine vergrößerte Ansicht des Bereichs II von Fig. 21,
- Fig. 22: eine Perspektivansicht eines Mundstücks, das Bestandteil der Kartusche gemäß der Fig. 18-20 ist,
- Fig. 23: einen Halbschnitt durch die Kartusche, analog zur Querschnittsansicht von Fig. 21,
- Fig. 24: eine Perspektivansicht eines Ultraschallgenerators und von zugehörigen Dichtungselementen,
- Fig. 25: eine schematisch vereinfachte Seitenansicht des Ultraschallgenerators von Fig. 24,
- Fig. 26: eine schematisch vereinfachte Seitenansicht des Ultraschallgenerators von Fig. 25, wenn angrenzend hierzu ein Docht bzw. Kapillarelement angeordnet ist, wobei sich der Ultraschallgenerator im Ruhezustand befindet, und
- Fig. 27-28: jeweils schematisch vereinfachte Seitenansichten des Ultraschallgenerators von Fig. 26, wenn dieser aktuiert und entsprechend in Schwingung versetzt wird.

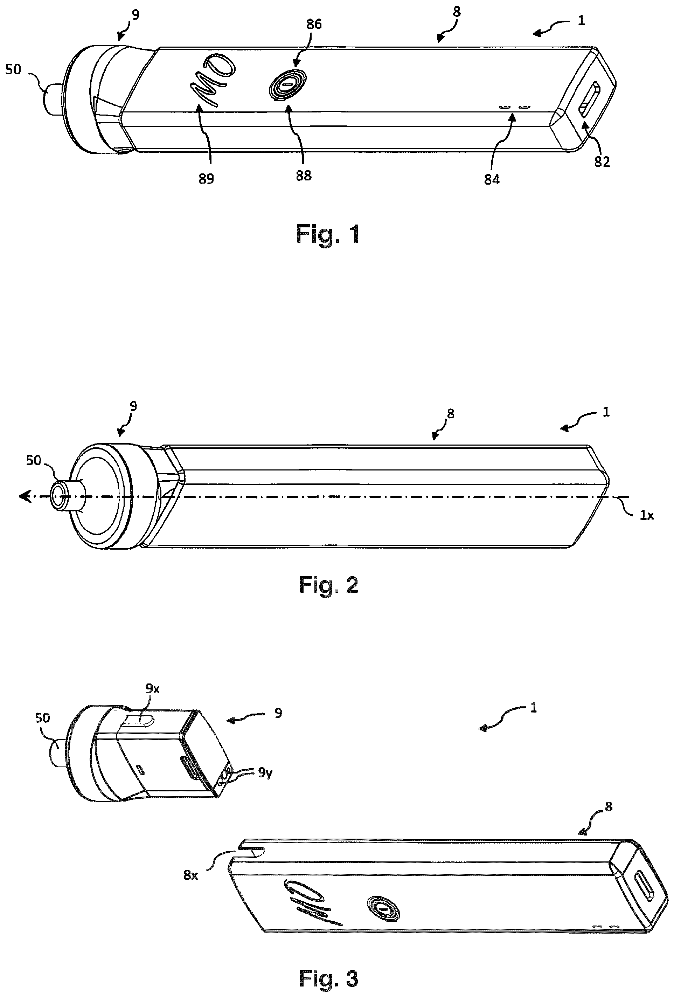

Nachstehend sind unter Bezugnahme auf die Fig. 1-28 bevorzugte Ausführungsformen einer erfindungsgemäßen Aerosolerzeugungsvorrichtung 1 und eines erfindungsgemäßen Verfahrens im Detail erläutert. Gleiche Merkmale in der Zeichnung sind jeweils mit gleichen Bezugszeichen versehen. An dieser Stelle wird gesondert darauf hingewiesen, dass die Zeichnung lediglich vereinfacht und insbesondere ohne Maßstab dargestellt ist.

Die Fig. 1 und 2 zeigen perspektivische Ansichten einer beispielhaften Ausführungsform einer Aerosolerzeugungsvorrichtung 1 (im Folgenden auch als Vorrichtung bezeichnet) in einem zusammengebauten bzw. montierten Zustand.

Fig. 3 zeigt die Aerosolerzeugungsvorrichtung 1 von Fig. 1 in einem zerlegten bzw. demontierten Zustand.

Die erfindungsgemäße Aerosolerzeugungsvorrichtung gemäß Fig. 1 bzw. Fig. 3 kann ein persönlicher Verdampfer, ein Inhalator, eine elektrische Zigarette oder E-Zigarette. Es sei darauf hingewiesen, dass, obwohl in der vorliegenden Beschreibung die Vorrichtung so dargestellt ist, dass sie eine Struktur aufweist, die zusammengebaut und zerlegt bzw. demontiert werden kann. Gleichwohl ist die erfindungsgemäße Vorrichtung hierauf nicht beschränkt. Dies bedeutet, dass die erfindungsgemäße Vorrichtung 1 eine einheitliche Struktur aufweisen kann, d. h. sie kann nicht zusammengebaut und zerlegt werden, oder sie kann eine Struktur aufweisen, die zusammengebaut und zerlegt werden kann, sich jedoch von der Struktur gemäß der Fig. 1 - 3 unterscheidet.

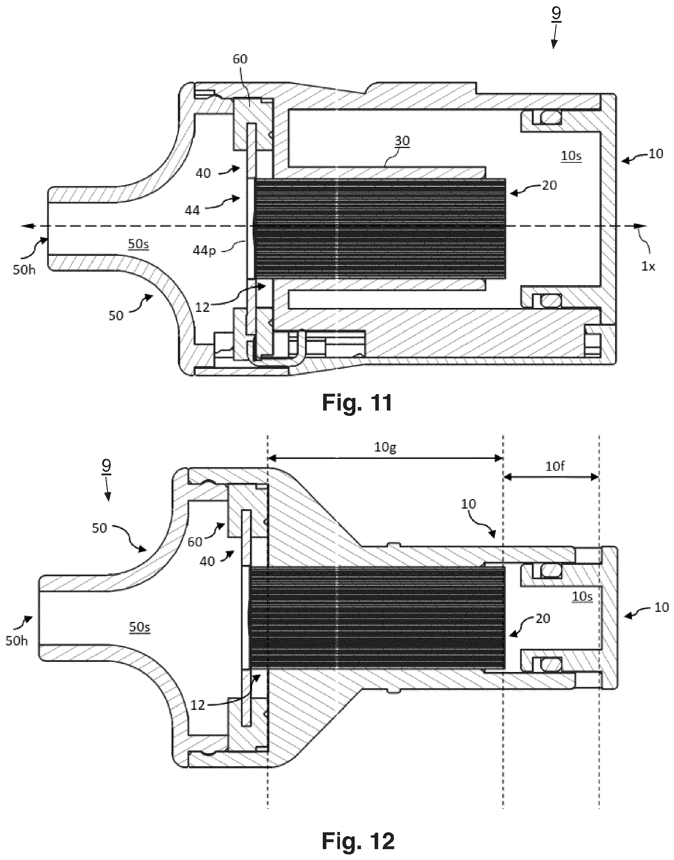

Die Aerosolerzeugungsvorrichtung 1 kann sich entlang einer Längsachse 1x erstrecken (vgl. Fig. 2, Fig. 11).

Die Aerosolerzeugungsvorrichtung 1 kann eine lineare Form aufweisen, die sich entlang der Längsachse 1x erstreckt, wie z.B. Stabform oder Stabform.

Die Aerosolerzeugungsvorrichtung 1 kann ein Hauptteil (bzw. Handstück) 8 und ein Patronenteil 9 (nachfolgend stets als "Kartusche" bezeichnet) aufweisen. Beispielsweise ist die Kartusche 9 in Fig. 3 gezeigt, wenn sie von dem Hauptteil 8 demontiert, d.h. hiervon entfernt worden ist.

Die Aerosolerzeugungsvorrichtung 1, z. B. deren Hauptteil 8, kann eine Stromversorgung (nicht gezeigt) aufnehmen, wie z. B. eine Batterieeinheit zur Stromversorgung eines Ultraschallgenerators (nicht in den Fig. 1-3 gezeigt, und später noch erläutert) und/oder anderer elektronischer Komponenten.

Die Aerosolerzeugungsvorrichtung 1 kann auch eine (nicht gezeigte) Steuerungseinrichtung zur Steuerung eines Betriebs der Stromversorgung und des Ultraschallgenerators und/oder anderer Komponenten der Vorrichtung 1 enthalten.

Die Aerosolerzeugungsvorrichtung 1 kann ferner einen Ladeanschluss 82 aufweisen, um die Stromversorgung der Vorrichtung 1 durch Bereitstellen einer externen Stromversorgung oder eine oder mehrere Anzeigen wie LED-Anzeigen 84, 88 aufzuladen. Diese Anzeigen dienen dazu, um den Betriebszustand der Vorrichtung 1 und/oder um einen Ladezustand der Vorrichtung 1 anzuzeigen, z.B. einen Zustand mit voller Ladung, einen Zustand mit niedrigem Batteriestand, einen Zustand, in dem der Ultraschallgenerator mit Strom versorgt wird, um Ultraschall zu erzeugen, oder einen Standby-Zustand.

Die Aerosolerzeugungsvorrichtung 1 kann eine Benutzerschnittstelle 88 (vgl. Fig. 1) aufweisen, z. B. eine Taste, wie z. B. eine EIN/AUS-Taste, die ein Benutzer betätigen, z. B. drücken kann, um die Funktion oder den Betrieb der Vorrichtung 1 zu steuern, wie z. B. die Eingabe eines Benutzerbefehls, um die Stromversorgung des Ultraschallgenerators zu implementieren, um Ultraschall zum Erzeugen oder Erzeugen von Aerosol zu erzeugen. Optional kann die Vorrichtung 1 ein Oberflächenmerkmal 89 aufweisen, wie beispielsweise ein Logo oder Branding oder eine rutschfeste Oberflächenmodifikation.

Die Aerosolerzeugungsvorrichtung 1, z. B. die Kartusche 9, kann ein Reservoir oder einen Behälter (nicht in den Fig. 1 - 3 gezeigt, jedoch später noch erläutert) enthalten, um eine Flüssigkeit aufzunehmen oder aufzunehmen, die vernebelt werden soll, um Aerosol zu erzeugen, und ein Mundstück 50, um das so erzeugte Aerosol an eine Außenseite der Vorrichtung 1 abzugeben.

Das Hauptteil 8 und die Kartusche 9 können als ein Körper ausgebildet sein oder eine Struktur aufweisen, die mit Hilfe von miteinander verbindbaren Eingriffsteilen 8x, 9x, die an dem Hauptteil 8 und der Kartusche 9 vorgesehen sind (vgl. Fig. 3), montiert und demontiert werden kann.

Insbesondere kann das Hauptteil 8 ein Haupteingriffsteil 8x und die Kartusche 9 ein Kartuscheneingriffsteil 9x enthalten (vgl. Fig. 3). Das Haupteingriffsteil 8x kann reversibel mit dem Kartuscheneingriffsteil 9x einrasten und auch davon wieder gelöst werden, um das Hauptteil 8 und die Kartusche 9 miteinander zu verbinden bzw. voneinander zu trennen. In dieser Weise kanndie Vorrichtung 1 mit ihren Komponenten Hauptteil 8 und Kartusche 9 montiert oder bei Bedarf auch wieder demontiert werden.

An der Kartusche 9 können elektrische Kontakte oder Anschlüsse 9y (vgl. Fig. 3) vorgesehen sein, um die Stromversorgung (im Hauptteil 8 enthalten) und den Ultraschallgenerator 40 (in der Kartusche 9 enthalten) miteinander elektrisch bzw. signaltechnisch miteinander zu verbinden.

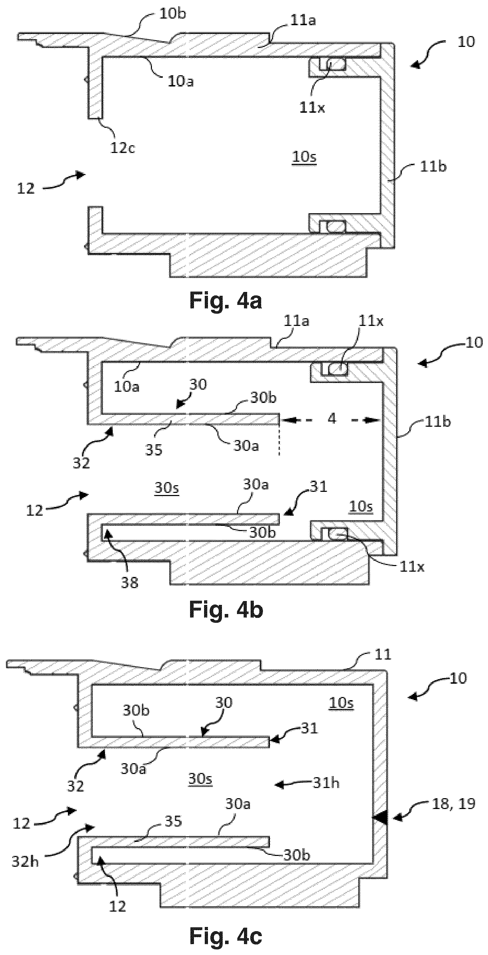

Im Folgenden werden unter Bezugnahme auf die Fig. 4a - 10 eine Anordnung und Struktur verschiedener Komponenten der Kartusche 9 der Vorrichtung 1 beschrieben.

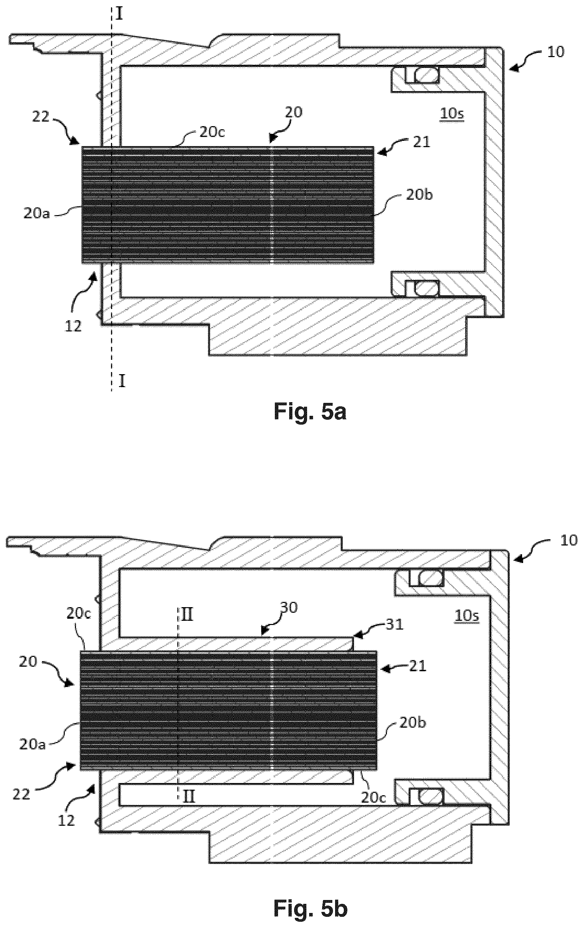

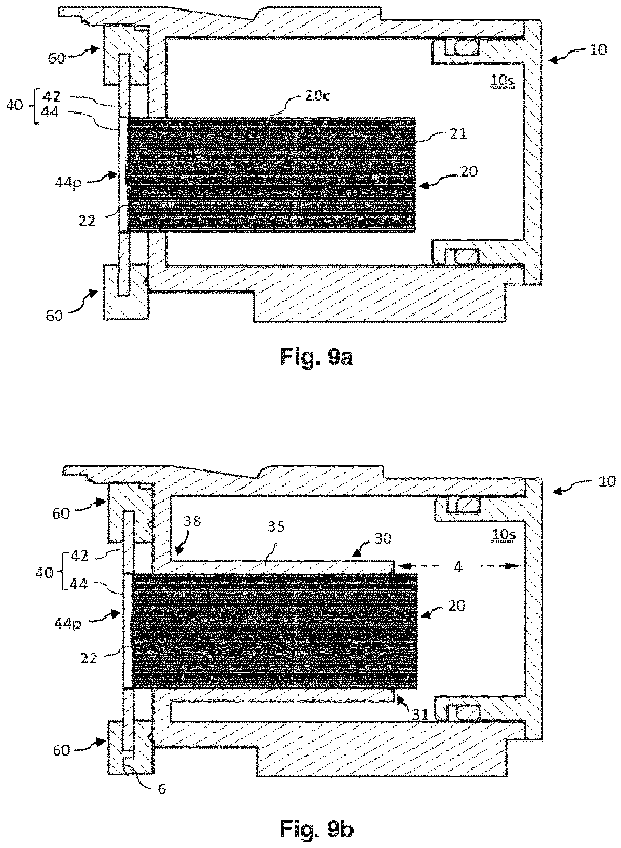

Die Vorrichtung 1, d. h. die Kartusche 9, weist einen Behälter 10 auf, wie in den Fig. 4a, 4b und 4c gezeigt, mit einer Auslassöffnung 12, einem Docht 20, der in die Auslassöffnung 12 eingepasst ist, wie in den Fig. 5a und 5b gezeigt, und einem Ultraschallgenerator 40 (auch als Ultraschallwandler oder Sonde oder Kopf oder Aktuator bezeichnet), der gegenüberliegend zum Docht 20 angeordnet ist, wie es in den Fig. 9a und Fig. 9b gezeigt ist.

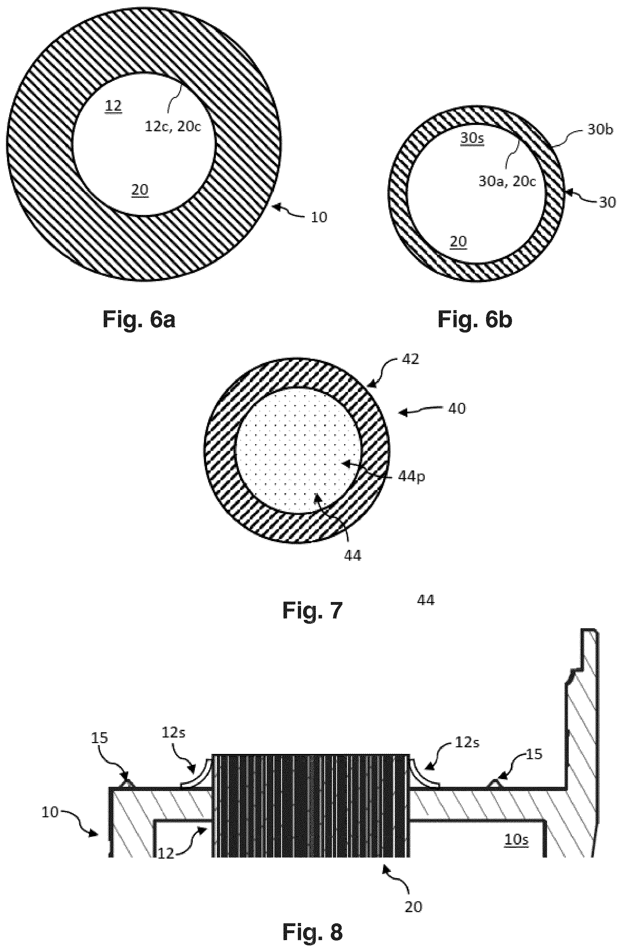

Fig. 7 zeigt eine beispielhafte Ausführungsform des Ultraschallgenerators 40, der Teil der erfindungsgemäßen Aerosolerzeugungsvorrichtung 1 ist und in der Kartusche 9 angebracht ist, Der Ultraschallgenerator 40 kann ein erstes Teil 42 aufweisen, das ein piezoelektrisches Element umfasst; und einen zweiten Teil 44, der eine Vielzahl von Durchgängen 44p oder ein Netz 44p, z. B. ein metallisches Netz, aufweist. Die Durchgänge 44p oder das Netz 44p machen den zweiten Teil 44 des Ultraschallgenerators 40 durchlässig für Flüssigkeit und insbesondere für das von dem Ultraschallgenerator 40 erzeugte Aerosol. Weitere Einzelheiten zum Ultraschallgenerator 40 und dessen Wechselwirkung mit dem Docht 20 sind nachfolgend noch gesondert erläutert.

Ausweislich der Fig. 7 kann der erste Teil 42 des Ultraschallgenerators 40 eine ringförmige Form aufweisen, wobei der zweite Teil 44 des Ultraschallgenerators 40 in einem Zentrum des ringförmig geformten ersten Teils 42 angeordnet sein kann.

Der zweite Teil 44 des Ultraschallgenerators 40 weist zwei entgegengesetzte oder umgekehrte Seiten oder Oberflächen auf - eine Aufnahmefläche und eine Austrittsfläche-, die über das Netz 44p oder die Durchgänge 44p in Fluidverbindung miteinander stehen.

Die Aufnahmefläche des zweiten Teils 44 des Ultraschallgenerators 40 kann erfindungsgemäß als die der Auslassöffnung 12 und dem Auslassende 22 des Dochtes 20 zugewandte Fläche des zweiten Teils 44 verstanden werden. Des Weiteren kann die Austrittsfläche des zweiten Teils 44 als die dem Mundstück 50 zugewandte Fläche des zweiten Teils 44 verstanden werden bzw. einer Austrittsöffnung 50hdes Mundstücks 50 zugewandt sein.

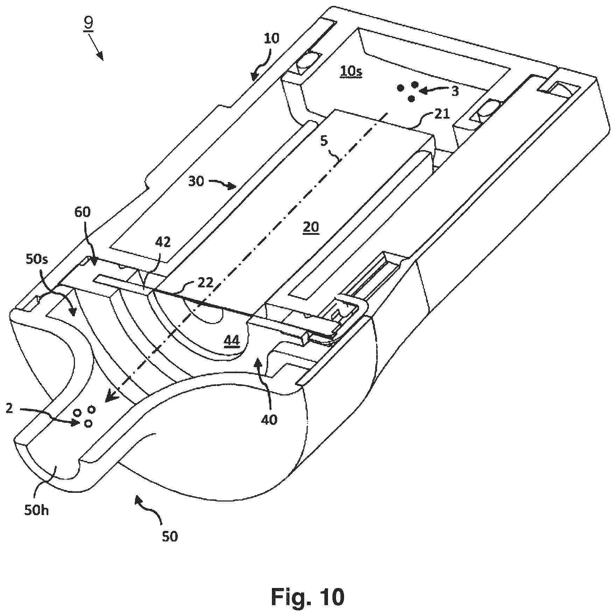

Die Fig. 10, 11 und 12 zeigen eine beispielhafte Ausführungsform der Vorrichtung 1, in welcher der Behälter 10, der Docht 20, der Ultraschallgenerator 40 und das Mundstück 50 in ihrer Anordnung relativ zueinander dargestellt sind.

Der Behälter 10 kann eine Führung 30 enthalten, wie in Fig. 4b und 4c gezeigt, oder kann die Führung 30, wie in Fig. 4a gezeigt, nicht enthalten. Die Fig. 10 und 11 zeigen eine beispielhafte Ausführungsform der Vorrichtung 1 mit der Führung 30. Gleichwohl kann die in den Fig. 10 und 11 gezeigte Anordnung mutatis mutandis auf die Vorrichtung 1 ohne die Führung 30 angewendet werden.

Figur 12 zeigt eine beispielhafte Ausführungsform der Vorrichtung 1 ohne die Führung 30.

Im Folgenden werden die vorstehend genannten genannten Komponenten der Erfindung näher beschrieben.

Wie in den Fig. 4a - 4c gezeigt, umfasst der Behälter 10 ein Flüssigkeitsreservoir in Form eines Unterbringungsraums 10s (nachfolgend stets als "Tank" bezeichnet) auf. Dieser Tank 10s dient zum Speichern oder Lagern oder Halten einer Flüssigkeit 3 (in Fig. 10 mit drei Punkten symbolisiert) auf, die versprüht werden soll.

Der Behälter 10 kann aus einem polymeren Material, wie z.B. Kunststoff, gebildet sein.

Der Behälter 10 kann zumindest einen Teil oder ein Segment aufweisen, das transparent ist und einen Blick auf ein Inneres des Behälters 10, d. h. des Tanks 10s, ermöglicht - wodurch ein Benutzer den Füllstand der in dem Tank 10s vorhandenen Flüssigkeit sehen kann.

Der Behälter 10 kann einen Körper 11 enthalten. Der Körper 11 kann die Auslassöffnung 12 umfassen.

Der Körper 11 kann in einem Teil ausgebildet sein, d. h. er kann einstückig ausgebildet sein, wie in Fig. 4c gezeigt; oder kann durch eine Vielzahl von Teilen 11a, 11b gebildet sein, wie in den Fig. 4a und 4b gezeigt, wie beispielsweise ein erstes Körpersegment oder Teil 11a, z. B. ein Hauptbehälterkörper 11a und ein zweites Körpersegment oder Teil 11b, z. B. ein Behälterdeckel 11b.

Die Teile 11a, 11b können mit einer flüssigkeitsdichten Verbindung 11x dazwischen verbindbar sein, um den Körper 11 des Behälters 10 zu bilden oder vollständig zusammenzusetzen. Die Teile 11a, 11b können mit der flüssigkeitsdichten Verbindung 11x reversibel verbindbar sein, d.h. sie können voneinander gekoppelt und entkoppelt sein.

Die flüssigkeitsdichte Verbindung 11x kann eine Schraub- und Gewindeverbindung oder eine Presspassverbindung usw. sein.

Der Behälter 10 - unabhängig davon, ob er in einem Teil ausgebildet ist, wie in Fig. 4c gezeigt, oder durch mehrere miteinander verbindbare Teile zusammengesetzt ist, wie in Fig. 4a und 4b gezeigt - kann den Tank 10s fluiddicht oder flüssigkeitsdicht oder hermetisch abgedichtet in Bezug auf eine Außenseite des Behälters 10 definieren, mit Ausnahme der Auslassöffnung 12 oder angrenzenden Bereichen hiervon.

Anders ausgedrückt, steht der Tank 10s des Behälters 10 in Fluidverbindung mit einer Außenseite des Behälters 10, um ein Strömen der Flüssigkeit aus dem Tank 10s zur Außenseite des Behälters 10 nur über die Auslassöffnung 12 zu ermöglichen.

Der Behälter 10 oder der Körper 11 des Behälters 10 kann die Führung 30 aufweisen, die sich von der Auslassöffnung 12 in den Tank 10s erstreckt.

Die Führung 30 kann eine hohle rohrförmige Form aufweisen und einen Aufnahmeraum 30s definieren.

Die Führung 30 kann an einem Teil der Innenfläche des Behälters 10 befestigt sein oder diesen berühren, der die Auslassöffnung 12 umgibt oder umschreibt. Mit anderen Worten kann die Auslassöffnung 12 an einer Innenseite des Behälters 10 innerhalb der Führung 30 oder innerhalb einer Umfangswand 35 der Führung 30 untergebracht sein. Eine Kontaktschnittstelle oder ein Kontaktteil 38 zwischen der Führung 30 und der Innenfläche 10a des Behälters 10 kann flüssigkeitsdicht oder hermetisch abgedichtet sein. Beispielsweise kann die Führung 30 einstückig mit dem Teil der Innenfläche des Behälters 10 ausgebildet sein, der die Auslassöffnung 12 umgibt oder umschreibt.

Insbesondere kann die Führung 30 einen hohlen, rohrförmigen Körper aufweisen, der ein Auslassende 32 aufweist, das mit der Innenfläche 10a des Behälters 10 verbunden ist, der den Auslass umgibt oder umgibt. Das Auslassende 32 der Führung 30 kann einen Auslass 32h aufweisen, der mit der Auslassöffnung 12 in Fluidverbindung steht.

Der hohle, rohrförmige Körper der Führung 30 kann ein Einlassende 31 (vgl. Fig. 4b, Fig. 4c) aufweisen, das innerhalb des Tanks 10s angeordnet ist und von der Innenfläche 10a des Behälters 10 (bzw. bei der Ausführungsform von Fig. 4a und Fig. 4b: von dem Behälterdeckel 11b) durch einen Spalt 4 beabstandet ist. Somit ist für den innerhalb des Behälters gebildeten Tank 10s ein ausreichend großes Volumen zur Aufnahme bzw. zum Speichern der Flüssigkeit 3 gewährleistet.

Das Einlassende 31 der Führung 30 kann einen Einlass 31h (vgl. Fig. 4c) aufweisen, der mit dem Tank 10s in Fluidverbindung steht.

Die Umfangswand 35 der Führung 30 kann sich in Längsrichtung zwischen dem Einlassende 31 und dem Auslassende 32 der Führung 30 erstrecken.

Anders ausgedrückt, steht der Tank 10s des Behälters 10 in Fluidverbindung mit einer Außenseite des Behälters 10, um ein Strömen der Flüssigkeit aus dem Tank 10s zur Außenseite des Behälters 10 nur über die Führung 30 und die Auslassöffnung 12 zu ermöglichen, beispielsweise nur über den hintereinander angeordneten Einlass 31h, den Tank 30s, den Auslass 32h und die Auslassöffnung 12.

Die Führung 30 kann innerhalb des Tanks 10s in einer freitragenden Anordnung angeordnet sein und kann mit der Innenfläche 10a des Behälters 10 nur an einem Ende der Führung 30, d. h. dem Auslassende 32 der Führung 30, verbunden sein.

Die Umfangswand 35 und der Einlass 31 der Führung 30 können von der Innenfläche 10a des Behälters 10 beabstandet sein.

Der Behälter 10 kann ferner eine Einlassöffnung 18 und einen Deckel 19 (vgl. Fig. 4c) aufweisen, der die Einlassöffnung 18 abdeckt oder abdichtet. Die Einlassöffnung 18 und/oder der Deckel 19 dürfen nur einen Flüssigkeitszufluss in den Tank 10s von einer Außenseite des Behälters zulassen, d.h. die Flüssigkeit aus dem Tank 10s darf nicht über die Einlassöffnung 18 und/oder den Deckel 19 aus dem Tank 10s herausfließen. Die Einlassöffnung 18 und/oder der Deckel 19 können zum Einfüllen oder Nachfüllen der Flüssigkeit in den Tank 10s verwendet werden.

Fig. 5a zeigt den Docht 20, der in und durch die Auslassöffnung 12 des in Fig. 4a gezeigten Behälters 10 eingeführt ist, und Fig. 9a zeigt den Ultraschallgenerator 40, der angrenzend zu dem in Fig. 5a gezeigten Docht 20 angeordnet ist.

Fig. 5b zeigt den Docht 20, der in und durch die Auslassöffnung 12 eingeführt ist, und die Führung 30 des Behälters 10, der in Fig. 4b und Fig. 9b gezeigt ist. Des Weiteren zeigt Fig. 9b den Ultraschallgenerator 40, der angrenzend zu dem in Figur 5b gezeigten Docht 20 angeordnet ist.

Der Docht 20 kann eine feste röhrenförmige Form haben und kann so konfiguriert sein, dass er Flüssigkeit durch Kapillarwirkung oder Dochtwirkung transportiert.

Der Docht 20 transportiert die Flüssigkeit aus dem Tank 10s zum Ultraschallgenerator 40 über die Auslassöffnung 12 (wenn der Behälter 10 die Führung 30 nicht enthält) oder über die Auslassöffnung 12 und die Führung 30 (wenn der Behälter 10 die Führung 30 enthält).

Ausweislich der Fig. 10 weist der Docht 20 ein Einlassende 21 auf, um die Flüssigkeit 3 aus dem Tank 10s in den Docht 20 aufzunehmen. Das Einlassende 21 ist innerhalb des Behälters 10, d.h. im Tank 10s, zur Aufnahme der Flüssigkeit 3 angeordnet.

Der Docht 20 weist an seiner entgegengesetzten Stirnseite, die sich in Gegenüberstellung zum Ultraschallgenerator 40 befindet, ein Auslassende 22 auf, das nicht in Kontakt mit dem Ultraschallgenerator 40 angeordnet ist. Jedenfalls versorgt das Auslassende 22 den Ultraschallgenerator 40 mit der Flüssigkeit 3.

Bei einer Anordnung, bei der das Auslassende 22 des Dochtes 20 innerhalb der Auslassöffnung 12 angeordnet ist (d. h. nicht über die Außenfläche 10b des Behälters 10 hinausragt oder nicht bündig mit der Außenfläche 10b des Behälters 10 abschließt), kann der Ultraschallgenerator 40 so geformt sein, dass er in die Auslassöffnung 12 hineinragt, wobei er das Auslassende 22 nicht direkt berührt. Beispielsweise kann ein zweiter Teil (später beschrieben) des Ultraschallgenerators 40 so geformt sein, dass er in die Austrittsöffnung12 hineinragt.

Der Ultraschallgenerator 40 wendet Ultraschall auf die Flüssigkeit an, die vom Auslassende 22 des Dochtes empfangen wird, wodurch die Flüssigkeit 3 versprüht und das Aerosol 2 aus der Flüssigkeit 3 erzeugt wird (wie in Fig. 10 gezeigt). Das Mundstück 50 gibt das erzeugte Aerosol 2 an eine Außenseite der Vorrichtung 1 ab, beispielsweise wenn es von einem Benutzer gezogen oder angesaugt wird.

Wie in Fig. 10 gezeigt, definieren der Tank 10s, der Docht 20 und das Mundstück 50 - alle zusammen - einen Fluidleitungspfad 5 für den Transport der Flüssigkeit 3 aus dem Tank 10s zum Ultraschallgenerator 40 und zum Transport des von dem Ultraschallgenerator 40 erzeugten Aerosols 2 zum Mundstück 50.

Der Ultraschallgenerator 40 ist, z.B. direkt angeordnet, zwischen dem Mundstück 50 und dem Docht 20 angeordnet. Der Fluidleitungspfad 5 verläuft auch durch den Ultraschallgenerator 40 hindurch.

Wie aus den Fig. 4a, 5a, 6a und 9a ersichtlich ist, ist der Docht 20 in der Auslassöffnung 12 derart angeordnet, dass der Docht 20 die Auslassöffnung 12 ausfüllt, d. h. vollständig ausfüllt.

Bei der Fig. 6a handelt es sich um einen Querschnitt entlang der Linie I-I von Fig. 5a. Wie aus der Fig. 6a ersichtlich ist, kann eine äußere Umfangsfläche 20c des Dochtes 20 direkt eine gesamte Umfangsfläche berühren, welche die Auslassöffnung 12 definiert.

Mit anderen Worten kann die Auslassöffnung 12 vollständig gefüllt oder durch den Docht 20 vollgestopft sein. Vereinfacht ausgedrückt wird die gesamte Querschnittsfläche der Auslassöffnung 12 von dem Docht 20 derart eingenommen, dass die Flüssigkeit im Tank 10s nur über den Docht 20 durch die Auslassöffnung 12 strömt.

Wie aus den Fig. 4b, 5b, 6b und 9b ersichtlich ist, ist der Docht 20 in der Auslassöffnung 12 und der Führung 30 derart angeordnet, dass der Docht 20 die Auslassöffnung 12 und/oder den Aufnahmeraum 30 der Führung 30 ausfüllt oder vollständig ausfüllt.

Wie in Fig. 6b gezeigt, bei der es sich um einen Querschnitt entlang der Linie II-II der Fig. 5b handelt, kann die äußere Umfangsfläche 20c des Dochtes 20 eine Innenfläche 30a der Umfangswand 35 der Führung 30 mindestens um einen seitlichen Querschnitt senkrecht zur Längsachse der Führung 30 direkt berühren.

Vorzugsweise nimmt der Docht 20 ein ganzes Volumen oder einen ganzen Raum des Aufnahmeraums 30s der Führung 30 ein.

Anders ausgedrückt, kann die Auslassöffnung 12 und/oder der Aufnahmeraum 30 der Führung 30 vollständig ausgefüllt oder durch den Docht 20 vollgestopft sein.

Der Docht 20 kann in der Auslassöffnung 12 und/oder dem Aufnahmeraum 30s der Führung 30 aufgenommen werden, indem er in die Auslassöffnung 12 und/oder den Aufnahmeraum 30 der Führung 30 eingepasst wird.

Um die Abdichtung zwischen dem Docht 20 und der Auslassöffnung 12 weiter zu verbessern, kann ein Dichtungselement 12s, z. B. eine Gummidichtung, wie sie beispielsweise in Figur 8 gezeigt ist, an der Auslassöffnung 12 angeordnet sein. Die Dichtung 12s kann die äußere Umfangsfläche 20c des Dochtes 20 berühren.

Das Auslassende 22 des Dochtes 10 steht nicht in direktem Kontakt mit dem zweiten Teil 44 des Ultraschallgenerators 40, wie nachstehend noch gesondert erläutert.

Die Vorrichtung 1 kann ferner ein Halteelement 60 (vgl. Fig. 10) aufweisen, das zwischen dem Mundstück 50 und dem Behälter 10 angeordnet ist.

Der Ultraschallgenerator 40 kann auf dem Halteelement 60 montiert oder in das Halteelement 60 aufgenommen werden, um den Ultraschallgenerator 40 zwischen dem Mundstück 50 und dem Behälter 10 zu stützen oder zu montieren oder zu fixieren.

Der Behälter kann ein Behälterverbindungselement 15 aufweisen (wie beispielsweise in Fig. 8 gezeigt), und das Halteelement 60 kann ein Halteelement-Verbindungselement (nicht gezeigt) aufweisen. Das Behälterverbindungselement 15 und das Halteelement-Verbindungselement können einander entsprechen und können miteinander in Eingriff stehen, um den Behälter 10 und das Halteelement 60 mit dem darauf montierten Ultraschallgenerator 40 zu fixieren oder zu koppeln. Dies ist in den Fig.9a und 9b dargestellt. Die Kupplung kann lösbar oder reversibel sein.

Fig. 12 zeigt eine weitere beispielhafte Ausführungsform der Vorrichtung 1, bei der der Docht 20 direkt im Tank 10s, d.h. in Kontakt mit der Innenfläche 10a des Tanks 10s, angeordnet sein kann und den Tank 10s - ganz oder teilweise - einnimmt.

Der Behälter 10 kann ein Führungssegment 10g aufweisen, das den Aufnahmeraum 30s definiert.

Wie aus Figur 12 ersichtlich ist, ist der Docht 20 in der Auslassöffnung 12 und dem Führungssegment 10g derart angeordnet, dass der Docht 20 die Auslassöffnung 12 und/oder den Aufnahmeraum 30s des Führungssegments 10g ausfüllt oder vollständig ausfüllt. Das Führungssegment 10g kann die Flüssigkeit 3 aufnehmen, die in dem darin angeordneten Docht 20 adsorbiert ist.

Das Führungssegment 10g kann den gesamten Tank 10s bilden.

Zusätzlich und optional kann der Behälter 10 zusätzlich zu dem Führungssegment 10g ein Reservoirsegment 10f aufweisen. Das Reservoirsegment 10f umfasst den Docht 20 nicht, d. h. es ist nicht durch den Docht 20 gefüllt und kann die Flüssigkeit 3 darin aufnehmen (vgl. Fig. 12).

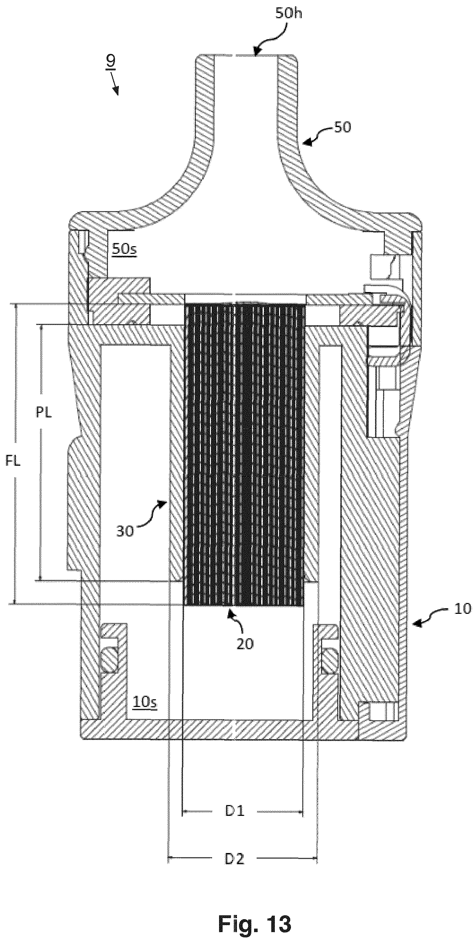

Bezugnehmend auf Fig. 13 kann der Docht 20 eine volle Länge FL zwischen 15 mm und 25 mm, beispielsweise 19 mm, aufweisen.

Eine Gesamtlänge PL der Führung 30 und der Auslassöffnung 12 kann zwischen 12 mm und 18 mm, beispielsweise 16 mm, liegen.

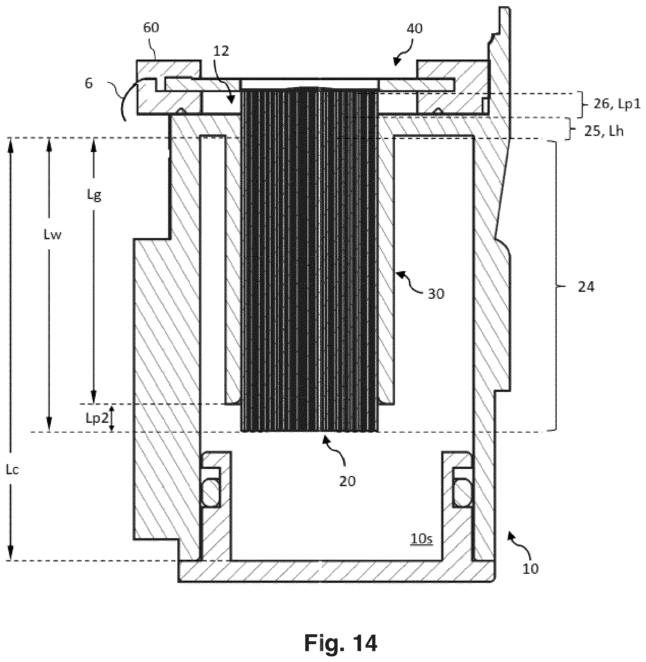

Unter Bezugnahme auf Fig. 14 kann der Docht 20 mindestens zwei Abschnitte oder Teile oder Segmente aufweisen - einen inneren Abschnitt 24, d. h. einen ersten Abschnitt des Dochtes 20, der ausserhalb der Auslassöffnung 12 und in dem Tank 10s angeordnet ist, und einen Öffnungsabschnitt 25, d. h. einen zweiten Abschnitt, des Dochtes 20, der vollständig in oder innerhalb der Auslassöffnung 12 angeordnet ist, d. h. der nicht an irgendeiner Seite von der Auslassöffnung 12 herausragt . Zusätzlich kann der Docht 20 optional einen dritten Abschnitt oder Teil oder ein Segment aufweisen - einen äußeren Abschnitt oder einen äußeren Abschnitt 26 des Dochtes, der aus der Auslassöffnung 12 zur Außenseite des Behälters 10 herausragend angeordnet ist, d. h. außerhalb des Tanks 10s und außerhalb der Auslassöffnung 12 angeordnet ist.

Der Docht 20 kann auf beiden Seiten vorstehen, d. h. das Einlassende 21 des Dochtes 20 kann aus der Führung 30 in den Tank 10s herausragen und das Auslassende 22 des Dochtes 20 kann aus der Auslassöffnung 12 herausragen zu einer Außenseite des Behälters 10 oder des Tanks 10s.

Unter Bezugnahme auf die Fig. 14 sind folgende Abmessungen des Behälters 10 und von darin angebrachten Komponenten der erfindungsgemäßen Aerosolerzeugungsvorrichtung ersichtlich und nachstehend wie folgt erläutert:
- Eine Länge Lw des inneren Abschnitts 24 des Dochtes 20 kann kleiner oder gleich einer Länge Lc des Tanks 10s sein und/oder zwischen 0 mm und 50 mm, vorzugsweise zwischen 10 mm und 30 mm liegen.
- Eine Länge Lh der Auslassöffnung 12 und/oder des Öffnungsabschnitts 25 des Dochtes 20 kann zwischen 0,5 mm und 3,5 mm, vorzugsweise zwischen 0,8 mm und 3,2 mm betragen.
- Eine Länge Lp1 des äußeren Abschnitts 26 des Dochtes 20 kann 0 mm oder zwischen 0 mm und 3,5 mm, insbesondere zwischen 0,1 mm und 3 mm, betragen.
- Eine Länge Lc des Tanks 10s kann zwischen 10 mm und 60 mm, insbesondere zwischen 15 und 50 mm liegen.
- Ein Teil, beispielsweise ein innerer hervorstehender Teil, des inneren Abschnitts 24 des Dochtes 20 kann aus der Führung 30 in den Tank 10s hineinragen.
- Eine Länge Lp2 des inneren hervorstehenden Teils des inneren Abschnitts 24 des Dochtes 20 kann gleich einer Differenz zwischen der Länge Lw des inneren Abschnitts 24 des Dochtes 20 und der Länge Lg der Führung 30 sein. Die Länge Lp2 des inneren hervorstehenden Teils des inneren Abschnitts 24 des Dochtes 20 kann 0 mm betragen oder kann zwischen 0 mm und 15 mm oder zwischen 5 und 10 mm liegen.
- Eine Länge Lg der Führung 30, die sich von der Auslassöffnung 12 in den Tank 10s hinein erstreckt, kann 0 mm betragen, oder kann größer als 0 mm und kleiner oder gleich der Länge Lc des Tanks 10s sein, insbesondere zwischen 0 mm und 45 mm oder zwischen 10 mm und 30 mm.
- Die Länge Lw des inneren Abschnitts 24 des Dochtes 20 kann gleich oder größer als 50 Prozent der Länge Lc des Tanks 10s oder gleich oder größer als 70 Prozent der Länge Lc des Tanks 10s sein, kann gleich oder größer als 80 Prozent der Länge Lc des Tanks 10s sein, oder kann gleich oder größer als 90 Prozent der Länge Lc des Tanks 10s sein.
- Die Länge Lg der Führung 30 kann gleich oder größer als 50 Prozent der Länge Lc des Tanks 10s sein, oder kann gleich oder größer als 70 Prozent der Länge Lc des Tanks 10s sein, oder kann gleich oder größer als 80 Prozent der Länge Lc des Tanks 10s sein, oder kann gleich oder größer als 90 Prozent der Länge Lc des Tanks 10s sein.
- Ein Durchmesser D1 des Aufnahmeraums 30s der Führung 30 und/oder der Auslassöffnung 12 kann zwischen 6 mm und 9 mm, beispielsweise 7,5 mm, liegen. In Anbetracht dessen, dass der Docht 20 in Bezug auf seinen Umfang vollständig in dem Aufnahmeraum 30s aufgenommen ist, ist verstehen, dass der Durchmesser D1 gleichzeitig auch den Außendurchmesser des Dochts bezeichnet.
- Ein Durchmesser D2 der Führung 30 kann zwischen 8 mm und 11 mm, beispielsweise 9,5 mm, liegen.

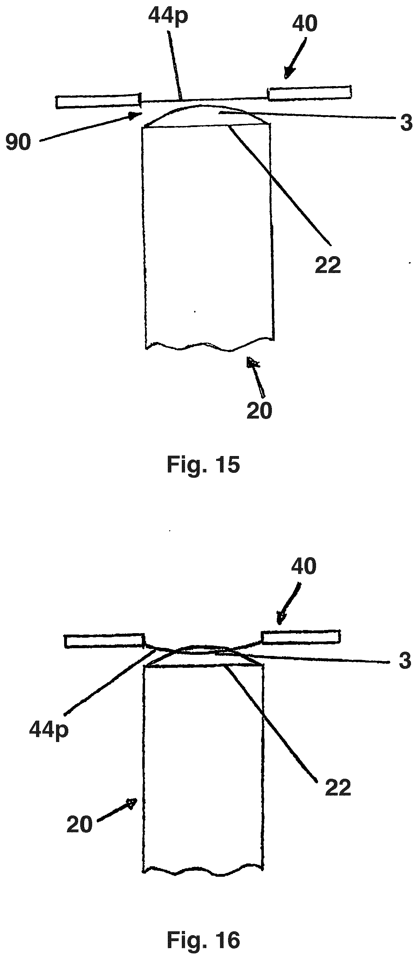

Nachfolgend werden unter Bezugnahme auf die Fig. 15 und Fig. 16 weitere Einzelheiten in Bezug auf den Ultraschallgenerator 40 und dessen Funktionsweise bzw. Wechselwirkung mit dem Docht 20 im Detail erläutert:
In Fig. 15 ist die Anordnung zwischen dem Ultraschallgenerator 40 und dem Auslassende 22 des Dochtes 20 im Ruhezustand des Ultraschallgenerators 40 dargestellt. Durch die Oberflächenspannung der Flüssigkeit 3 bildet sich eine Ansammlung der Flüssigkeit 3 als bogenförmiger Tropfen auf bzw. an dem Auslassende 22 des Dochts 20.

An dieser Stelle wird gesondert darauf hingewiesen, dass erfindungsgemäß die Oberflächenspannung der Flüssigkeit 3 ausreichend hoch gewählt ist bzw. einen ausreichend hohen Wert aufweist, dass sich auf bzw. an dem Auslassende 22 des Dochts 20 eine Ansammlung der Flüssigkeit 3 als bogenförmiger Tropfen bildet (vgl. Fig. 15, Fig. 26, Fig. 27). In diesem Zusammenhang wird gesondert darauf hingewiesen, dass erfindungsgemäß gewährleistet ist, dass in der Flüssigkeit 3 ggf. enthaltene verkeimungshemmende Stoffe bzw. andere Flüssigkeitsanteile, die eine Verkeimung der Aerosolerzeugungsvorrichtung 1 verhindern, die Oberflächenspannung der Flüssigkeit 3 nicht so weit reduzieren, dass es nicht mehr zu einer Ansammlung von Flüssigkeit 3 an dem Auslassende 22 des Dochtes 20 kommt.

Erfindungsgemäß dient die vorstehend genannte Ansammlung von Flüssigkeit 3, die sich an bzw. auf dem Auslassende 22 des Dochts bildet, dann zur Übergabe an den Ultraschallgenerator 40, insbesondere dann, wenn dieser bei seiner Aktuierung in Schwingung versetzt wird.

Das Auslassende 22 des Dochts 20 weist einen Abstand 90 (vgl. Fig. 15) zu den in dem zweiten Teil 44 des Ultraschallgenerators 40 ausgebildeten Durchgängen 44p auf. Dieser Abstand 90 kann durch nicht dargestellte Anschläge, Kanten, Abstandsscheiben oder Abstandsringe oder jegliche andere baulichen Möglichkeiten zur Erzeugung eines Abstandes erreicht werden.

Die Fig. 16 stellt eine ausschnittsweise Ansicht der Anordnung zwischen dem Ultraschallgenerator 40 und dem Auslassende 22 des Dochtes 20 dar, während der zweite Teil 44 (mit seinem Durchgängen 44p) in Schwingung versetzt wird.

Der zweite Teil 44 des Ultraschallgenerators 40 kann durch dessen ersten Teil 42 in Schwingung versetzt werden.

Wenn der zweite Teil 44 des Ultraschallgenerators 40 in seinem Schwingungszustand nach unten, d.h. in Richtung des Dochts 20 verformt wird, berühren oder tauchen die in dem zweiten Teil 44 ausgebildeten Durchgänge 44p in die Flüssigkeit 3 an dem Auslassende 22 des Dochts 20 ein, so dass diese Durchgänge 44p sich mit der Flüssigkeit 3 füllen. Durch die hohe Frequenz der Schwingungen tritt dann die Flüssigkeit 3 als Aerosol aus dem zweiten Teil 44 des Ultraschallgenerators 40 an seiner entgegensetzen Seite aus.

Das Auslassende 22 des Dochtes 30 ist einer Außenseite der Auslassöffnung 12 ausgesetzt und kann entweder bündig mit der Außenfläche 10b des Behälters 10 abschließen oder von der Außenfläche 10b des Behälters 10 nach außen herausragen oder innerhalb der Auslassöffnung 12 angeordnet sein.

Im Zusammenhang mit dem Schwingungszustand des Ultraschallgenerators 40 wird an dieser Stelle gesondert hervorgehoben, dass das Auslassende 22 22 des Dochtes 20 hierbei nicht in direktem Kontakt mit dem zweiten Teil 44 des Ultraschallgenerators 40 steht. Anders ausgedrückt, steht das Auslassende 22 des Dochtes 20 nicht in Kontakt mit den Durchgängen 44p oder dem Netz 44p, die in oder durch den zweiten Teil 44 des Ultraschallgenerators 40 gebildet sind.

Das Auslassende 22 des Dochtes 20 steht nicht in direktem Kontakt mit dem zweiten Teil 44 des Ultraschallgenerators 40 an dessen Aufnahmefläche. Die Flüssigkeit 3 wird durch das Auslassende 22 des Dochtes zugeführt und von der Aufnahmefläche des zweiten Teils 44 des Ultraschallgenerators 40 aufgenommen. Der erste Teil 42 des Ultraschallgenerators 40 schwingt durch piezoelektrische Wirkung und implementiert oder überträgt die Schwingungen dann auf den zweiten Teil 44 des Ultraschallgenerators 40, beispielsweise indem er in direktem Kontakt mit dem zweiten Teil 44 steht. Infolgedessen schwingt auch der zweite Teil 44 des Ultraschallgenerators 40. In Folge dieser hochfrequenten Schwingung des zweiten Teils 44 des Ultraschalgenerators 40 wird die Flüssigkeit, die vom Auslassende 22 des Dochtes 20 aufgenommen und geeignet vernebelt, und sodann an der entgegengesetzten Austrittsfläche des zweiten Teils 44 als Aerosol 2 (vgl. Fig. 10) ausgestoßen, hinein in die Aerosolkammer 50s und in Richtung der Austrittsöffnung 50h des Mundstücks 50.

Das erzeugte Aerosol 2 (vgl. Fig. 10) verlässt dann den zweiten Teil 44 des Ultraschallgenerators 40 an oder von der Austrittsfläche des zweiten Teils 44 des Ultraschallgenerators 40, d.h. an dessen zum Docht 20 entgegengesetzten Seite.

Wie vorstehend bereits erläutert, ist die Oberflächenspannung der Flüssigkeit 3 ausreichend hoch gewählt bzw. eingestellt, dass sich an dem Auslassende 22 des Dochtes 20, gegenüberliegend zum Ultraschallgenerator 40 und dessen zweiten Teil 44, stets eine Ansammlung von Flüssigkeit 3 bildet. Erfindungsgemäß dient diese Ansammlung von Flüssigkeit 3 wie erläutert zur Übergabe an den Ultraschallgenerator 40 und dessen zweiten Teil 44, insbesondere dann, wenn dieser bei seiner Aktuierung in Schwingung versetzt wird.

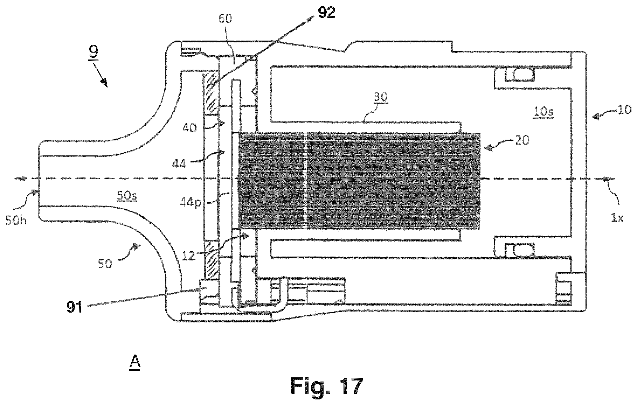

Das erzeugte Aerosol 2 wird von der Austrittsfläche des zweiten Teils 44 des Ultraschallgenerators 40 in eine Aerosolkammer 50s (vgl. Fig. 10) ausgestoßen, die zwischen der Austrittsöffnung 50hdes Mundstücks 50 und dem Ultraschallgenerator 40 definiert ist, und dann über die Austrittsöffnung 50h des Mundstücks 50 nach außen an die Vorrichtung 1 bzw. an den Außenbereich A (vgl. Fig. 17) der Kartusche 9 geleitet.

Fig. 17 zeigt eine weitere Ausführungsform der erfindungsgemäßen Aerosolerzeugungsvorrichtung 1, bei der zumindest ein aromatisiertes Element 92 mit einer vorbestimmten Geschmacksrichtung vorgesehen ist. Im Detail ist bei dieser Ausführungsform innerhalb des Mundstücks 50 ein Bypass 91 ausgebildet, durch den eine Fluidverbindung zwischen der Aerosolkammer 50s des Mundstücks 50 und einem Außenbereich A der Aerosolerzeugungsvorrichtung 1 bzw. der zugehörigen Kartusche 9 gebildet wird. Entsprechend wird im Betrieb der Aerosolerzeugungsvorrichtung 1, wenn ein Benutzer auf bzw. durch die Austrittsöffnung 50h des Mundstücks 50 eine Saugkraft ausübt, dann Frischluft von außen mit angesaugt und strömt durch den Bypass 91 hinein in die Aerosolkammer 50s.

Bei der Ausführungsform von Fig. 17 kann das besagte aromatisierte Element 92 an bzw. in dem Mundstück 50 strömungstechnisch angrenzend zu dem Bypass 91 angeordnet sein, oder auch direkt innerhalb des Bypass 91. Das aromatisierte Element 92 ist mit einer vorbestimmten Geschmacksrichtung versehen, je nach Wunsch des jeweiligen Anwenders. Jedenfalls ist das aromatisierte Element 92 in Bezug auf seine Geschmacksrichtung erfindungsgemäß derart beschaffen, dass eine Luftströmung, falls sie an diesem aromatisierten Element 92 entweder vorbeiströmt und/oder durch dieses Element 92 hindurchströmt, Geschmackspartikel von bzw. aus den aromatisierten Elementen 92 aufnimmt und dadurch die Luftströmung dann den jeweiligen Geschmack von dem aromatisierten Element 92 zusammen mit dem erzeugten Aerosol 2 annimmt. In Folge des vom Anwender ausgeübten Sogs wird dann die mit dem vorbestimmten Geschmack von dem aromatisierten Element 92 angereichte Luftströmung zusammen mit dem Aerosol 2, welches mittels der Schwingung des Ultraschallgenerators 40 erzeugt worden ist (vgl. hierzu die drei Punkte in Fig. 10, mit denen das Aerosol 2 in innerhalb der Aerosolkammer 50s vereinfacht symbolisiert ist), gemeinsam in Richtung der Austrittsöffnung 50h des Mundstücks 50 geleitet und tritt dort aus dem Mundstück 50 heraus, zwecks Inhalation durch den Anwender.

Bei der Ausführungsform von Fig. 17 ist das aromatisierte Element 92 ringförmig ausgebildet.

Zweckmäßigerweise sollte das aromatisierte Elemente 92 sich strömungstechnisch zwischen der Austrittsöffnung 50h des Mundstückes 50 und dem Bypass 91 befinden. Alternativ hierzu ist es auch möglich, dass das aromatisierte Element 92 auch direkt im Bypass 91 oder angrenzend hierzu, z.B. an einer (in Fig. 17 nicht gezeigten) Eingangsöffnung des Bypasses 91 angeordnet ist.

Bei der Ausführungsform von Fig. 17 kann auch vorgesehen sein, dass eine Mehrzahl von aromatisierten Elementen 92 an dem Mundstück angebracht sind. Für diesen Fall können die aromatisierten Elementen 92 entweder mit der gleichen Geschmacksrichtung ausgestattet sein, oder aber mit jeweils verschiedenen Geschmacksrichtungen versehen sein. Jedenfalls versteht sich bei einer Mehrzahl von aromatisierten Elementen 92, dass dann alle dieser Elemente 92 sich, wie bereits erläutert, strömungstechnisch zwischen der Austrittsöffnung 50h des Mundstückes 50 und dem Bypass 91 befinden. Damit ist gewährleistet, dass die Frischluft, die in Folge des vom Anwender erzeugten Sogs durch den Bypass 91 vom Außenbereich A der Kartusche 9 bzw. des Mundstücks 50 in dessen Aerosolkammer 50s hineinströmt, stets an den aromatisierten Elementen 92 vorbeiströmt und/oder durch diese Elemente 92 hindurchströmt und dadurch die Geschmacksrichtung der aromatisierten Elemente 92 annimmt.

Bei der vorstehend genannten Ausführungsform von Fig. 17 kann vorgesehen sein, dass sich ein aromatisiertes Element 92 vom Anwender einfach und vorzugsweise ohne die Verwendung von gesondertem Werkzeug austauschen lässt. Dies kann in einfacher Weise erreicht werden, wenn die Kartusche 9 von dem Hauptteil 8 der erfindungsgemäßen Aerosolerzeugungsvorrichtung 1 demontiert ist (vgl. Fig. 3), und/oder falls das Mundstück 50 von dem Behälter 10 abgenommen ist. Eine solche Austauschmöglichkeit gilt mutatis mutandis auch für eine Mehrzahl von aromatisierten Elementen 9, die gleichzeitig an bzw. in dem Mundstück 50 vorgesehen sein können.

Das vorstehend genannte Austauschen des aromatisierten Elements 92 ist bei der Ausführungsform von Fig. 17 in der Weise einfach möglich, weil hierbei, wie erläutert, dass aromatisierte Element 92 ringförmig ausgebildet ist. Insoweit lässt es sich in die Stirnseite des Mundstücks 50, wenn diese von den Behälter 10 demontiert ist, in einfacher Weise einlegen oder aber herausnehmen.

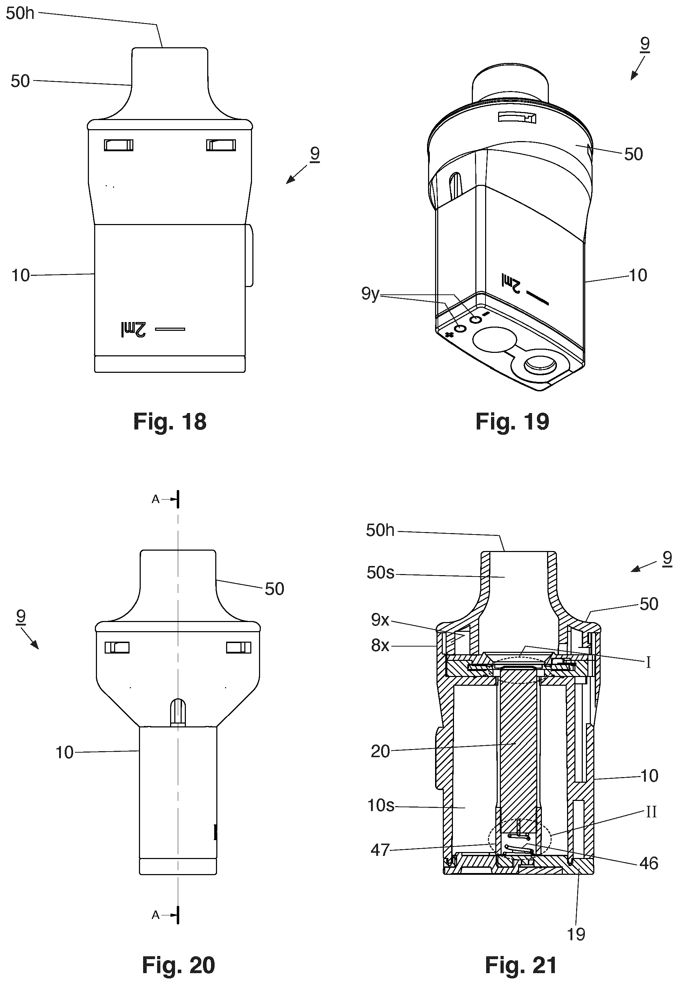

Nachstehend ist unter Bezugnahme auf die Fig. 18-28 eine weitere Ausführungsform der erfindungsgemäßen Aerosolerzeugungsvorrichtung 1 beschrieben und erläutert. Hierzu im Einzelnen:
Zunächst sind in den Fig. 18-20 nochmals verschiedene Ansichten der Kartusche 9 gezeigt, wenn diese von dem Hauptteil 8 der Aerosolerzeugungsvorrichtung 1 demontiert ist: Fig. 18 zeigt eine Vorderansicht der Kartusche 9, Fig. 19 eine Perspektivansicht der Kartusche 9 von schräg unten, und Fig. 20 eine Seitenansicht der Kartusche 9.

In der Fig. 19 sind nochmals die elektrischen Anschlüsse 9y gezeigt, mit denen eine Stromversorgung des Ultraschallgenerators 40, der innerhalb der Kartusche 9 angebracht ist, gewährleistet ist.

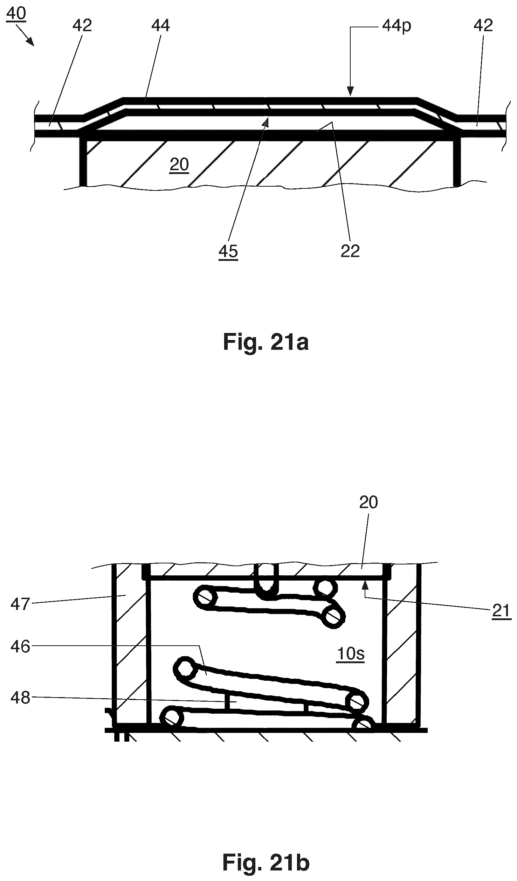

Fig. 21 zeigt eine Schnittansicht der Kartusche 9 entlang der Linie A-A von Fig. 20. Zur Verdeutlichung von Einzelheiten dieser Schnittansicht ist der Bereich I von Fig. 21 in der Fig. 21a vergrößert gezeigt. In gleicher Weise ist der Bereich II von Fig. 21 in der Fig. 21b vergrößert dargestellt.

Ein charakteristisches Merkmal der Ausführungsform der erfindungsgemäßen Aerosolerzeugungsvorrichtung 1 von Fig. 21 besteht darin, dass der Docht 20 durch eine Druckfeder 46 entlang seiner Längserstreckung in Richtung des Ultraschallgenerators 40 unter Vorspannung gesetzt ist, derart, dass damit der Docht 20 mit seinem Auslassende 22 gegen den ersten Teil 42 des Ultraschallgenerators 40 gedrückt wird. Anders aus, befindet sich bei der Ausführungsform von Fig. 21 der Docht 22 mit seiner Stirnseite, an welcher sein Auslassende 22 ausgebildet ist (vgl. hierzu auch Fig. 10), in Kontaktanlage mit dem ersten Teil 42 des Ultraschallgenerators 40.

Wie im unteren Bildbereich von Fig. 21, und in gleicher Weise auch in der Fig. 21b zu erkennen, ist hier die Druckfeder (46) in axialer Ausrichtung zum Docht (20) angeordnet und liegt dabei an einer unteren Stirnseite des Dochts (20) an, an der das Einlassende 21 (vgl. hierzu auch Fig. 10, in analoger Betrachtung) ausgebildet ist.

Bezüglich der Ausführungsform von Fig. 21 ist des Weiteren hervorzuheben, dass hierbei der zweite Teil 44 des Ultraschallgenerators 40 gegenüber dem Auslassende 22 des Dochts gesehen einen konkaven Abschnitt 45 aufweist. In Bezug auf diesen konkaven Abschnitt 45 ist zu verstehen, dass die Durchgänge 44p des zweiten Teils 44 des Ultraschallgenerators 40 in diesem konkaven Abschnitt 45 ausgebildet sind.

Der obere Bereich des Dochts 20, und der gegenüberliegend hierzu angeordnete Ultraschallgenerator 40, sind in der vergrößerten Ansicht gemäß Fig. 21a dargestellt wird. Hierin ist der vorstehend genannte konkave Abschnitt 45 des zweiten Teils 44 des Ultraschallgenerators 40 zu erkennen, der in Folge seiner gekrümmten Formgebung sich weg von dem Docht 20 erstreckt ist. Die Funktionsweise der Anordnung von Fig. 21a wird nachstehend noch im Detail erläutert.

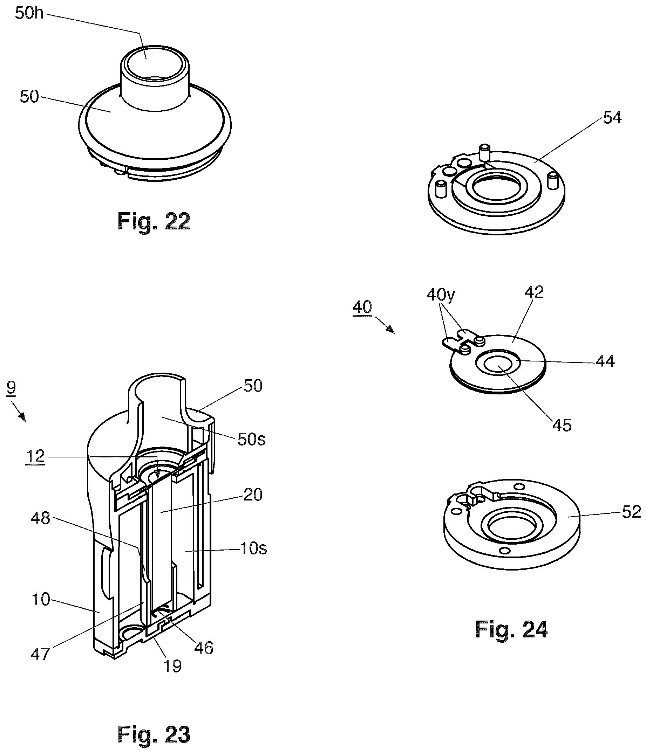

Der innere Bereich des Behälters 10 gemäß der Ausführungsform von Fig. 21 ist auch anhand der Fig. 23 gezeigt, welche die Schnittansicht von Fig. 21 nunmehr perspektivisch in einem Halbschnitt des Behälters 10 veranschaulicht.

Innerhalb des Behälters 10 ist eine Führungshülse 47 vorgesehen, in welcher der Docht 20 entlang seiner Längserstreckung eingefasst ist. Innerhalb dieser Führungshülse 47 sind mehrere Ausnehmungen 48 ausgebildet. Diese Ausnehmungen 48 erfüllen den Zweck, dass die Flüssigkeit, welche in dem Tank 10s des Behälters 10 gespeichert aufgenommen ist, radial durch die Ausnehmung(en) 48 in Richtung des Dochts 20 strömen kann, um an dem Einlassende 21 und/oder an anderen Stellen des Dochts 20 in die einzelnen Kapillare des Dochts 20 einzutreten, um anschließend in Richtung des Auslassendes 22 des Dochts zu strömen.

Fig. 22 zeigt nochmals - im demontierten Zustand - das Mundstück 50. Wie es beispielsweise aus der Fig. 23 ersichtlich ist, kann dieses Mundstück 50 an dem Behälter 10 an dessen Stirnseite, wo sich die Auslassöffnung 12 befindet, angebracht werden.

Fig. 24 zeigt nochmals den Ultraschallgenerator 40 in einer Perspektivansicht. Wie bereits im Zusammenhang mit der Fig. 7 erläutert, weist der Ultraschallgenerator 40 einen ersten ringförmigen Teil 42 (mit einem nicht näher gezeigten piezoelektrischen Element) auf, in dessen Zentrum das scheibenförmige zweite Teil 44 mit der Vielzahl von Durchgängen 44p (in Fig. 24 zur Vereinfachung nicht gezeigt) aufgenommen ist.

Zur flüssigkeits- bzw. fluiddichten Anbringung des Ultraschallgenerators 40 in der Auslassöffnung 12 des Behälters 10 bzw. angrenzend zu dieser Auslassöffnung 12 sind erfindungsgemäß geeignete Dichtelemente vorgesehen, beispielsweise eine Dichtungsaufnahme 52 und eine Dichtungsabdeckung 54. Im montierten Zustand ist der Ultraschallgenerator 40, nach Art eines "Sandwichs", zwischen der Dichtungsaufnahme 52 und der Dichtungsabdeckung 54 aufgenommen und zusammen mit diesen Dichtungselementen 52, 54 in der Auslassöffnung 12 oder angrenzend hierzu geeignet angeordnet.

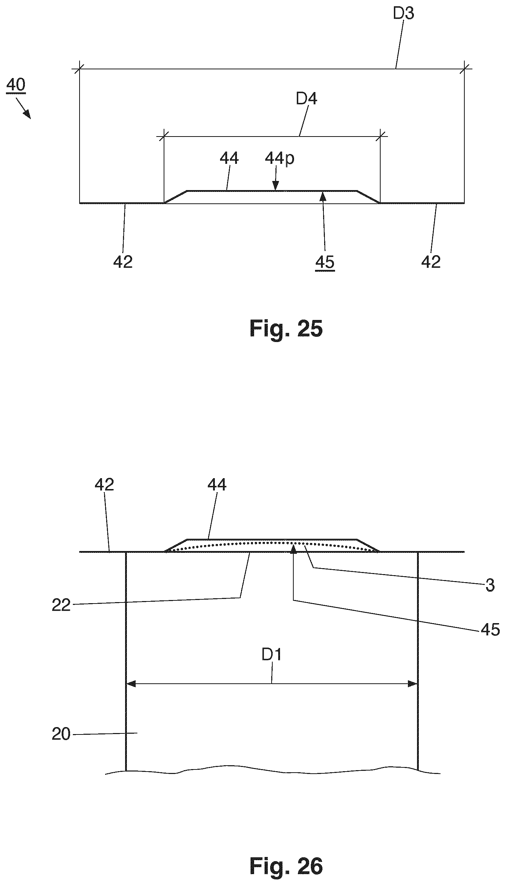

In der Fig. 25 ist der Ultraschallgenerator 40 mit seinem ersten Teil 42 und seinem zweiten Teil in einer vereinfachten Seitenansicht gezeigt. Hierin ist zu erkennen, dass das zweite Teil 44 den vorstehend bereits genannten konkaven Abschnitt 45 aufweist.

Im Einzelnen sind in der Fig. 25 folgende Abmessungen des Ultraschallgenerators 40 gezeigt bzw. erkennbar:
- Der ringförmige erste Teil 42 des Ultraschallgenerators 40 hat einen Außendurchmesser D3.
- Der scheibenförmigen zweiten Teil 44 einen Außendurchmesser D4.
- Der Außendurchmesser D1 des Dochts (20 (vgl. Fig. 14, und auch Fig. 26) ist größer ist als der Außendurchmesser D4 des scheibenförmigen zweiten Teils 44 des Ultraschallgenerators 40.
- Der Außendurchmesser D1 des Dochts 20 ist kleiner ist als der Außendurchmesser D3 des ringförmigen ersten Teils 42 des Ultraschallgenerators 40.

Fig. 26 zeigt für die Ausführungsform von Fig. 21 prinzipiell vereinfacht eine Anordnung des Dochts 20, wenn dieser sich in Kontakt mit dem zweiten Teil 42 des Ultraschallgenerators 40 befindet. In Folge der Vorspannung, die mittels der Druckfeder 46 auf den Docht 20 ausgeübt wird, wird der Docht 20 von unten her gegen den ersten Teil 42 des Ultraschallgenerators 40 gedrückt.

Qua dessen, dass der Außendurchmesser D1 des Dochts 20 wie erläutert kleiner ist als der Außendurchmesser D3 des ersten Teils 42 des Ultraschallgenerators 40 (vgl. Fig. 26), ist bei einem Kontakt des Dochts 20 mit bzw. an dem zweiten Teil 42 des Ultraschallgenerators 40 die eingangs bereits erläutert Dichtwirkung gewährleisten, wonach an dem Auslassende 22 des Dochts 20 ein unkontrolliertes Wegströmen von Flüssigkeit zur Seite hin nicht möglich ist. Stattdessen kommt es in Folge der erfindungsgemäß geeignet gewählten Oberflächenspannung der Flüssigkeit 3 dazu, dass sich hiervon eine bogenförmige Ansammlung an dem Auslassende 22 des Dochts 20 bildet. Diese Ansammlung von Flüssigkeit 3 ist in der Fig. 26 durch eine (nach oben) konvex gekrümmte punktierte Linie symbolisiert.

In Bezug auf die Fig. 26 wird an dieser Stelle gesondert darauf hingewiesen, dass der zweite Teil des 44 des Ultraschallgenerators 40 mit seinem konkaven Abschnitt 45 den Docht 22 mit seinem Auslassende 22 nicht berührt, und umgekehrt: Der Docht 22 hat keinen Kontakt mit dem zweiten Teil 22 des Ultraschallgenerators 40 bzw. mit dessen konkaven Abschnitt 45. Dies entspricht mutatis mutandis auch der Ausführungsform gemäß der Fig. 15 und Fig. 16.

Im Hinblick auf die erfindungsgemäße Erzeugung eines Aerosols 2 funktioniert die Ausführungsform gemäß Fig. 21 in gleicher Weise wie die bereits erläutert der Ausführungsform von Fig. 15 und Fig. 16:
Bereits im Ruhezustand des Ultraschallgenerators 40 kommt es zu einer Ansammlung der Flüssigkeit an bzw. auf dem Auslassende 22 des Dochts 20 (vgl. Fig. 26). Wenn nun der Ultraschallgenerator 40 bei einer Betätigung der Schaltfläche 88 (vgl. Fig. 1) aktuiert und in Folge dessen der zweite Teil 44 des Ultraschallgenerators 44 in Schwingung versetzt wird, taucht dieser zweite Teil 44 bei einer Verformung nach unten, d.h. in Richtung des Dochts 20 in die Flüssigkeit 3 ein, die sich am Auslassende 22 des Dochts 20 angesammelt hat, wodurch dann die Flüssigkeit 3 in den Durchgängen 44p des zweiten Teils 44 des Ultraschallgenerators 40 aufgenommen wird.

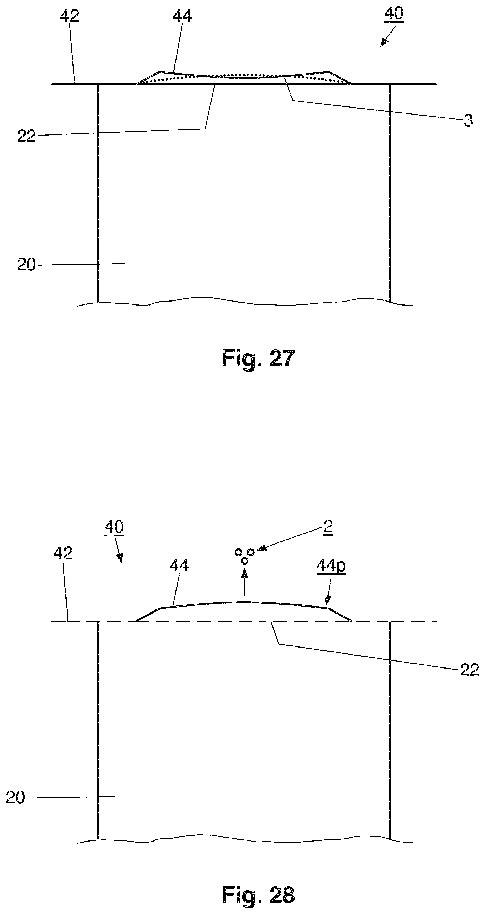

Bei fortgesetzter Schwingung des Ultraschallgenerators 40 verformt sich dessen zweiter Teil 44, in dessen Durchgängen 44p nunmehr wie erläutert die Flüssigkeit 3 aufgenommen worden ist, nach oben, so wie es prinzipiell vereinfacht in der Seitenansicht von Fig. 28 gezeigt ist. Bei dem Nach-Oben-Schwingen des zweiten Teils 44 des Ultraschallgenerators 40 wird dann die Flüssigkeit aus den Durchgängen 44p des zweiten Teils 44 herausgeschleudert und damit das gewünschte Aerosol 2 erzeugt. Dieses Aerosol 2 ist in Fig. 28 vereinfacht durch drei Punkte symbolisiert, in gleicher Weise wie auch in Fig. 10 bereits gezeigt und erläutert.

In Bezug auf die Ausführung gemäß der Fig. 21-28 wird nochmals gesondert darauf hingewiesen, dass der Kontakt, der sich hierbei zwischen dem Docht 20 und dem ersten Teil 42 des Ultraschallgenerators 40 bildet, in keiner Weise das Schwingungsverhalten des zweiten Teils 44 mit den darin ausgebildeten Durchgängen 44p beeinflusst oder stört. Vielmehr ist es infolge der Federvorspannung, die von der Druckfeder 46 auf den Docht 20 in seiner Axial- bzw Längsrichtung ausgeübt wird, und einer axialen Verschieblichkeit des Dochts 20 innerhalb der Führungshülse 47 möglich, dass der Docht 20 die Schwingungen des Ultraschallgenerators 40, die von dessen ersten Teil 42 induziert werden, "mitmacht" und entsprechend mitschwingt. Damit wird ein gleichbleibender Kontakt zwischen dem Auslassende 22 des Dochts 20 und dem ersten Teil des Ultraschallgenerators 40 erreicht und somit an dem Auslassende 22 des Dochts die besagte Dichtwirkung zur Seite hin realisiert.

Die vorliegende Erfindung sieht auch ein Verfahren zur Erzeugung eines Aerosols 2 vor. Das Aerosol 2 (vgl. Fig. 10, Fig. 28) kann durch eine Aerosolerzeugungsvorrichtung 1 gemäß der vorstehend erläuterten Ausführungsform(en) erzeugt werden. Jedenfalls kann bei dem erfindungsgemäßen Verfahren eine Flüssigkeit 2 in dem Tank 10s des Behälters 10 gespeichert sein, wobei die so gespeicherte Flüssigkeit 3 wie erläutert durch eine Betätigung des Ultraschallgenerators 40 der Vorrichtung 1 geeignet aerosolisiert bzw. vernebelt wird.

Gemäß einer vorteilhaften Weiterbildung der Erfindung besteht die Flüssigkeit aus Wasser und Ethanol. Eine Wassermenge kann gleich oder größer als 80 Gew.-% sein. In diesem Zusammenhang ist hervorzuheben, dass Stoffe oder Flüssigkeiten, die eine Verkeimung der Aerosolerzeugungsvorrichtung 1 verhindern, die Oberflächenspannung der Flüssigkeit 3 reduzieren können. Im Allgemeinen kann eine Verringerung der Oberflächenspannung Auswirkungen auf den Transport der Flüssigkeit 3 durch den Docht 20 haben. Falls nämlich die Oberflächenspannung der Flüssigkeit 3 zu gering ist, bildet sich keine ausreichende Ansammlung der Flüssigkeit 3 auf dem Auslassende 22 des Dochtes 20. Somit ist erfindungsgemäß vorgesehen, dass die Flüssigkeit 3 eine wasserbasierte Nikotinflüssigkeit ist, wobei andere Flüssigkeitsanteile, die eine Verkeimung der Aerosolerzeugungsvorrichtung 1 verhindern, die Oberflächenspannung der Flüssigkeit 3 nicht so weit reduzieren, dass es nicht mehr zu einer Ansammlung von Flüssigkeit 3 an dem Auslassende 22 des Dochtes 20 kommt. Erfindungsgemäß kann dies durch eine Verwendung der Substanzen Wasserstoffperoxid, ätherische Öle, Silberionen-Lösung und/oder Natriumchlorid erreicht werden, die in der Flüssigkeit 3 enthalten und/oder geeignet in den Docht 20 eingebracht sind.

Schließlich wird darauf hingewiesen, dass erfindungsgemäß auch zusätzliche Stoffe und oder Flüssigkeiten der wasserbasierten Flüssigkeit 3 zugefügt sein können, beispielsweise ein Lebensmittelzusatzstoff in Form von Milchsäure. Durch alle Anteile der wasserbasierten Flüssigkeit 3 kann der gewünschte pH-Wert eingestellt werden. Der pH-Wert der wasserbasierten Flüssigkeit 3 sollte so eingestellt sein, dass eine Korrosion an den Durchgängen 44p des Ultraschallgenerators 40 vermieden wird.

In Bezug auf den vorstehend genannten pH-Wert der Flüssigkeit 3 wird gesondert darauf hingewiesen, dass es durch die Zugabe eines Lebensmittelzusatzstoffs, beispielsweise Milchsäure, möglich ist, für die Flüssigkeit 3 einen pH-Wert zwischen 4 und 9,5 einzustellen. Durch eine modifizierte Konzentration von Milchsäure kann der pH-Wert der Flüssigkeit 3 auch im Bereich zwischen 4 und 8 liegen, weiter vorzugsweise auch in einem Bereich zwischen 5 und 7. Anders ausgedrückt, liegt dann für die letztgenannte Variante der pH-Wert der Flüssigkeit 3 im Bereich 5-7. Jedenfalls wird durch die Verbindung mit der zugegebenen Milchsäure aus der Flüssigkeit 3 (z.B. eine wasserbasierte Nikotinflüssigkeit) dann ein Nikotin-Laktat gebildet.

### Bezugszeichenliste

- 1: Aerosolerzeugungsvorrichtung
- 1x: Längsachse des Gerätes
- 2: Aerosol
- 3: Flüssigkeit, die versprüht werden soll
- 4: Lücke
- 5: Fluidleitungspfad
- 6: Draht
- 8: Hauptteil
- 8x: Haupteingriffsteil
- 9: Kartusche
- 9x: Kartuscheneingriffsteil
- 9y: Elektrische Anschlüsse
- 10: Behälter
- 10a: Innenfläche des Behälters 10
- 10b: Außenfläche des Behälters 10
- 10s: Tank
- 10f: Reservoirabschnitt des Behälters 10
- 10g: Führungssegment des Behälters 10
- 11: Körper des Behälters 10
- 11a: erster Teil des Körpers 11
- 11b: zweiter Teil des Körpers 11
- 11x: fluiddichte Verbindung
- 12: Auslassöffnung
- 12c: Innenfläche der Auslassöffnung 12
- 12s: Dichtung
- 15: Behälterverbindungselement
- 18: Einlassöffnung
- 19: Deckel
- 20: Docht
- 20a: Oberseite des Dochts 20
- 20b: Unterseite des Dochts 20
- 20c: Außenfläche des Dochts 20
- 21: Einlassende des Dochts 20
- 22: Auslassende des Dochts 20
- 24: Innerer Abschnitt des Dochts 20
- 25: Öffnungsabschnitt des Dochtes 20
- 26: Äußerer Abschnitt des Dochts 20
- 30: Führung
- 30a: Innenfläche der Führung 30
- 30b: Außenfläche der Führung 30
- 30s: Aufnahmeraum
- 31: Einlassende der Führung 30
- 31h: Einlass der Führung 30
- 32: Auslassende der Führung
- 32h: Auslass der Führung 30
- 35: Umfangswand der Führung 30
- 38: Kontaktteil
- 40: Ultraschallgenerator
- 40y: elektrische Anschlüsse des Ultraschallgenerators 40
- 42: erster Teil des Ultraschallgenerators 40
- 44: zweiter Teil des Ultraschallgenerators 40
- 44p: Durchgänge
- 45: konkaver Abschnitt des zweiten Teils 44 des Ultraschallgenerators 40
- 46: Druckfeder
- 47: Führungshülse (für die Druckfeder 46)
- 48: Ausnehmung (in der Führungshülse 47)
- 50: Mundstück
- 50h: Austrittsöffnung des Mundstücks 50
- 50s: Aerosolkammer
- 52: Dichtungsaufnahme
- 54: Dichtungsabdeckung
- 60: Halteelement
- 82: Ladeanschluss
- 84: Anzeige/Display
- 86: Benutzeroberfläche/Schaltfläche
- 88: LED-Anzeige
- 89: Logo
- 90: Abstand
- A: Außenbereich (der Kartusche 9 bzw. des Mundstücks 50)
- PL: Kombinierte Länge
- FL: volle Länge des Dochts 20
- D1: Außendurchmesser des Dochts 20
- D1: Innendurchmesser der Führung 30 bzw. der Auslassöffnung 22
- D2: Außendurchmesser der Führung 30 bzw. der Auslassöffnung 22
- D3: Außendurchmesser des ersten Teils 42 des Ultraschallgenerators 40
- D4: Außendurchmesser des zweiten Teils 44 des Ultraschallgenerators 40
- Lc: Länge des Tanks 10s
- Lg: Länge der Führung 30 (ab Auslassöffnung 22)
- Lw: Länge des Dochts 20 (ab der Auslassöffnung 22)
- Lp1: Länge des Dochts 20 (ausserhalb des Behälters 10)
- Lp2: Länge des Dochts 20 (ausserhalb der Führung 30)

## Patentansprüche

1. Aerosolerzeugungsvorrichtung (1) zum Erzeugen eines Aerosols (2), umfassend:
- einen Behälter (10) mit einem darin ausgebildeten Tank (10s), in dem eine zu versprühende Flüssigkeit (3) gespeichert aufgenommen ist, wobei der Behälter (10) eine Auslassöffnung (12) aufweist,
- einen Ultraschallgenerator (40), mittels dessen das Aerosol (2) aus der Flüssigkeit (3) erzeugbar ist,
- ein an dem Behälter (10) angrenzend zu dessen Auslassöffnung angebrachtes Mundstück (50), das eine Austrittsöffnung (50h) zum Freisetzen des von dem Ultraschallgenerator (40) erzeugten Aerosols (2) aufweist, und
- einen in der Auslassöffnung (12) des Behälters (10) angeordneten Docht (20) zum Transport der Flüssigkeit (3) aus dem Tank (10s) zum Ultraschallgenerator (40), wobei der Docht (20) ein im Inneren des Tanks (10s) angeordnetes Einlassende (21) zur Aufnahme der in dem Tank (10s) gespeicherten Flüssigkeit (3) und ein angrenzend zum Ultraschallgenerator (40) angeordnetes Auslassende (22) zur Bereitstellung der Flüssigkeit (3) für den Ultraschallgenerator (40) aufweist,
wobei der Ultraschallgenerator (40) zwischen der Austrittsöffnung (50h) des Mundstücks (50) und dem Docht (20) angeordnet ist,
wobei der Ultraschallgenerator (40) einen ersten Teil (42) mit einem piezoelektrischen Element und einen zweiten Teil (44) mit einer Vielzahl von Durchgängen (44p), durch welche die Flüssigkeit (3) hindurchtreten kann, umfasst,
**dadurch gekennzeichnet,**
**dass** das Auslassende (22) des Dochtes (20) so weit von dem zweiten Teil (44) des Ultraschallgenerators (40) beabstandet ist, dass der in Schwingung versetzte zweite Teil (44) mit der Vielzahl von Durchgängen (44p) nur die Flüssigkeit (3), die sich durch die Oberflächenspannung der selbigen Flüssigkeit (3) am Auslassende (22) des Dochts (20) angesammelt hat und dort in Folge ihrer Oberflächenspannung einen bogenförmigen Tropfen bildet, berührt oder in diese Flüssigkeit (3) eintaucht und dadurch die Flüssigkeit (3) in den Durchgängen (44p) des zweiten Teils (44) des Ultraschallgenerators (40) aufgenommen wird.

2. Aerosolerzeugungsvorrichtung (1) nach Anspruch 1, **dadurch gekennzeichnet, dass** der Behälter (10) bis auf seine Auslassöffnung (12) flüssigkeitsdicht ist, so dass die Flüssigkeit (3) aus dem Tank (10s) nur über den Docht (20) zum Ultraschallgenerator (40) transportiert wird.

3. Aerosolerzeugungsvorrichtung (1) nach Anspruch 1 oder 2, **dadurch gekennzeichnet, dass** der Behälter (10) in seinem Innenraum eine rohrförmig ausgebildete Führung (30) aufweist, die sich von der Auslassöffnung (12) des Behälters (10) parallel zu einer Längsachse (1x) der Vorrichtung (1) in den Tank (10s) hinein erstreckt und einen Aufnahmeraum (30s) aufweist, wobei der Docht (20) in dem Aufnahmeraum (30s) aufgenommen ist, vorzugsweise, dass eine Querschnittsfläche des Aufnahmeraums (30s) durch den Docht (20) ausgefüllt ist.

4. Aerosolerzeugungsvorrichtung (1) nach Anspruch 3, **dadurch gekennzeichnet, dass** eine Länge (Lg) der Führung (30), die sich von der Austrittsöffnung (12) des Behälters (10) in den Tank (10s) erstreckt, gleich oder größer als 50 % oder gleich oder größer als 70 % einer Länge (Lc) des Tanks (10s) ist.

5. Aerosolerzeugungsvorrichtung (1) nach einem der vorhergehenden Ansprüche, **dadurch gekennzeichnet, dass** eine Länge (Lw) des Dochtes (20), die sich von der Auslassöffnung (12) des Behälters (10) in den Tank (10s) hinein erstreckt, gleich oder größer als 50 % oder gleich oder größer als 70 % einer Länge (Lc) des Tanks (10s) ist; und/oder dass ein Durchmesser (D1) des Dochtes (20) und/oder ein Durchmesser der Auslassöffnung (12) des Behälters (10) und/oder ein Innendurchmesser (D1) der Führung (30) größer oder gleich 5 mm oder größer als 8 mm ist bzw. sind.

6. Aerosolerzeugungsvorrichtung (1) nach einem der vorhergehenden Ansprüche, **dadurch gekennzeichnet, dass** der erste Teil (42) des Ultraschallgenerators (40) ringförmig ausgebildet ist, wobei der zweite Teil (44) des Ultraschallgenerators (40) in einem Zentrum des ringförmig ausgebildeten ersten Teils (42) angeordnet ist, vorzugsweise, dass der zweite Teil (44) des Ultraschallgenerators (40) in Form einer Scheibe ausgebildet ist.

7. Aerosolerzeugungsvorrichtung (1) nach einem der vorhergehenden Ansprüche, **dadurch gekennzeichnet, dass** der zweite Teil (44) des Ultraschallgenerators (40) gegenüber dem Auslassende (22) des Dochts (20) gesehen einen konkaven Abschnitt (45) aufweist, wobei die Durchgänge (44p) des zweiten Teils (44) des Ultraschallgenerators (40) in dem konkaven Abschnitt (45) ausgebildet sind.

8. Aerosolerzeugungsvorrichtung (1) nach Anspruch 7, soweit rückbezogen auf Anspruch 6, **dadurch gekennzeichnet, dass** der Docht (20) einen Außendurchmesser (D1) aufweist, und dass der Ultraschallgenerator (40) für seinen ringförmigen ersten Teil (42) einen Außendurchmesser (D3) und für seinen scheibenförmigen zweiten Teil (44) einen Außendurchmesser (D4) aufweist, wobei der Außendurchmesser (D1) des Dochts (20) größer ist als der Außendurchmesser (D4) des scheibenförmigen zweiten Teils (44) des Ultraschallgenerators (40), vorzugsweise, dass der Außendurchmesser (D1) des Dochts (20) kleiner ist als der Außendurchmesser (D3) des ringförmigen ersten Teils (42) des Ultraschallgenerators (40), vorzugsweise, dass der Docht (20) mit seinem Auslassende (22) sich in Kontakt mit dem ersten Teil (42) des Ultraschallgenerators (40) befindet.

9. Aerosolerzeugungsvorrichtung (1) nach einem der vorhergehenden Ansprüche, **dadurch gekennzeichnet, dass** der Docht (20) durch eine Druckfeder (46) entlang seiner Längserstreckung in Richtung des Ultraschallgenerators (40) unter Vorspannung gesetzt ist, derart, dass damit der Docht (20) mit seinem Auslassende (22) gegen den ersten Teil (42) des Ultraschallgenerators (40) gedrückt wird, vorzugsweise, dass die Druckfeder (46) in axialer Ausrichtung zum Docht (20) angeordnet ist und an einer Stirnseite des Dochts (20) anliegt, an der dessen Einlassende (21) ausgebildet ist.

10. Verfahren zum Erzeugen eines Aerosols (2) unter Verwendung einer Aerosolerzeugungsvorrichtung (1) mit einem Docht (20) und einem Ultraschallgenerator (40), der einen ersten Teil (42) mit einem piezoelektrischen Element und einen zweiten Teil (44) mit einer Vielzahl von für Flüssigkeit durchlässigen Durchgängen (44p) umfasst, insbesondere nach einem der Ansprüche 1 bis 9, wobei bei dem Verfahren vorgesehen ist, dass eine Flüssigkeit (3), mit der das Aerosol (2) erzeugt wird, eine wasserbasierte Nikotinflüssigkeit ist, wobei die Flüssigkeit (3) eine Oberflächenspannung hat, die ausreichend hoch gewählt bzw. eingestellt ist, dass sich an einem Auslassende (22) des Dochtes (20) und gegenüberliegend zum Ultraschallgenerator (40) und dessen zweiten Teil (44) eine Ansammlung von Flüssigkeit (3) als bogenförmiger Tropfen bildet, wobei Flüssigkeit (3) von der Flüssigkeitsansammlung am Auslassende (22) des Dochtes (20) dann, wenn der Ultraschallgenerator (40) bei seiner Aktuierung in Schwingung versetzt wird und sich dabei in Richtung des Auslassendes (22) des Dochts (20) verformt, an den zweiten Teil (44) des Ultraschallgenerators (40) übergeben wird.

11. Verfahren nach Anspruch 10, **dadurch gekennzeichnet, dass** die Flüssigkeit (3) Nikotin in einer Menge enthält, die aus einem Bereich von 0,05 mg/ml bis 20 mg/ml gewählt ist.

12. Verfahren nach Anspruch 10 oder 11, **dadurch gekennzeichnet, dass** die Flüssigkeit (3) verkeimungshemmende Stoffe enthält, vorzugsweise, dass in der Flüssigkeit (3) als verkeimungshemmender Stoff zumindest eine Substanz gebildet aus der Gruppe Wasserstoffperoxid, ätherische Öle, Silberionen-Lösung und Natriumchlorid enthalten ist, vorzugsweise, dass in der Flüssigkeit (3) eine Kombination von Substanzen gebildet aus der Gruppe von Wasserstoffperoxid, ätherische Öle, Silberionen-Lösung und/oder Natriumchlorid enthalten ist.

13. Verfahren nach einem der Ansprüche 10 bis 12, **dadurch gekennzeichnet, dass** die Flüssigkeit (3) Natriumchlorid in einer Menge von 0,5 bis 3 Gewichtsprozent bezogen auf den Anteil von Wasser enthält, vorzugsweise, dass die Flüssigkeit (3) 0,9 Gewichtsprozent Natriumchlorid bezogen auf den Anteil von Wasser enthält.

14. Verfahren nach einem der Ansprüche 10 bis 13, **dadurch gekennzeichnet, dass** die Flüssigkeit (3) enthält: 85 Volumenprozent Wasser, 15 Volumenprozent Ethanol, 0,5 mg/ml Nikotin, und 0,9 Gewichtsprozent Natriumchlorid bezogen auf den Anteil von Wasser.

15. Verfahren nach einem der Ansprüche 10 bis 14, **dadurch gekennzeichnet, dass** für die Flüssigkeit (3) durch Zugabe eines Lebensmittelzusatzstoffs insbesondere in Form von Milchsäure ein pH-Wert zwischen 4 und 9,5 eingestellt wird, vorzugsweise, dass für die Flüssigkeit (3) durch Zugabe eines Lebensmittelzusatzstoffs insbesondere in Form von Milchsäure ein pH-Wert zwischen 4 und 8 eingestellt wird, weiter vorzugsweise, dass für die Flüssigkeit (3) durch Zugabe eines Lebensmittelzusatzstoffs insbesondere in Form von Milchsäure ein pH-Wert zwischen 5 und 7 eingestellt wird.

## Claims

1. Aerosol generating device (1) for generating an aerosol (2), comprising:
- a container (10) with a tank (10s) formed therein, in which a liquid (3) to be aerosolized is accomodated, wherein the container (10) has an outlet opening (12),
- an ultrasonic generator (40) by means of which the aerosol (2) can be generated from the liquid (3),
- a mouthpiece (50) attached to the container (10) adjacent to its outlet opening, which has an discharge opening (50h) for discharging the aerosol (2) generated by the ultrasonic generator (40), and
- a wick (20) arranged in the outlet opening (12) of the container (10) for transporting the liquid (3) from the tank (10s) to the ultrasonic generator (40), wherein the wick (20) has an inlet end (21) disposed inside the tank (10s) for receiving the liquid (3) stored in the tank (10s) and an outlet end (22) disposed adjacent to the ultrasonic generator (40) for providing the liquid (3) to the ultrasonic generator (40),
wherein the ultrasonic generator (40) is disposed between the discharge opening (50h) of the mouthpiece (50) and the wick (20),
wherein the ultrasonic generator (40) comprises a first part (42) with a piezoelectric element and a second part (44) with a plurality of passageways (44p) for allowing the liquid (3) to pass therethrough,
**characterised in that**
that the outlet end (22) of the wick (20) is spaced apart from the second part (44) of the ultrasonic generator (40) to such an extent that the vibrating second part (44) with the plurality of passageways (44p) only touches the liquid (3) which has accumulated at the outlet end (22) of the wick (20) due to the surface tension of this liquid (3) and forms an arcuate drop there as a result of its surface tension, or immerses into this liquid (3) and thereby the liquid (3) is absorbed into the passageways (44p) of the second part (44) of the ultrasonic generator (40).

2. Aerosol generating device (1) according to claim 1, **characterised in that** the container (10) is liquid-tight except for its outlet opening (12), so that the liquid (3) is transported from the tank (10s) to the ultrasonic generator (40) only via the wick (20).

3. Aerosol generating device (1) according to claim 1 or 2, **characterised in that** the container (10) has a tubular guide (30) in its interior, which extends from the outlet opening (12) of the container (10) parallel to a longitudinal axis (1x) of the device (1) into the tank (10s) and has a receiving space (30s), wherein the wick (20) is received in the receiving space (30s), preferably **in that** a cross-sectional area of the receiving space (30s) is filled by the wick (20).

4. Aerosol generating device (1) according to claim 3, **characterised in that** a length (Lg) of the guide (30) extending from the outlet opening (12) of the container (10) into the tank (10s) is equal to or greater than 50% or equal to or greater than 70% of a length (Lc) of the tank (10s).

5. Aerosol generating device (1) according to any of the preceding claims, **characterised in that** a length (Lw) of the wick (20) extending from the outlet opening (12) of the container (10) into the tank (10s) is equal to or greater than 50% or equal to or greater than 70% of a length (Lc) of the tank (10s); and/or **in that** a diameter (D1) of the wick (20) and/or a diameter of the outlet opening (12) of the container (10) and/or an inner diameter (D1) of the guide (30) is/are greater than or equal to 5 mm or greater than 8 mm.

6. Aerosol generating device (1) according to one of the preceding claims, **characterised in that** the first part (42) of the ultrasonic generator (40) is ring-shaped, wherein the second part (44) of the ultrasonic generator (40) is disposed in a centre of the ring-shaped first part (42), preferably **in that** the second part (44) of the ultrasonic generator (40) is designed as a disc.

7. Aerosol generating device (1) according to one of the preceding claims, **characterised in that** the second part (44) of the ultrasonic generator (40) has a concave section (45) as seen from the outlet end (22) of the wick (20), wherein the passageways (44p) of the second part (44) of the ultrasonic generator (40) are formed in the concave section (45).

8. Aerosol generating device (1) according to claim 7, as far as referred back to claim 6, **characterised in that** the wick (20) has an outer diameter (D1) and that the ultrasonic generator (40) has an outer diameter (D3) for its annular first part (42) and an outer diameter (D4) for its disc-shaped second part (44), wherein the outer diameter (D1) of the wick (20) is greater than the outer diameter (D4) of the disc-shaped second part (44) of the ultrasonic generator (40), preferably **in that** the outer diameter (D1) of the wick (20) is smaller than the outer diameter (D3) of the ring-shaped first part (42) of the ultrasonic generator (40), preferably **in that** the wick (20) with its outlet end (22) is in contact with the first part (42) of the ultrasonic generator (40).

9. Aerosol generating device (1) according to one of the preceding claims, **characterised in that** the wick (20) is preloaded by a compression spring (46) along its longitudinal extension in the direction of the ultrasonic generator (40) in such a way that the wick (20) with its outlet end (22) is pressed against the first part (42) of the ultrasonic generator (40), preferably **in that** the compression spring (46) is arranged in axial alignment with the wick (20) and rests against an end face of the wick (20) at which its inlet end (21) is formed.

10. Method for generating an aerosol (2) using an aerosol generating device (1) with a wick (20) and an ultrasonic generator (40), which comprises a first part (42) with a piezoelectric element and a second part (44) with a plurality of passageways (44p) permeable to liquid, in particular according to one of claims 1 to 9, wherein the method provides that a liquid (3) with which the aerosol (2) is generated is a water-based nicotine liquid, wherein the liquid (3) has a surface tension that is selected or set sufficiently high that an accumulation of liquid (3) forms as an arcuate drop at an outlet end (22) of the wick (20) and opposite the ultrasonic generator (40) and its second part (44), wherein liquid (3) is transferred from the accumulation of liquid at the outlet end (22) of the wick (20) to the second part (44) of the ultrasonic generator (40) when the ultrasonic generator (40) is set into vibration during its actuation and thereby deforms in the direction of the outlet end (22) of the wick (20).

11. Method according to claim 10, **characterised in that** the liquid (3) contains nicotine in an amount selected from a range of 0.05 mg/ml to 20 mg/ml.

12. Method according to claim 10 or 11, **characterised in that** the liquid (3) contains antimicrobial substances, preferably **in that** the liquid (3) contains at least one substance from the group consisting of hydrogen peroxide, essential oils, silver ion solution and sodium chloride as an antimicrobial agent, preferably **in that** the liquid (3) contains a combination of substances from the group consisting of hydrogen peroxide, essential oils, silver ion solution and/or sodium chloride.

13. Method according to one of claims 10 to 12, **characterised in that** the liquid (3) contains sodium chloride in an amount of 0.5 to 3 percentage by weight based on the proportion of water, preferably **in that** the liquid (3) contains 0.9 percentage by weight of sodium chloride based on the proportion of water.

14. Method according to one of claims 10 to 13, **characterised in that** the liquid (3) contains: 85 percentage by volume of water, 15 percentage by volume of ethanol, 0.5 mg/ml nicotine, and 0.9 percentage by weight of sodium chloride based on the proportion of water.

15. Method according to one of claims 10 to 14, **characterised in that** a pH value between 4 and 9.5 is set for the liquid (3) by adding a food additive in particular in the form of lactic acid, preferably **in that** a pH value between 4 and 8 is set for the liquid (3) by adding a food additive in particular in the form of lactic acid, and further preferably that a pH value between 5 and 7 is set for the liquid (3) by adding a food additive in particular in the form of lactic acid.

## Revendications

1. Dispositif de génération d'aérosol (1) destiné à générer un aérosol (2), comprenant :
- un réservoir (10) présentant, à l'intérieur de celui-ci, une citerne (10s) dans laquelle est contenue un liquide (3) à nébuliser, le réservoir (10) présentant une ouverture de sortie (12),
- un générateur ultrasonore (40) au moyen duquel l'aérosol (2) peut être généré à partir du liquide (3),
- un embout (50) fixé au réservoir (10) adjacent à son ouverture de sortie (12), l'embout (50) présentant une ouverture de sortie (50h) destinée à libérer l'aérosol (2) généré par le générateur ultrasonore (40), et
- une mèche (20) disposée dans l'ouverture de sortie (12) du réservoir (10) pour transporter le liquide (3) de la citerne (10s) vers le générateur ultrasonore (40), la mèche (20) présentant une extrémité d'entrée (21) disposée à l'intérieur de la citerne (10s) pour recevoir le liquide (3) stocké dans la citerne (10s) et une extrémité de sortie (22) disposée adjacent au générateur ultrasonore (40) pour mettre le liquide (3) à disposition du générateur ultrasonore (40),
le générateur ultrasonore (40) étant disposé entre l'ouverture de sortie (50h) de l'embout (50) et la mèche (20),
le générateur ultrasonore (40) comprenant une première partie (42) comportant un élément piézoélectrique et une seconde partie (44) comportant une pluralité de passages (44p) à travers lesquels le liquide (3) peut passer,
**caractérisé en ce que**
l'extrémité de sortie (22) de la mèche (20) est espacée de la seconde partie (44) du générateur ultrasonore (40) de telle sorte que la seconde partie (44) mise en vibration avec la pluralité de passages (44p) ne touche que le liquide (3) qui, en raison de la tension superficielle dudit liquide (3), s'est accumulé à l'extrémité de sortie (22) de la mèche (20) et y forme, du fait de sa tension superficielle, une goutte arquée, ou s'immerge dans ce liquide (3), et que ce faisant le liquide (3) est entraîné dans les passages (44p) de la seconde partie (44) du générateur ultrasonore (40).

2. Dispositif de génération d'aérosol (1) selon la revendication 1, **caractérisé en ce que** le réservoir (10) est étanche aux liquides à l'exception de son ouverture de sortie (12), de sorte que le liquide (3) est transporté de la citerne (10s) vers le générateur ultrasonore (40) uniquement par l'intermédiaire de la mèche (20).

3. Dispositif de génération d'aérosol (1) selon l'une des revendications 1 ou 2, **caractérisé en ce que** le réservoir (10) présente, dans son espace intérieur, un guide (30) de forme tubulaire qui s'étend à partir de l'ouverture de sortie (12) du réservoir (10), parallèlement à un axe longitudinal (1x) du dispositif (1), dans la citerne (10s) et présente un espace de réception (30s), la mèche (20) étant reçue dans l'espace de réception (30s), de préférence **en ce qu'**une surface de section transversale de l'espace de réception (30s) est remplie par la mèche (20).

4. Dispositif de génération d'aérosol (1) selon la revendication 3, **caractérisé en ce qu'**une longueur (Lg) du guide (30), qui s'étend à partir de l'ouverture de sortie (12) du réservoir (10) dans la citerne (10s), est supérieure ou égale à 50% ou supérieure ou égale à 70% d'une longueur (Lc) de la citerne (10s).

5. Dispositif de génération d'aérosol (1) selon l'une des revendications précédentes, **caractérisé en ce qu'**une longueur (Lw) de la mèche (20), qui s'étend à partir de l'ouverture de sortie (12) du réservoir (10) dans la citerne (10s), est supérieure ou égale à 50% ou supérieure ou égale à 70% d'une longueur (Lc) de la citerne (10s), et/ou **en ce qu'**un diamètre (D1) de la mèche (20) et/ou un diamètre de l'ouverture de sortie (12) du réservoir (10) et/ou un diamètre intérieur (D1) du guide (30) est/sont supérieur ou égal à 5 mm ou supérieur à 8 mm.

6. Dispositif de génération d'aérosol (1) selon l'une des revendications précédentes, **caractérisé en ce que** la première partie (42) du générateur ultrasonore (40) est de forme annulaire, la seconde partie (44) du générateur ultrasonore (40) étant disposée au centre de la première partie (42) de forme annulaire, de préférence **en ce que** la seconde partie (44) du générateur ultrasonore (40) est conçue sous la forme d'un disque.

7. Dispositif de génération d'aérosol (1) selon l'une des revendications précédentes, **caractérisé en ce que** la seconde partie (44) du générateur ultrasonore (40) présente, vue depuis l'extrémité de sortie (22) de la mèche (20), une section concave (45), les passages (44p) de la seconde partie (44) du générateur ultrasonore (40) étant formés dans la section concave (45).

8. Dispositif de génération d'aérosol (1) selon la revendication 7, en référence à la revendication 6, **caractérisé en ce que** la mèche (20) présente un diamètre extérieur (D1), et **en ce que** le générateur ultrasonore (40) présente, pour sa première partie annulaire (42), un diamètre extérieur (D3) et, pour sa seconde partie (44) en forme de disque, un diamètre extérieur (D4), le diamètre extérieur (D1) de la mèche (20) étant supérieur au diamètre extérieur (D4) de la seconde partie (44) en forme de disque du générateur ultrasonore (40), de préférence **en ce que** le diamètre extérieur (D1) de la mèche (20) est inférieur au diamètre extérieur (D3) de la première partie annulaire (42) du générateur ultrasonore (40), de préférence **en ce que** la mèche (20), avec son extrémité de sortie (22), est en contact avec la première partie (42) du générateur ultrasonore (40).

9. Dispositif de génération d'aérosol (1) selon l'une des revendications précédentes, **caractérisé en ce que** la mèche (20) est mise sous précontrainte, le long de son extension longitudinale, en direction du générateur ultrasonore (40) par un ressort de compression (46), de telle sorte que la mèche (20), avec son extrémité de sortie (22), est poussée contre la première partie (42) du générateur ultrasonore (40), de préférence **en ce que** le ressort de compression (46) est disposé dans un alignement axial avec la mèche (20) et vient en appui contre une face d'extrémité de la mèche (20) sur laquelle est formée son extrémité d'entrée (21).

10. Procédé de génération d'un aérosol (2) utilisant un dispositif de production d'aérosol (1) comprenant une mèche (20) et un générateur d'ultrasons (40) comprenant une première partie (42) avec un élément piézoélectrique et une deuxième partie (44) avec une pluralité de passages (44p) perméables au liquide, en particulier selon l'une des revendications 1 à 9, dans lequel procédé il est prévu qu'un liquide (3) avec lequel l'aérosol (2) est généré est un liquide à base d'eau contenant de la nicotine, le liquide (3) présentant une tension superficielle choisie ou réglée suffisamment élevée pour qu'une accumulation de liquide (3) se forme, sous la forme d'une goutte arquée, à une extrémité de sortie (22) de la mèche (20) et en regard du générateur ultrasonore (40) et de sa seconde partie (44), un liquide (3) étant transféré depuis l'accumulation de liquide (3) à l'extrémité de sortie (22) de la mèche (20) vers la seconde partie (44) du générateur ultrasonore (40) lorsque le générateur ultrasonore (40), lors de son activation, est mis en vibration et se déforme ainsi en direction de l'extrémité de sortie (22) de la mèche (20).

11. Procédé selon la revendication 10, **caractérisé en ce que** le liquide (3) contient de la nicotine en une quantité choisie dans un intervalle allant de 0,05 mg/ml à 20 mg/ml.

12. Procédé selon la revendication 10 ou 11, **caractérisé en ce que** le liquide (3) contient des substances antimicrobiennes, de préférence **en ce que** le liquide (3) contient, en tant que substance antimicrobienne, au moins une substance choisie dans le groupe constitué de peroxyde d'hydrogène, huiles essentielles, solution d'ions argent et chlorure de sodium, de préférence **en ce que** le liquide (3) contient une combinaison de substances choisies dans le groupe constitué de peroxyde d'hydrogène, huiles essentielles, solution d'ions argent et/ou chlorure de sodium.

13. Procédé selon l'une des revendications 10 à 12, **caractérisé en ce que** le liquide (3) contient du chlorure de sodium en une quantité de 0,5 à 3 pour cent en poids par rapport à la fraction d'eau, de préférence **en ce que** le liquide (3) contient 0,9 pour cent en poids de chlorure de sodium par rapport à la fraction d'eau.

14. Procédé selon l'une des revendications 10 à 13, **caractérisé en ce que** le liquide (3) contient : 85 pour cent en volume d'eau, 15 pour cent en volume d'éthanol, 0,5 mg/ml de nicotine et 0,9 pour cent en poids de chlorure de sodium par rapport à la fraction d'eau.

15. Procédé selon l'une des revendications 10 à 14, **caractérisé en ce qu'**une valeur de pH entre 4 et 9,5 est réglée pour le liquide (3) par addition d'un additif alimentaire, en particulier sous la forme d'acide lactique, de préférence **en ce qu'**une valeur de pH entre 4 et 8 est réglée pour le liquide (3) par addition d'un additif alimentaire, en particulier sous la forme d'acide lactique, et plus préférentiellement **en ce qu'**une valeur de pH entre 5 et 7 est réglée pour le liquide (3) par addition d'un additif alimentaire, en particulier sous la forme d'acide lactique.
